# EUROPEAN PATENT APPLICATION

(11) **EP 4 696 327 A1**
(43) Date of publication of application: **18.02.2026**
(21) Application number: 25195876.5
(22) Date of filing: 14.08.2025
(51) Int. Cl.: A61K 47/54, A61P 1/16

(54) **GALNAC COMPOUND CONTAINING TRIAZOLE STRUCTURE AND OLIGONUCLEOTIDE CONJUGATE THEREOF**

(30) Priority: 15.08.2024 CN 202411117768
(71) Applicant: Beijing Youcare Kechuang Pharmaceutical Technology Co., Ltd., Daxing District Beijing (CN)
(72) Inventor: HUANG, Zeao, Beijing (CN); SONG, Gengshen, Beijing (CN); LI, Zhenmin, Beijing (CN); WANG, Jie, Beijing (CN); XIE, Hao, Beijing (CN)
(74) Representative: Novagraaf Technologies

(57) **Abstract**

Provided in the present application are a GalNAc compound containing a triazole structure, and an oligonucleotide conjugate thereof, and specifically disclosed is a GalNAc compound shown in formula (I) or a pharmaceutically acceptable salt thereof. The GalNAc compound in the present application can form a conjugate with oligonucleotide, and the oligonucleotide can realize the efficient liver targeting delivery of the oligonucleotide, regulate gene expression, and can be used for preventing and/or treating diseases caused by expression of specific genes in liver cells.

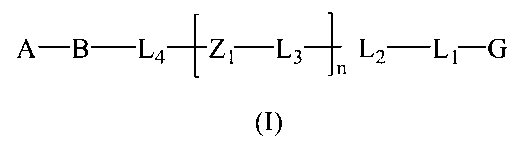

## Description

### FIELD

The present disclosure relates to the field of biomedicine, and specifically, to a GalNAc compound having a triazole ring structure, and a GalNAc oligonucleotide conjugate prepared by the compound.

### BACKGROUND

In recent years, after small molecule and antibody drugs, oligonucleotide drugs are emerging as the third wave of new drug development. The oligonucleotide drugs mainly bind to upstream mRNA of disease-causing proteins, and can be regulated at a gene level, and thus may provide therapeutic possibilities for a number of urgent clinical needs without available drugs, and have already shown great potential in the treatment of neuropathy and cardiovascular diseases.

Small nucleic acid drugs (oligonucleotide) are being developed as an alternative to traditional small molecule drugs by virtue of their unique properties, and are used to regulate and control the function of disease-related proteins. The oligonucleotide may be used for silencing or activating gene expression of specific diseases, so as to prevent or promote the formation of specific proteins, thereby achieving the effect of treating diseases. The oligonucleotide includes, but is not limited to, Antisense Oligonucleotide (ASO), small interfering RNA (siRNA), small activating RNA (saRNA), and microRNA (miRNAs). Due to the better efficacy of the oligonucleotide drugs and technological breakthroughs, it has become the technology that currently receives the most attention, and several drugs have already been approved and marketed around the world.

At present, the delivery systems used for marketed oligonucleotide drugs are: a Lipid Nanoparticle (LNP) delivery system and a N-acetylgalgactosamine (GalNAc) delivery system, herein the GalNAc delivery system is the most widely used. The GalNAc can bind to an endocytic receptor-Asialoglycoprotein Receptor (ASGPR) that is specifically expressed on surfaces of liver cells, and may realize the liver targeting delivery of the oligonucleotide drugs. Therefore, the improvement of the GalNAc may improve the delivery efficiency of the oligonucleotide drugs.

Galactose (Gal) and GalNAc are the ligand capable of binding to the ASGPR on the liver surface. The affinity of the binding of the GalNAc to the ASGPR is approximately 50 times higher than that of the Gal. Research found that the sequence of the affinity of the GalNAc with different antennary structures was: tetra-antennary > tri-antennary >> bi-antennary >> mono-antennary galactosamine. In recent years, progress has been made in liver targeting delivery of nucleic acid drugs with the high-affinity ligand GalNAc of the ASGPR as a targeting molecule. A variety of GalNAc may achieve conjugation with siRNA and ASO, and drug development for diseases such as amyloidosis, hemophilia, hypercholesterolemia, hepatitis B, etc. has been performed using this technology.

The delivery efficiency of nucleic acids varies greatly between different GalNAc structures. In order to improve the delivery efficiency of liver targeting drugs, such as complement inhibitor drugs, there is a need for the development of a new GalNAc compound in the art.

### SUMMARY

The present disclosure provides a new GalNAc compound containing a triazole structure. Triazole is introduced in a linker of the GalNAc compound, by means of the advantage of click chemistry, a series of new GalNAc compounds containing triazole structure may be constructed modularly, conveniently and rapidly. A GalNAc ligand is coupled to the 3' end of oligonucleotide with a phosphoramidite solid-phase synthesis method. Compared with the prior art, the efficiency of delivering the oligonucleotide to the liver by the GalNAc compound of the present disclosure is significantly improved, such that the compound may be used for developing drugs for treating liver tissue related diseases.

The present disclosure provides a GalNAc compound shown in formula (I) or a pharmaceutically acceptable salt thereof. 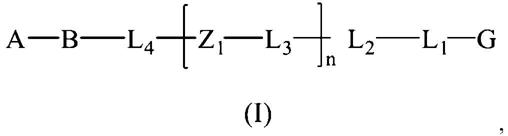
L₁ is -HNC(O)- or -C(O)NH-.
L₂ is -(CH₂)n₁- or -(CH₂CH₂O)n₂CH₂-, where n₁ and n₂ are integers from 1 to 7.
Z₁ is -(CH2)n₃-, where n₃ is an integer from 1 to 7.
L₃ is -HNC(O)- or -C(O)NH-.
n is an integer from 0 to 10.
L₄ is -(CH₂)n₄-, where n₄ is an integer from 0 to 7.
A is 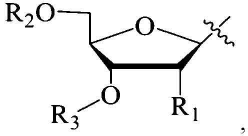 R₁ is H, C₁₋₆ alkyl, C₁₋₆ alkoxy or halogen; R₂ is 4,4'-dimethoxytrityl, monomethoxytrityl, triphenylmethyl, tert-butyldimethylsilyl, tert-butyldiphenylsilyl, triisopropylsilyl, or isopropyldimethylsilyl; R₃ is H, a hydroxyl protecting group, a phosphorus-containing reactive group, - CO(CH₂)ₓCONH-E, or -CO(CH₂)ₓCOOH, where x is an integer from 1 to 10, and E is Controlled Pore Glass (CPG) or polystyrene.
B is O or S;
G is 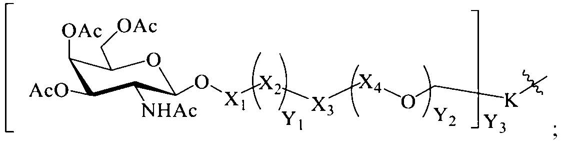
herein,
X₁ is -(CH₂)ₐ- or -(CH₂CH₂O)ₐCH₂-, a is an integer from 1 to 5.
X₂ is -HNC(O)- or -C(O)NH-.
X₃ is -(CH₂)_{b}-, b is an integer from 1 to 6.
X₄ is 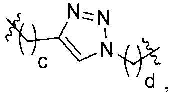 where c and d are integers from 0 to 5.
Y₁ is 0 or 1.
Y₂ is 0 or 1.
Y₃ is 1, 2, or 3.

When Y₃ is 1, K is CH₂; when Y₃ is 2, K is CH; and when Y₃ is 3, K is a carbon atom.

In some implementations of the present disclosure, L₁ is -C(O)NH-*, and the end * is connected to G.

In some implementations of the present disclosure, L₂ is -(CH₂)₃-, -(CH₂)₄-, -(CH₂)₅-, -(CH₂)₆-, -(CH₂)₇-, or -(CH₂CH₂O)₃CH₂-.

In some implementations of the present disclosure, L₂ is -(CH₂)₅- or -(CH₂CH₂O)₃CH₂-.

In some implementations of the present disclosure, n is 0.

In some implementations of the present disclosure, L₄ is not present, or is -(CH₂)₃-, -(CH₂)₄-, -(CH₂)₅-, - (CH₂)₆-, or -(CH₂)₇-.

In some implementations of the present disclosure, L₄ is not present, or is -(CH₂)₃- or -(CH₂)₅-.

In some implementations of the present disclosure, R₁ is H or methoxy.

In some implementations of the present disclosure, R₂ is 4,4'-dimethoxytrityl (DMTr).

In some implementations of the present disclosure, R₃ is -CO(CH₂)₂CONH-E, and E is CPG.

In some implementations of the present disclosure, B is O.

In some implementations of the present disclosure, X₁ is -(CH₂)₃-.

In some implementations of the present disclosure, X₂ is -HNC(O)-**, and the end ** is connected to X₃.

In some implementations of the present disclosure, X₃ is -(CH₂)₂-, -(CH₂)₄-, or -(CH₂)₅-.

In some implementations of the present disclosure, X₄ is 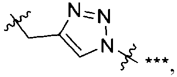 and the end *** is connected to X₃.

In some implementations of the present disclosure, Y₁ is 1.

In some implementations of the present disclosure, Y₂ is 1.

In some implementations of the present disclosure, Y₃ is 3, and K is a carbon atom.

In some implementations of the present disclosure, A is 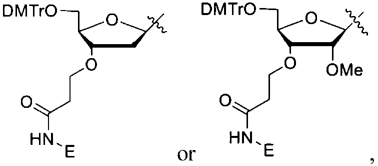 and E is CPG.

In some implementations of the present disclosure, G is: 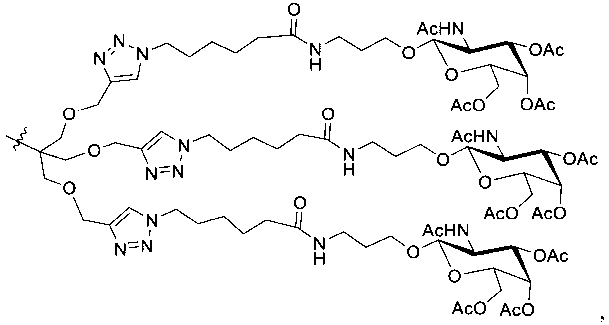 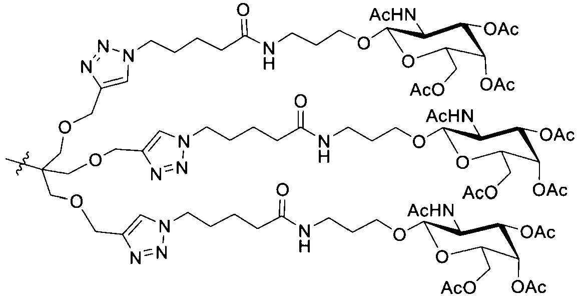 or

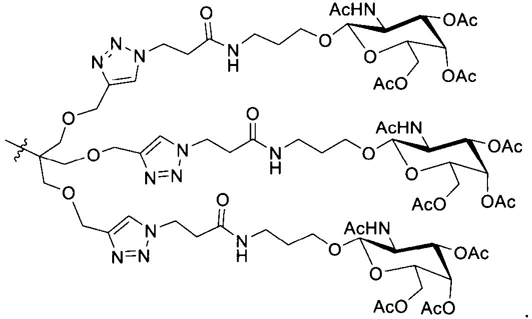

In some implementations of the present disclosure, the compound shown in formula (I) has a structure shown as formula (IA):

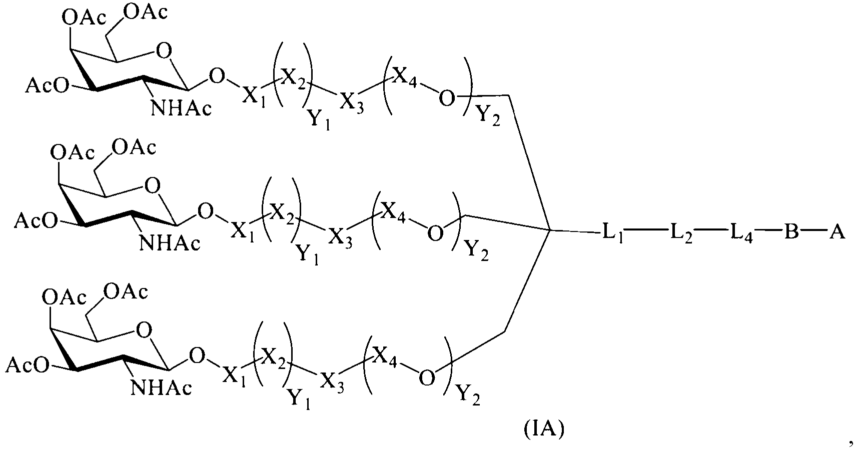

L₁, L₂, L₄, B, A, X₁, X₂, X₃, X₄, Y₁, and Y₂ are defined as described above.

In some implementations of the present disclosure, the compound shown in formula (I) or a pharmaceutically acceptable salt thereof can bind an Asialoglycoprotein Receptor (ASGPR).

In some implementations of the present disclosure, the compound shown in formula (I) is any one of the following compounds:

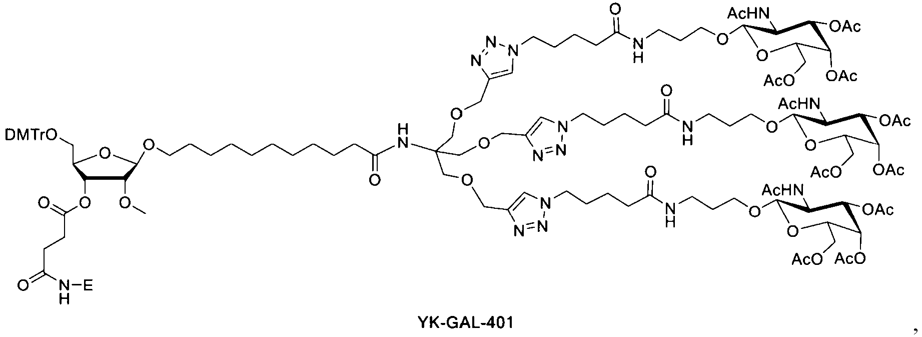

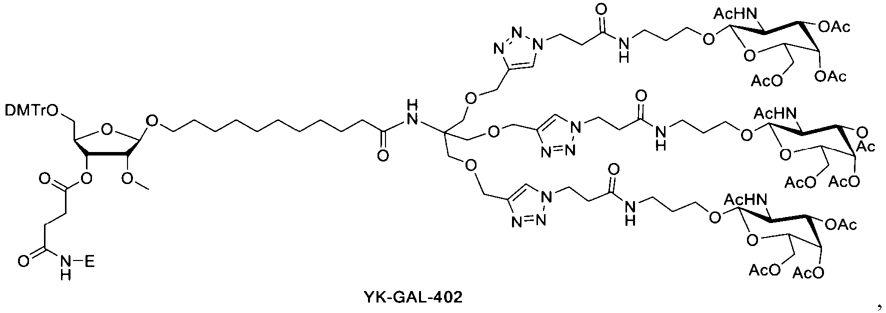

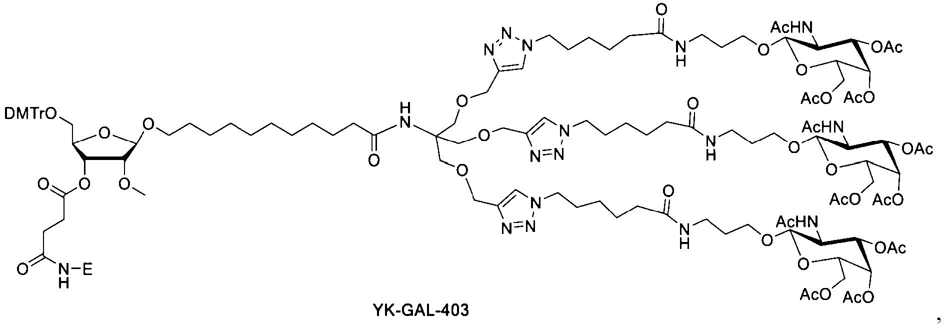

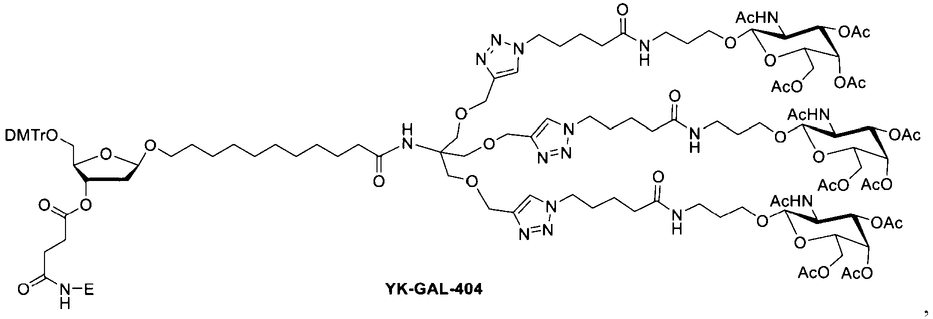

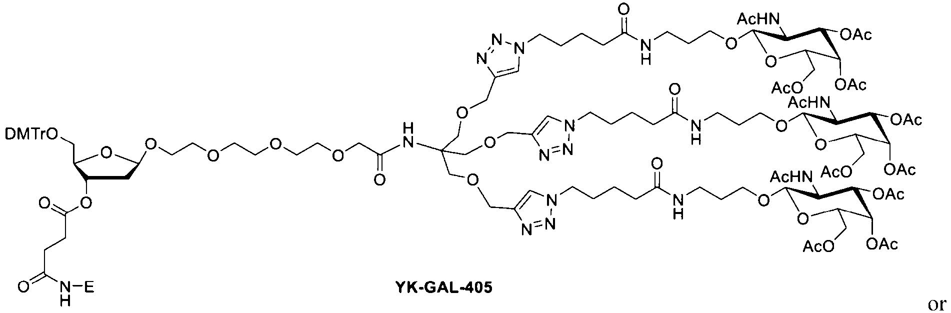

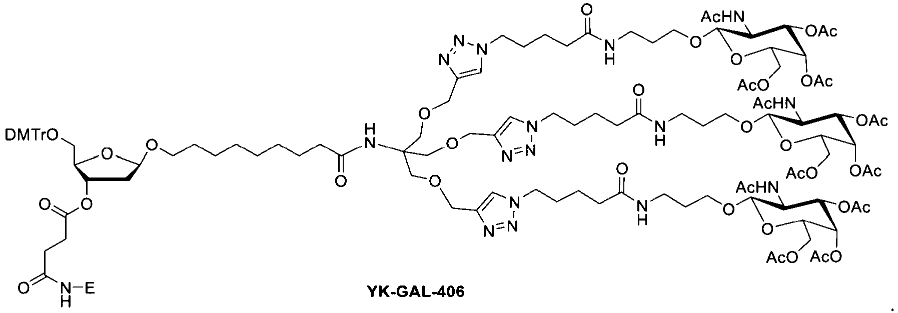

The present disclosure further provides a conjugate shown in formula (IIA) or (IIB) or a pharmaceutically acceptable salt thereof.

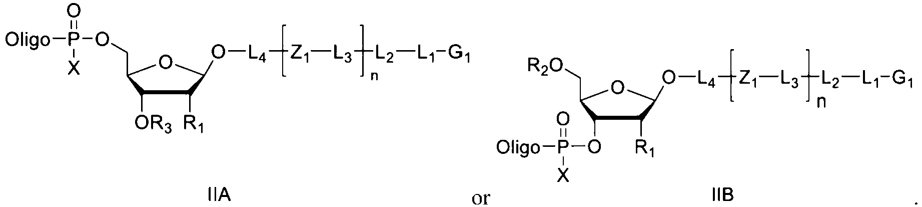

Oligo represents oligonucleotide, X represents hydroxyl or sulfhydryl, and G₁ is

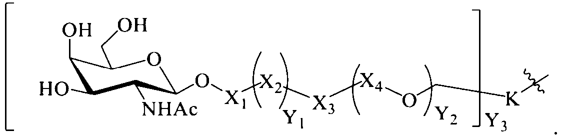

L₁, L₂, Z₁, L₃, L₄, n, R₁, R₂, R₃, X₁, X₂, X₃, X₄, Y₁, Y₂, Y₃, and K are defined as described above.

It is understandable by those skilled in the art that, in the conjugate shown in formula (IIA) or (IIB), a structure unit 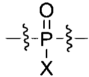 is formed by connecting a monomer with hydroxyl in a five-membered sugar ring (i.e., 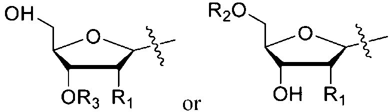 ) in solid-phase synthesis of the oligonucleotide.

In the conjugate shown in formula (IIA), R₃ is preferably H.

In the conjugate shown in formula (IIB), R₂ is preferably H.

In some implementations of the present disclosure, the oligonucleotide is selected from any one of groups consisting of small interfering nucleotide (siRNA), DNA, microRNA (miRNA), small activating RNA (saRNA), small guide RNA (sgRNA), transfer RNA (tRNA), ASO, and an aptamer, or a combination of at least two of the above, and preferably the ASO or the siRNA.

In some implementations of the present disclosure, each nucleotide in the ASO or the siRNA is each independently a modified or unmodified nucleotide.

In some implementations of the present disclosure, the oligonucleotide regulates expression of a target gene.

In some implementations of the present disclosure, the oligonucleotide is a double-stranded RNAi agent. In some implementations of the present disclosure, the double-stranded RNAi agent is an RNAi agent that inhibits expression of PCSK9 gene.

In some implementations of the present disclosure, at least one strand of the double-stranded RNAi agent contains a 3' protruding end of at least 1 nucleotide.

In some implementations of the present disclosure, at least one strand of the double-stranded RNAi agent contains 3' protruding ends of at least 2 nucleotides.

In some implementations of the present disclosure, a double-stranded region of the double-stranded RNAi agent is a length of 15-30 pairs of nucleotides.

In some implementations of the present disclosure, the double-stranded region of the double-stranded RNAi agent is the length of 15, 16, 17, 18, 19, 20, 21, 22, 23, 24, 25, 26, 27, 28, 29, or 30 pairs of nucleotides.

In some implementations of the present disclosure, a double-stranded region of the double-stranded RNAi agent is a length of 17-25 pairs of nucleotides.

In some implementations of the present disclosure, a double-stranded region of the double-stranded RNAi agent is a length of 19-23 pairs of nucleotides.

In some implementations of the present disclosure, a double-stranded region of the double-stranded RNAi agent is a length of 21 pairs of nucleotides.

In some implementations of the present disclosure, each strand of the double-stranded RNAi agent has 15-30 nucleotides.

In some implementations of the present disclosure, the number of the nucleotides of the sense strand and/or antisense strand of the double-stranded RNAi agent is selected from any one of 15, 16, 17, 18, 19, 20, 21, 22, 23, 24, 25, 26, 27, 28, 29, and 30, respectively.

In some implementations of the present disclosure, each strand of the double-stranded RNAi agent has 19-25 nucleotides.

In some implementations of the present disclosure, the sense strand of the double-stranded RNAi agent has 21 nucleotides, and the antisense strand has 23 nucleotides.

In some implementations of the present disclosure, the double-stranded RNAi agent contains any one of groups consisting of oligonucleotide double-stranded bodies formed by the pairing of unmodified sense strands and unmodified antisense strands in a table below, or a combination of at least two of the above (sequences being from Table 8 of the specification of CN117210468A).

| Sense strand sequence (basic sequence) 5'-3' | SE Q ID N O: | Correspond ing sequence ID number in CN1172104 68A | Antisense strand sequence (basic sequence) 5'-3' | SE Q ID N O: | Correspond ing sequence ID number in CN1172104 68A |
|---|---|---|---|---|---|
| AAGAUCCUGCAUGUC UUCCAU | 1 | 1 | AUGGAAGACAUGCAGGA UCUUGG | 13 | 13 |
| UGGAGGCUUAGCUUU CUGGAU | 2 | 2 | AUCCAGAAAGCUAAGCC UCCAUU | 14 | 14 |
| CUUUUGUAACUUGAA GAUAUU | 3 | 3 | AAUAUCUUCAAGUUACA AAAGCA | 15 | 15 |
| GAAGAUAUUUAUUCU GGGUUU | 4 | 4 | AAACCCAGAAUAAAUAU CUUCAA | 16 | 16 |
| GUUUUGUAGCAUUUU UAUUAA | 5 | 5 | UUAAUAAAAUGCUACA AAACCC | 17 | 17 |
| CACCAAGAUCCUGCA UGUCUU | 6 | 6 | AAGACAUGCAGGAUCUU GGUGAG | 18 | 18 |
| CUCAUAGGCCUGGAG UUUAUU | 7 | 7 | AAUAAACUCCAGGCCUA UGAGGG | 19 | 19 |
| GGCCUGGAGUUUAUU CGGAAA | 8 | 8 | UUUCCGAAUAAACUCCA GGCCUA | 20 | 20 |
| CAGCACCUGCUUUGU | 9 | 9 | UGUGACACAAAGCAGGU | 21 | 21 |
| GUCACA | | | GCUGCA | | |
| GGUCUGGAAUGCAAA GUCAAG | 10 | 10 | CUUGACUUUGCAUUCCA GACCUG | 22 | 22 |
| GCGGAGAUGCUUCUA AGGCAU | 11 | 11 | AUGCCUUAGAAGCAUCU CCGCCA | 23 | 23 |
| GGGUUUUGUAGCAUU UUUAUU | 12 | 12 | AAUAAAAAUGCUACAAA ACCCAG | 24 | 24 |

In some implementations of the present disclosure, the sense strand of the double-stranded RNAi agent has substitutions, additions, or deletions of 1 to 3 nucleotides compared with any one of the sequences shown in SEQ ID NO: 1 to 12.

In some implementations of the present disclosure, the antisense strand of the double-stranded RNAi agent has substitutions, additions, or deletions of 1 to 3 nucleotides compared with any one of the sequences shown in SEQ ID NO: 13 to 24.

In some implementations of the present disclosure, the double-stranded RNAi agent contains at least one modified nucleotide, and the modified nucleotide is selected from any one of groups consisting of deoxynucleotide, a 3'-end deoxythymidine (dT) nucleotide, a 2'-O-methyl modified nucleotide, a 2'-fluorine modified nucleotide, a 2'-deoxy modified nucleotide, a locked nucleotide, an unlock nucleotide, a configuration-restricted nucleotide, a restricted ethyl nucleotide, a base-free nucleotide, a 2'-amino modified nucleotide, a 2'-O-allyl modified nucleotide, a 2'-C-alkyl modified nucleotide, a 2'-hydroxy modified nucleotide, a 2'-O-methoxyethyl modified nucleotide, a 2'-O-alkyl modified nucleotide, a morpholine nucleotide, an aminophosphonate, a nucleotide containing a non-natural base, a tetrahydropyran-modified nucleotide, a 1,5-anhydrohexitol-modified nucleotide, a cyclohexenyl-modified nucleotide, a nucleotide containing a phosphorothioate group, a nucleotide containing a methylphosphonate group, a nucleotide containing 5'-phosphate, and a nucleotide containing a 5'-phosphate mimetic, or a combination of at least two of the above.

In some implementations of the present disclosure, a mode of modifying the nucleotides of the sense strand and antisense strand of the double-stranded RNAi agent is chemical modification of a 2' position of nucleotide ribose.

In some implementations of the present disclosure, the chemical modification of the 2' position of the nucleotide ribose of the double-stranded RNAi agent is selected from any one of groups consisting of 2'-methoxy modification, 2'-O-methoxyethyl modification, 2'-fluoro modification, 2'-benzyloxy modification, 2'-methylcarbonylamino modification, and 2'-pyridinylmethoxy modification, or a combination of at least two of the above.

In some implementations of the present disclosure, the chemical modification of the 2' position of each nucleotide ribose of the double-stranded RNAi agent is selected from a combination of 2'-methoxy and 2'-fluoro.

In some implementations of the present disclosure, the chemical modification mode of the 2' position of each nucleotide ribose of the double-stranded RNAi agent is that, odd positions of the sense strand are all modified with 2'-fluoro group, and even positions are all modified with 2'-methoxy group; and odd positions of the antisense strand are all modified with 2'-methoxy group, and even positions are all modified with 2'-fluoro group.

In some implementations of the present disclosure, nucleotide monomers of the double-stranded RNAi agent are connected through a 3',5'-phosphodiester bond.

In some implementations of the present disclosure, the nucleotide monomers of the double-stranded RNAi agent are connected through a thio-modified 3',5'-phosphodiester bond.

In some implementations of the present disclosure, the double-stranded RNAi agent has the following modification.

The antisense strand has any one of the following A, B, or C modification mode.

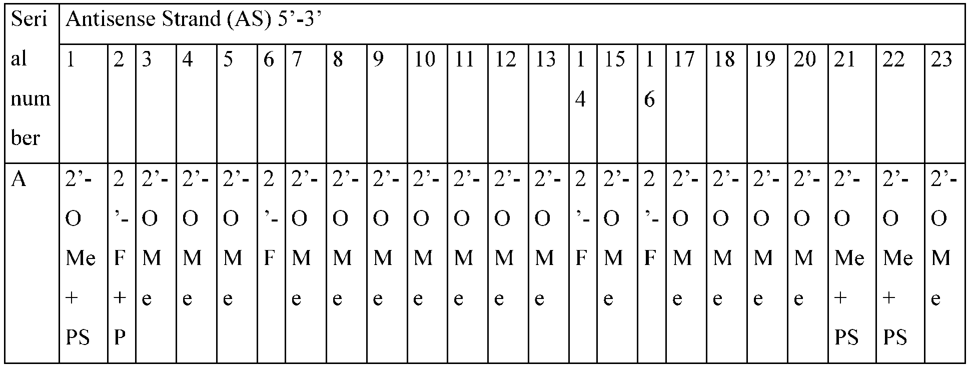

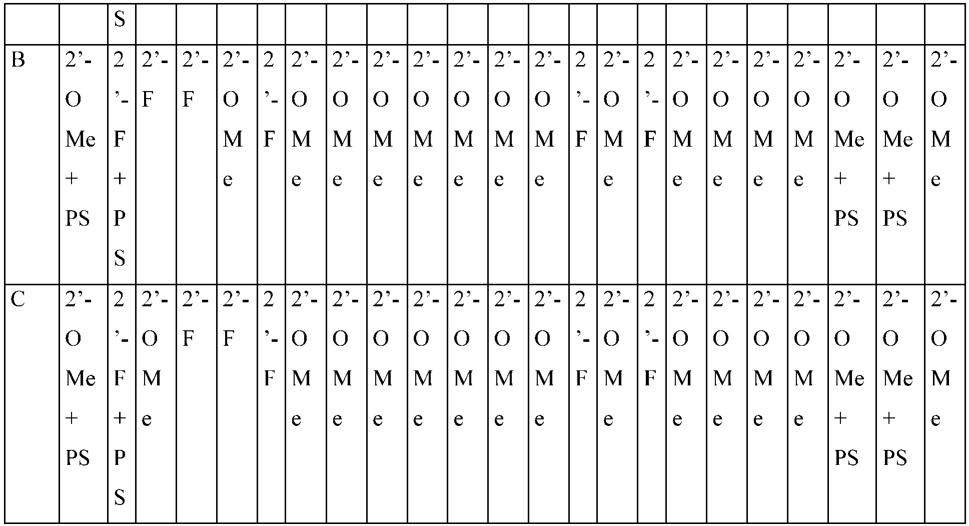

The sense strand has any one of the following a or b modification mode.

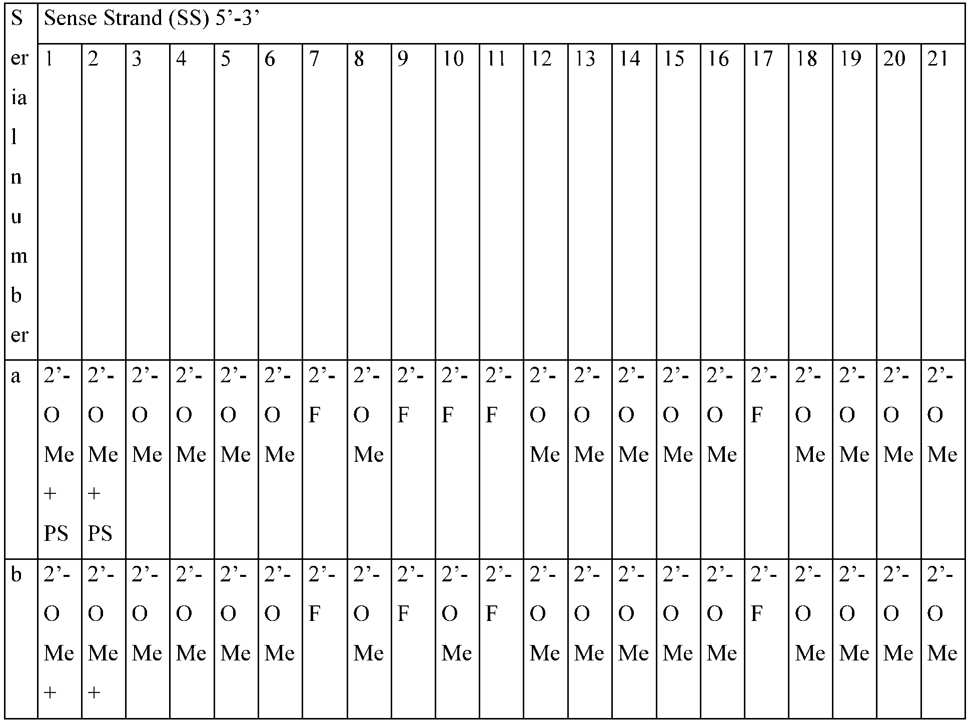

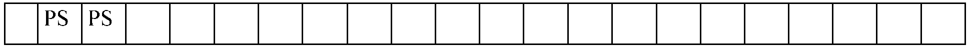

In the table, 2'-OMe is 2'-methoxy; 2'-F is 2'-fluoro; PS is a phosphorothioate skeleton.

In some implementations of the present disclosure, the antisense strand of the double-stranded RNAi agent has any one of the following (I)-(X) modification modes.
(I) In a 5'-3' direction, the 2nd, 3rd, 4th, 6th, 14th, and 16th positions are all modified with 2' position fluoro group, the 7th position is modified with GNA group, and other positions are all modified with 2' position methoxy group; and the nucleotides at the 1st and 2nd positions, the nucleotides at the 2nd and 3rd positions, the nucleotides at the last 1st and 2nd positions, and the nucleotides at the last 2nd and 3rd positions are connected to each other through a phosphorothioate bond.
(II) In the 5'-3' direction, the 2nd, 3rd, 4th, 6th, 14th, and 16th positions are all modified with 2' position fluoro group, and other positions are all modified with 2' position methoxy group; and the nucleotides at the 1st and 2nd positions, the nucleotides at the 2nd and 3rd positions, the nucleotides at the last 1st and 2nd positions, and the nucleotides at the last 2nd and 3rd positions are connected to each other through the phosphorothioate bond.
(III) In the 5'-3' direction, the 2nd, 3rd, 4th, 6th, 14th, and 16th positions are all modified with 2' position fluoro group, and other positions are all modified with 2' position methoxy group; the nucleotides at the 1st and 2nd positions, the nucleotides at the 2nd and 3rd positions, the nucleotides at the last 1st and 2nd positions, and the nucleotides at the last 2nd and 3rd positions are connected to each other through the phosphorothioate bond; and the 1st position is a 5'-trans vinyl phosphonate nucleotide.
(IV) In the 5'-3' direction, the 2nd, 4th, 5th, 6th, 14th, and 16th positions are all modified with 2' position fluoro group, the 7th position is modified with GNA group, and other positions are all modified with 2' position methoxy group; the nucleotides at the 1st and 2nd positions, the nucleotides at the 2nd and 3rd positions, the nucleotides at the last 1st and 2nd positions, and the nucleotides at the last 2nd and 3rd positions are connected to each other through the phosphorothioate bond; and the 1st position is the 5'-trans vinyl phosphonate nucleotide.
(V) In the 5'-3' direction, the 2nd, 4th, 5th, 6th, 14th, and 16th positions are all modified with 2' position fluoro group, and other positions are all modified with 2' position methoxy group; and the nucleotides at the 1st and 2nd positions, the nucleotides at the 2nd and 3rd positions, the nucleotides at the last 1st and 2nd positions, and the nucleotides at the last 2nd and 3rd positions are connected to each other through the phosphorothioate bond.
(VI) In the 5'-3' direction, the 2nd, 4th, 5th, 6th, 14th, and 16th positions are all modified with 2' position fluoro group, and other positions are all modified with 2' position methoxy group; the nucleotides at the 1st and 2nd positions, the nucleotides at the 2nd and 3rd positions, the nucleotides at the last 1st and 2nd positions, and the nucleotides at the last 2nd and 3rd positions are connected to each other through the phosphorothioate bond; and the 1st position is the 5'-trans vinyl phosphonate nucleotide.
(VII) In the 5'-3' direction, the 2nd, 6th, 14th, and 16th positions are all modified with 2' position fluoro group, the 7th position is modified with GNA group, and other positions are all modified with 2' position methoxy group; and the nucleotides at the 1st and 2nd positions, the nucleotides at the 2nd and 3rd positions, the nucleotides at the last 1st and 2nd positions, and the nucleotides at the last 2nd and 3rd positions are connected to each other through the phosphorothioate bond.
(VIII) In the 5'-3' direction, the 2nd, 6th, 14th, and 16th positions are all modified with 2' position fluoro group, and other positions are all modified with 2' position methoxy group; and the nucleotides at the 1st and 2nd positions, the nucleotides at the 2nd and 3rd positions, the nucleotides at the last 1st and 2nd positions, and the nucleotides at the last 2nd and 3rd positions are connected to each other through the phosphorothioate bond.
(IX) In the 5'-3' direction, the 2nd, 6th, 14th, and 16th positions are all modified with 2' position fluoro group, and other positions are all modified with 2' position methoxy group; the nucleotides at the 1st and 2nd positions, the nucleotides at the 2nd and 3rd positions, the nucleotides at the last 1st and 2nd positions, and the nucleotides at the last 2nd and 3rd positions are connected to each other through the phosphorothioate bond; and the 1st position is the 5'-trans vinyl phosphonate nucleotide.
(X) In the 5'-3' direction, the 2nd, 4th, 5th, 6th, 8th, 10th, 12th, 14th, 16th, and 18th positions are all modified with 2' position fluoro group, and other positions are all modified with 2' position methoxy group; and the nucleotides at the 1st and 2nd positions, the nucleotides at the 2nd and 3rd positions, the nucleotides at the last 1st and 2nd positions, and the nucleotides at the last 2nd and 3rd positions are connected to each other through the phosphorothioate bond.

In some implementations of the present disclosure, the sense strand of the double-stranded RNAi agent has any one of the following (1)-(3) modification modes.
(1) In the 5'-3' direction, the 7th, 9th, 10th, 11th, and 17th positions are all modified with 2' position fluoro group, and other positions are all modified with 2' position methoxy group; and the nucleotides at the 1st and 2nd positions, and the nucleotides at the 2nd and 3rd positions are connected to each other through the phosphorothioate bond.
(2) In the 5'-3' direction, the 7th, 9th, 11th, and 17th positions are all modified with 2' position fluoro group, and other positions are all modified with 2' position methoxy group; and the nucleotides at the 1st and 2nd positions, and the nucleotides at the 2nd and 3rd positions are connected to each other through the phosphorothioate bond.
(3) In the 5'-3' direction, the 7th and 9th positions are all 2' position fluoro group, and other positions are all modified with 2' position methoxy group; the nucleotides at the 1st and 2nd positions, and the nucleotides at the 2nd and 3rd positions are connected to each other through the phosphorothioate bond; and the 11th position is thymidine.

In some implementations of the present disclosure, the double-stranded RNAi agent has any one of the following modifications.

The antisense strand has the modification mode A, and the sense strand has the modification mode a.

The antisense strand has the modification mode B, and the sense strand has the modification mode a.

The antisense strand has the modification mode C, and the sense strand has the modification mode a.

The antisense strand has the modification mode B, and the sense strand has the modification mode b.

The antisense strand has the modification mode C, and the sense strand has the modification mode b.

In some implementations of the present disclosure, the antisense strand of the double-stranded RNAi agent has a modification group in second to eighth positions from a 5' end, herein the modification group is an Unlock Nucleic Acid (UNA), a Glycol Nucleic Acid (GNA), or DNA, and structures of the UNA and the GNA are as follows. 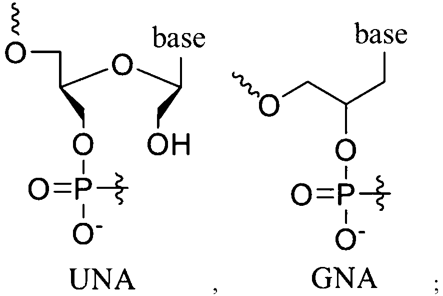 and the base is adenine, guanine, cytosine, thymine, or uracil.

In some implementations of the present disclosure, phosphorylation of a 5' position carbon atom of 5' end nucleotide glycoside of the modified antisense strand of the double-stranded RNAi agent includes, but is not limited to, the following 5' position phosphorylation groups: a 5'-vinyl phosphonate group (5'-E-VP), a 5'-methyl phosphonate group (5'-MP), a 5'-C-methyl phosphonate group, a 5'-phosphorothioate group (5'-PS), and a 5'-phosphate group (5'-P), with structures shown as follows.

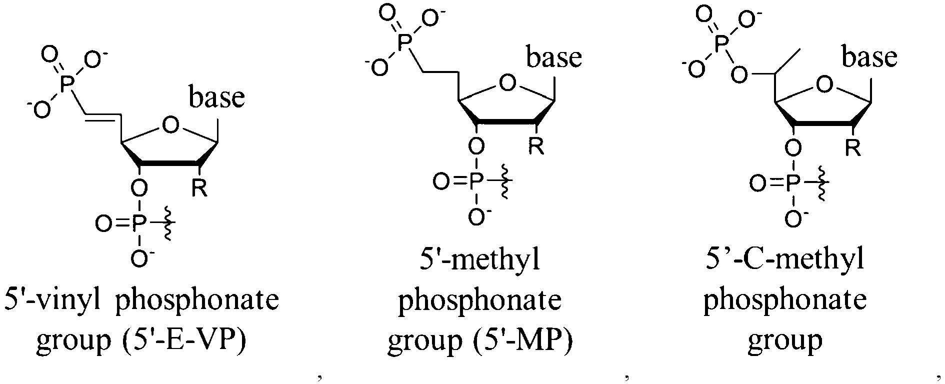

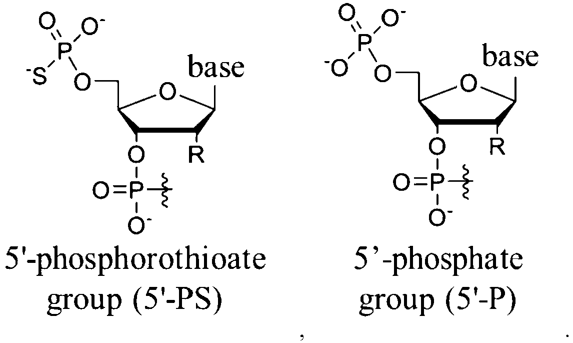

R is H, hydroxyl, amino, C₁₋₄ alkyl, aryl, C₁₋₄ alkoxy, C₁₋₄ alkylcarbonylamino, or halogen.

The base is adenine, guanine, cytosine, thymine, or uracil.

In some implementations of the present disclosure, the double-stranded RNAi agent contains any one of oligonucleotide double-stranded bodies formed by the pairing of modified sense strands and modified antisense strands in a table below (sequences being from Table 38 of the specification of CN117210468A).

| Sense Strand 5'-3' | Modification sequence SEQ ID NO:/corresponding basic sequence SEQ ID NO: | Corresponding sequence ID number in CN117210468A | Antisense Strand 5'-3' | Modification sequence SEQ ID NO:/corresponding basic sequence SEQ ID NO: | Corresponding sequence ID number in CN117210468A |
|---|---|---|---|---|---|
| Ams-Ams-Gm-Am-Um-Cm-Cf-Um-Gf-Cf-Af-Um-Gm-Um-Cm-Um-Uf-Cm-Cm-Am-Um | 25/1 | 337 | Ams-Ufs-Gm-Gm-Am-Af-Ggna-Am-Cm-Am-Um-Gm-Cm-Af-Gm-Gf-Am-Um-Cm-Um-Ums-Gms-Gm | 32/13 | 427 |
| Ams-Ams-Gm-Am-Um-Cm-Cf-Um-Gf-Cf-Af-Um-Gm-Um-Cm-Um-Uf-Cm-Cm-Am-Um | 25/1 | 337 | AmsEVP-Ufs-Gm-Gm-Am-Af-Gm-Am-Cm-Am-Um-Gm-Cm-Af-Gm-Gf-Am-Um-Cm-Um-Ums-Gms-Gm | 33/13 | 428 |
| Ums-Gms-Gm-Am-Gm-Gm-Cf-Um-Uf-Af-Gf-Cm-Um-Um-Um-Cm-Uf-Gm-Gm-Am-Um | 26/2 | 342 | Ams-Ufs-Cf-Cf-Am-Gf-Am-Am-Am-Gm-Cm-Um-Am-Af-Gm-Cf-Cm-Um-Cm-Cm-Ams-Ums-Um | 34/14 | 381 |
| Ums-Gms-Gm-Am-Gm-Gm-Cf-Um-Uf-Af-Gf-Cm-Um-Um-Um-Cm-Uf-Gm-Gm-Am-Um | 26/2 | 342 | Ams-Ufs-Cf-Cf-Am-Gf-Agna-Am-Am-Gm-Cm-Um-Am-Af-Gm-Cf-Cm-Um-Cm-Cm-Ams-Ums-Um | 35/14 | 430 |
| Ums-Gms-Gm-Am-Gm-Gm-Cf-Um-Uf-Af-Gf-Cm-Um-Um-Um-Cm-Uf-Gm-Gm-Am-Um | 26/2 | 342 | AmsEVP-Ufs-Cf-Cf-Am-Gf-Am-Am-Am-Gm-Cm-Um-Am-Af-Gm-Cf-Cm-Um-Cm-Cm-Ams-Ums-Um | 36/14 | 431 |
| Cms-Ums-Um-Um-Um-Gm-Uf-Am-Af-Cf-Uf-Um-Gm-Am-Am-Gm-Af-Um-Am-Um-Um | 27/3 | 347 | Ams-Afs-Um-Am-Um-Cf-Um-Um-Cm-Am-Am-Gm-Um-Uf-Am-Cf-Am-Am-Am-Am-Gms-Cms-Am | 37/15 | 384 |
| Cms-Ums-Um-Um-Um-Gm-Uf-Am-Af-Cf-Uf-Um-Gm-Am-Am-Gm-Af-Um-Am-Um-Um | 27/3 | 347 | AmsEVP-Afs-Um-Af-Uf-Cf-Um-Um-Cm-Am-Am-Gm-Um-Uf-Am-Cf-Am-Am-Am-Am-Gms-Cms-Am | 38/15 | 437 |
| Cms-Ums-Um-Um-Um-Gm-Uf-Am-Af-Cf-Uf-Um-Gm-Am-Am-Gm-Af-Um-Am-Um-Um | 27/3 | 347 | AmsEVP-Afs-Um-Af-Uf-Cf-Ugna-Um-Cm-Am-Am-Gm-Um-Uf-Am-Cf-Am-Am-Am-Am-Gms-Cms-Am | 39/15 | 438 |
| Cms-Ums-Um-Um-Um-Gm-Uf-Am-Af-Cm-Uf-Um-Gm-Am-Am-Gm-Af-Um-Am-Um-Um | 28/3 | 348 | Ams-Afs-Uf-Af-Um-Cf-Um-Um-Cm-Am-Am-Gm-Um-Uf-Am-Cf-Am-Am-Am-Am-Gms-Cms-Am | 40/15 | 385 |
| Gms-Ams-Am-Gm-Am-Um-Af-Um-Uf-Uf-Af-Um-Um-Cm-Um-Gm-Gf-Gm-Um-Um-Um | 29/4 | 352 | AmsEVP-Afs-Am-Cf-Cf-Cf-Am-Gm-Am-Am-Um-Am-Am-Af-Um-Af-Um-Cm-Um-Um-Cms-Ams-Am | 41/16 | 448 |
| Gms-Ams-Am-Gm-Am-Um-Af-Um-Uf-Um-Af-Um-Um-Cm-Um-Gm-Gf-Gm-Um-Um-Um | 30/4 | 353 | Ams-Afs-Am-Cf-Cf-Cf-Am-Gm-Am-Am-Um-Am-Am-Af-Um-Af-Um-Cm-Um-Um-Cms-Ams-Am | 42/16 | 390 |
| Gms-Ams-Am-Gm-Am-Um-Af-Um-Uf-Um-Af-Um-Um-Cm-Um-Gm-Gf-Gm-Um-Um-Um | 30/4 | 353 | AmsEVP-Afs-Am-Cf-Cf-Cf-Am-Gm-Am-Am-Um-Am-Am-Af-Um-Af-Um-Cm-Um-Um-Cms-Ams-Am | 41/16 | 448 |
| Gms-Ums-Um-Um-Um-Gm-Uf-Am-Gf-Cf-Af-Um-Um-Um-Um-Um-Af-Um-Um-Am-Am | 31/5 | 357 | Ums-Ufs-Af-Af-Um-Af-Am-Am-Am-Am-Um-Gm-Cm-Uf-Am-Cf-Am-Am-Am-Am-Cms-Cms-Cm | 43/17 | 393 |
| Gms-Ums-Um-Um-Um-Gm-Uf-Am-Gf-Cf-Af-Um-Um-Um-Um-Um-Af-Um-Um-Am-Am | 31/5 | 357 | UmsEVP-Ufs-Am-Af-Uf-Af-Am-Am-Am-Am-Um-Gm-Cm-Uf-Am-Cf-Am-Am-Am-Am-Cms-Cms-Cm | 44/17 | 446 |
| Gms-Ums-Um-Um-Um-Gm-Uf-Am-Gf-Cf-Af-Um-Um-Um-Um-Um-Af-Um-Um-Am-Am | 31/5 | 357 | UmsEVP-Ufs-Am-Af-Uf-Af-Agna-Am-Am-Am-Um-Gm-Cm-Uf-Am-Cf-Am-Am-Am-Am-Cms-Cms-Cm | 45/17 | 447 |

In some implementations of the present disclosure, the conjugate is conjugated by the double-stranded RNAi agent shown in formula (IIA) or (IIB) and the YK-GAL-401, YK-GAL-402, YK-GAL-403, YK-GAL-404, YK-GAL-405, or YK-GAL-406 described above; and the double-stranded RNAi agent contains any one of oligonucleotide double-stranded bodies formed by the pairing of the following sense strands and the antisense strands.
(1) The sense strand has a sequence shown in SEQ ID NO: 29, and the antisense strand has a sequence shown in SEQ ID NO: 41.
(2) The sense strand has a sequence shown in SEQ ID NO: 30, and the antisense strand has a sequence shown in SEQ ID NO: 42.
(3) The sense strand has a sequence shown in SEQ ID NO: 30, and the antisense strand has a sequence shown in SEQ ID NO: 41.

In some implementations of the present disclosure, the conjugate is conjugated by the double-stranded RNAi agent and the YK-GAL-401, YK-GAL-402, YK-GAL-403, YK-GAL-404, YK-GAL-405, or YK-GAL-406 described above; and the double-stranded RNAi agent contains the oligonucleotide double-stranded body formed by the pairing of the sense strand shown in SEQ ID NO: 30 and the antisense strand shown in SEQ ID NO: 41.

In some implementations of the present disclosure, the conjugate is conjugated by the double-stranded RNAi agent and the YK-GAL-401, YK-GAL-402, YK-GAL-403, YK-GAL-404, YK-GAL-405, or YK-GAL-406 described above; and the double-stranded RNAi agent has the following sequences.

Sense strand: 5'-Cms-Ums-Am-Gm-Am-Cm-Cf-Um-Gf-Um-dT-Um-Um-Gm-Cm-Um-Um-Um-Um-Gm-Um-3' (SEQ ID NO: 46).

Antisense strand: 5'-Ams-Cfs-Am-Af-Af-Af-Gm-Cf-Am-Af-Am-Af-Cm-Af-Gm-Gf-Um-Cf-Um-Am-Gms-Ams-Am-3' (SEQ ID NO: 47).

In some implementations of the present disclosure, the conjugate is conjugated by the double-stranded RNAi agent and the YK-GAL-401, YK-GAL-402, YK-GAL-403, YK-GAL-404, YK-GAL-405, or YK-GAL-406 described above; and the double-stranded RNAi agent has the following sequences.

Sense strand: 5'-Cms-Ums-Am-Gm-Am-Cm-Cf-Um-Gf-Um-dT-Um-Um-Gm-Cm-Um-Um-Um-Um-Gm-Um-3' (SEQ ID NO: 46).

Antisense strand: 5'-Ams-Cfs-Am-Af-Af-Af-Gm-Cf-Am-Af-Am-Af-Cm-Af-Gm-Gf-Um-Cf-Um-Am-Gms-Ams-Am-3' (SEQ ID NO: 47).

In some implementations of the present disclosure, the conjugate shown in formula (IIA) is any one of the following conjugates. 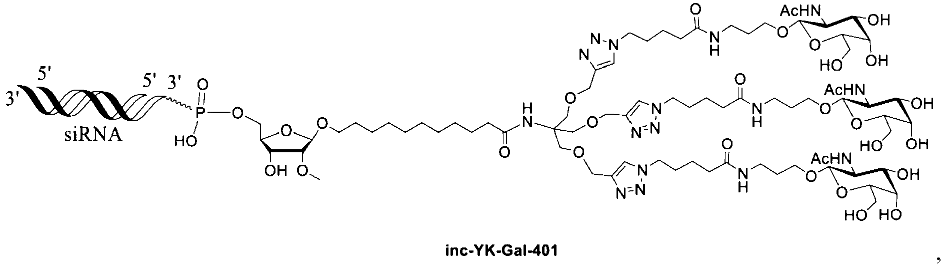 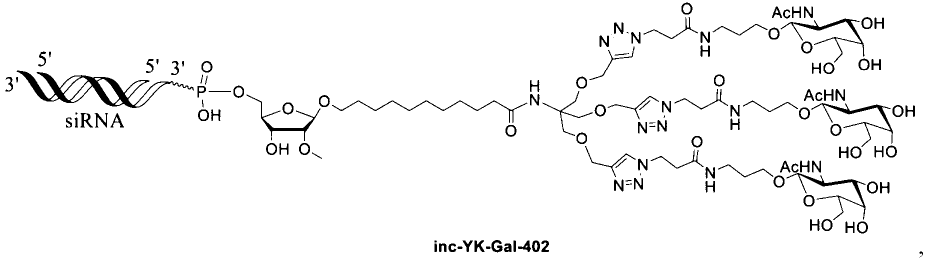 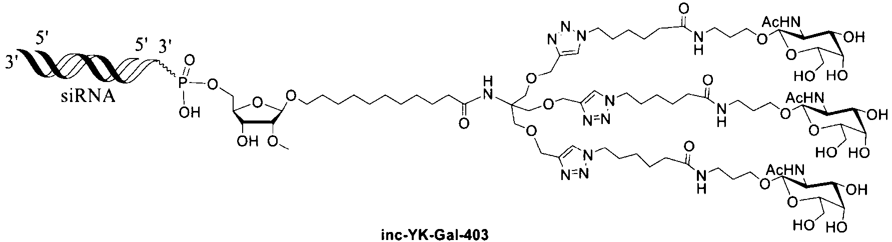 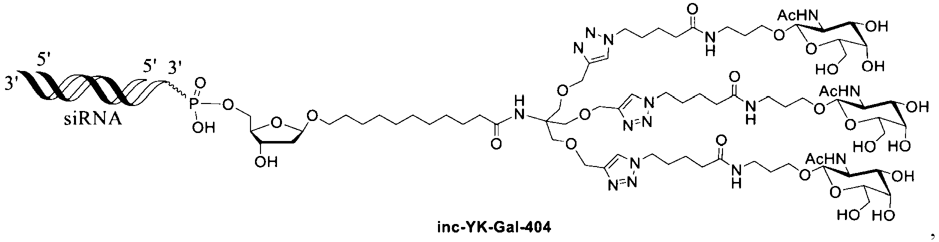 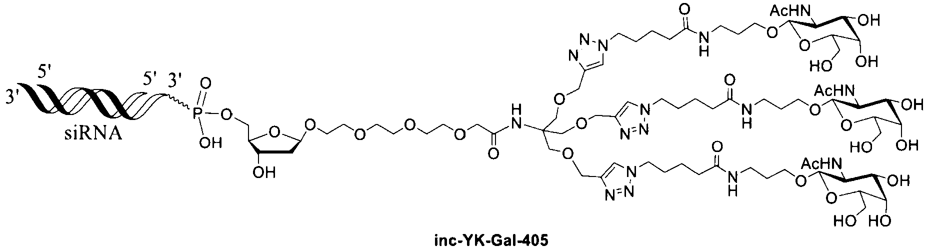 or 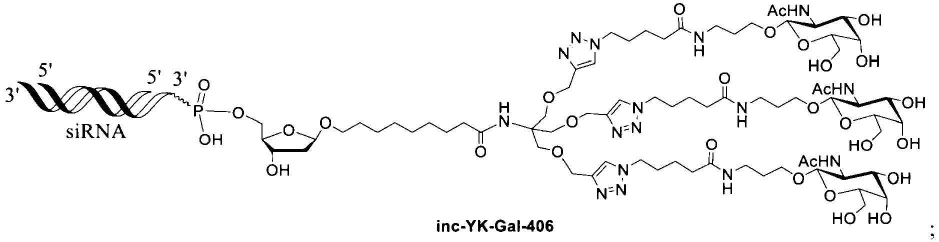 wherein, the sense strand of siRNA has a sequence shown in SEQ ID NO: 46, and the antisense strand of the siRNA has a sequence shown in SEQ ID NO: 47.

The present disclosure further provides a pharmaceutical composition, containing the conjugate shown in formula (IIA) or (IIB) described above and at least one pharmaceutically acceptable excipient.

The present disclosure further provides an application of the conjugate shown in formula (IIA) or (IIB) described above or the pharmaceutical composition described above for preparation of medicine for treating and/or preventing pathological conditions or diseases caused by expression of specific genes in liver cells.

In some implementations of the present disclosure, the diseases caused by the expression of the specific genes in the liver cells are selected from a chronic liver disease, hepatitis, a liver fibrosis disease, a hepatic proliferative disease, dyslipidemia, and a complement-related disease.

The present disclosure further provides a method for inhibiting expression of specific genes in liver cells. The method includes: an effective dose of the conjugate shown in formula (IIA) or (IIB) is in contact with the liver cells.

The present disclosure further provides a kit. The kit includes the conjugate shown in formula (IIA) or (IIB).

All publications and patents referred to in the present disclosure are hereby incorporated by reference in their entirety. If the uses or terms used in any of the publications and patents incorporated by reference conflict with the uses or terms used in the present disclosure, then the uses and terms of the present disclosure prevail.

The section headings used herein are for the sole purpose of organizing the article and should not be construed as a limitation on the subject matter described.

Unless otherwise specified, all technical terms and scientific terms used herein have the ordinary meaning of the field in which protection of the subject matter is claimed. Where more than one definition of a term exists, the definition herein shall prevail.

Except as noted in working embodiments or otherwise, all numbers of quantitative properties, such as dosage, stated in the specification and claims should be understood to be modified by the term "appropriate" or "about" in all cases. It should also be understood that any numerical range enumerated in the present disclosure is intended to include all sub-ranges within the range and any combination of the various endpoints of the range or sub-ranges.

As used in the present disclosure, the words "comprising", "containing" or "including" and the like are intended to mean that the elements appearing in front of the word encompass the elements appearing in the enumeration following the word, as well as their equivalents, and do not exclude undocumented elements. As used herein, the terms "containing" or "comprising (including)" may be open, semi-closed, and closed. In other words, the term also includes "consisting essentially of', or "consisting of".

The "hydroxyl protecting group" in the present disclosure refers to a protecting group that is generally used for protecting the hydroxyl of a ribose structure in synthesis of RNA or derivatives thereof, such as acetyl, phenoxyacetyl, pivaloyl, benzyl, 4-methoxybenzyl, benzoyl, triphenylmethyl, 4,4'-dimethoxytrityl (DMTr), monomethoxytrityl (MMTr), 9-phenylxanthen-9-yl, 9-(p-tolyl)-xanthen-9-yl, trimethylsilyl, tert butyldimethylsilyl (TBDMS), cyanomethoxymethyl, 2-(cyanoethoxy)ethyl, cyanoethoxymethyl, and the like, and preferably the 4,4'-dimethoxytrityl.

The "phosphorus-containing reactive group" in the present disclosure refers to a phosphorus-containing group that reacts with the hydroxyl or amino contained in another molecule, especially another nucleotide unit or another nucleotide analogue through a nucleophilic attack reaction. Generally, such reaction generates an ester nucleoside bond for connecting the nucleotide unit or nucleotide analogue with another nucleotide unit or nucleotide analogue. These phosphorus-containing reactive groups are known in the art and contain phosphorus atoms in a PIII or PV valence state. The phosphorus-containing reactive group includes, but is not limited to, phosphoramidite, H-phosphonate, phosphotriester, and a phosphorus-containing chiral auxiliary, such as

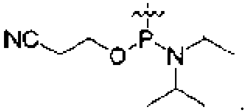

The CPG and polystyrene (highly cross-linked polystyrene microspheres) in the present disclosure are solid carriers for oligonucleotide synthesis, are insoluble particles bound to the oligonucleotide during synthesis, and are commercially available.

The term "halogen" refers to fluorine, chlorine, bromine, or iodine.

The term "alkyl" refers to straight or branched chain alkyl having a specified number of carbon atoms (e.g., C₁-C₆). The alkyl includes, but is not limited to, methyl, ethyl, n-propyl, isopropyl, n-butyl, t-butyl, isobutyl, sec-butyl, n-pentyl, n-hexyl, n-heptyl, n-octyl, and the like.

The term "alkoxy" refers to a group R^{X}-O-, herein R^{X} is the alkyl as defined above.

The term "pharmaceutically acceptable salt" refers to a salt obtained by reacting a compound with a pharmaceutically acceptable (relatively non-toxic, safe and suitable for the use of patients) acid or base. When the compound contains a relatively-acidic functional group, an alkali addition salt may be obtained by means of contacting a free form of the compound with a sufficient amount of the pharmaceutically acceptable base in a suitable inert solvent. The pharmaceutically acceptable alkali addition salt includes, but is not limited to, a sodium salt, a potassium salt, a calcium salt, an aluminum salt, a magnesium salt, a bismuth salt, an ammonium salt, and the like. When the compound contains a relatively-basic functional group, an acid addition salt may be obtained by means of contacting a free form of the compound with a sufficient amount of the pharmaceutically acceptable acid in a suitable inert solvent. The pharmaceutically acceptable acid addition salt includes, but is not limited to, hydrochloride, sulfate, methanesulfonate, and the like.

The term "pharmaceutically acceptable excipient" in the present disclosure refers to all substances, other than the active ingredient, included in a pharmaceutical preparation.

The term "treatment" refers to any one of the following situations: (1) one or more biological manifestations mitigating illness; (2) one or more points interfering with biological cascade that causes disease; and (3) progression of one or more biological manifestations mitigating illness.

The term "prevention" refers to reducing the risk of disease.

The oligonucleotide in the present disclosure includes single-stranded oligonucleotide (e.g., ASO) and double-stranded oligonucleotide (e.g., siRNA).

The oligonucleotide in the present disclosure includes natural oligonucleotide and chemically-modified oligonucleotide. The chemical modification here includes nucleoside modification (including sugar modification and nucleobase modification) and inter-nucleoside bonding modification. The chemical modification of the oligonucleotide does not include situations where there is a difference only in a nucleobase sequence. Natural here refers to the case corresponding to naturally occurring RNA or DNA. On the basis of conforming to common knowledge in the field, the foregoing preferred conditions may be combined arbitrarily to obtain preferred embodiments of the present disclosure.

The reagents and raw materials used in the present disclosure are commercially available.

Positive and progressive effects of the present disclosure are that, the present disclosure designs a series of new GalNAc compounds, which has a completely different chemical structure from a GalNAc compound in the prior art. For the GalNAc compound designed in the present disclosure, the triazole structure is introduced in a linker arm. By means of the advantage of click chemistry, a series of new GalNAc compounds containing triazole structures may be constructed rapidly. The GalNAc oligonucleotide conjugate prepared by the GalNAc compound of the present disclosure may realize efficient liver targeting delivery, and activity is significantly improved compared with that of the representative GalNAc compounds in the prior art.
1. Compared with the GalNAc in the prior art, the oligonucleotide conjugates prepared by the GalNAc compounds YK-Gal-401 to YK-Gal-403 of the present disclosure shows a significantly higher inhibition rate of PCSK9 protein expression in mouse serum. For example, the inhibition rate of inc-YK-Gal-401 on Day 7 is increased by 21.5%, 14.5%, and 12.7% compared with inc-YK-Gal-301, inc-GalNAc 1b, and inc-NAG0052, and the inhibition rate on Day 14 is increased by 17.3%, 10.4%, and 10.8%, respectively.
   1) The siRNA conjugated by the GalNAc compound of the present disclosure has a significant difference in the inhibition rate of PCSK9 protein expression in the mouse serum, and YK-Gal-401 to YK-Gal-403 are significantly higher than YK-Gal-404 to 406. For example, inc-YK-Gal-401 on Day 7 and Day 14 is 20.7% and 16.0% higher than inc-YK-Gal-404.
   2) Compared with the GalNAc compound in the prior art, the siRNA conjugated by the GalNAc compounds YK-Gal-401 to YK-Gal-403 of the present disclosure shows a significantly higher inhibition rate of PCSK9 protein expression in the mouse serum. For example, inc-YK-Gal-401 on Day 7 and Day 14 is 21.5% and 17.3% higher than inc-YK-Gal-301.
   3) The GalNAc compounds having similar structures and siRNA conjugates show a significant difference in the inhibition rate of PCSK9 protein expression in the mouse serum. Therefore, not all the GalNAc compounds having similar chemical structures have the close efficiency of delivering the oligonucleotide, the inhibition rate of PCSK9 protein expression in the mouse serum by the GalNAc oligonucleotide conjugates prepared by the compounds is not consistent and may be significantly variable.
2. Compared with the GalNAc in the prior art, the oligonucleotide conjugates prepared by the GalNAc compounds YK-Gal-401 to YK-Gal-403 show a significantly higher inhibition rate of PCSK9 gene expression in mouse liver. For example, the inhibition rate of inc-YK-Gal-401 is increased by 21.1% and 9.8% respectively compared with inc-YK-Gal-301 and inc-GalNAc 1b, and thus is significantly increased.
   1) The siRNA conjugated by the GalNAc compound of the present disclosure has a significant difference in the inhibition rate of PCSK9 gene expression in the mouse liver, and YK-Gal-401 to YK-Gal-403 are significantly higher than YK-Gal-404 to YK-Gal-406. For example, inc-YK-Gal-401 is 19.4% higher than inc-YK-Gal-404.
   2) Compared with the GalNAc compound in the prior art, the siRNA conjugated by the GalNAc compounds YK-Gal-401 to YK-Gal-403 of the present disclosure shows a significantly higher inhibition rate of PCSK9 gene expression in the mouse liver. For example, inc-YK-Gal-401 is 21.1% higher than inc-YK-Gal-301.
   3) The GalNAc compounds having similar structures and siRNA conjugates show a significant difference in the inhibition rate of PCSK9 gene expression in the mouse liver. Therefore, not all the GalNAc compounds having similar chemical structures have the close efficiency of delivering the oligonucleotide, the inhibition rate of PCSK9 gene expression in the mouse liver by the GalNAc oligonucleotide conjugates prepared by the compounds is not consistent and may be significantly variable.
3. Compared with the GalNAc in the prior art, the oligonucleotide conjugates prepared by the GalNAc compounds YK-Gal-401 to YK-Gal-403 of the present disclosure significantly elevate a LDL-C lowering level in the mouse serum. For example, the LDL-C lowering levels in the mice in the inc-YK-Gal-401 group on Day 14 are increased by 36.4%, 20.6%, and 17.8% respectively compared with those in the inc-YK-Gal-301, inc-GalNAc 1b, and NAG0052 groups, and thus are significantly increased.
   1) The siRNA conjugated by the GalNAc compound of the present disclosure has a significant difference in the reducing of the LDL-C level in the mouse serum, and the reduced level in YK-Gal-401 to YK-Gal-403 is significantly higher than that in YK-Gal-404 to 406. For example, inc-YK-Gal-401 on Day 7 and Day 14 is 33.0% and 36.0% higher than inc-YK-Gal-404, which is significantly increased.
   2) Compared with the GalNAc compound in the prior art, the siRNA conjugated by the GalNAc compounds YK-Gal-401 to YK-Gal-403 of the present disclosure significantly elevates the LDL-C lowering level in the mouse serum. For example, inc-YK-Gal-401 on Day 7 and Day 14 is 33.4% and 36.4% higher than inc-YK-Gal-301.
   3) The GalNAc compounds having similar structures and siRNA conjugates show a significant difference in the LDL-C lowering level in the mouse serum. Therefore, not all the GalNAc compounds having similar chemical structures have the close efficiency of delivering the oligonucleotide, the inhibition rate of PCSK9 protein expression in the mouse serum by the GalNAc oligonucleotide conjugates prepared by the compounds is not consistent and may be significantly variable.
4. The compound designed in the present disclosure may efficiently deliver the oligonucleotide to the liver.

For example, the liver fluorescence intensity at 4 hours in the inc-YK-Gal-401, inc-YK-Gal-402, and inc-YK-Gal-403 groups may respectively reach 9.03E+09, 8.79E+09, and 8.92E+09, and respectively reach 9.11E+09, 8.95E+09, and 9.01E+09 at 8 hours, indicating that the GalNAc compound designed in the present disclosure may efficiently deliver the oligonucleotide to animal liver.

### BRIEF DESCRIPTION OF DRAWINGS

Fig. 1 shows an inhibition rate of PCSK9 protein expression in mouse serum at 7 and 14 days after administration in each test medicine group (inc-YK-Gal-401, inc-YK-Gal-402, inc-YK-Gal-403, inc-YK-Gal-404, inc-YK-Gal-405, inc-YK-Gal-406, inc-YK-Gal-301, inc-GalNAc 1b, and inc-NAG0052).
Fig. 2 shows an inhibition rate of PCSK9 gene expression in mouse liver at 14 days after administration in each test medicine group (inc-YK-Gal-401, inc-YK-Gal-402, inc-YK-Gal-403, inc-YK-Gal-404, inc-YK-Gal-405, inc-YK-Gal-406, inc-YK-Gal-301, inc-GalNAc 1b, and inc-NAG0052).
Fig. 3 shows a LDL-C lowering level in mouse serum at 7 and 14 days after administration in each test medicine group (inc-YK-Gal-401, inc-YK-Gal-402, inc-YK-Gal-403, inc-YK-Gal-404, inc-YK-Gal-405, inc-YK-Gal-406, inc-YK-Gal-301, ine-GalNAc 1b, and inc-NAG0052).
Fig. 4 shows fluorescence intensity in mouse liver and kidney at 4 hours and 8 hours after administration in each test medicine group (inc-YK-Gal-401, inc-YK-Gal-402, inc-YK-Gal-403, inc-YK-Gal-404, inc-YK-Gal-405, inc-YK-Gal-406, inc-YK-Gal-301, inc-GalNAc 1b, and inc-NAG0052).

### DESCRIPTION OF EMBODIMENTS

In order to make objectives, technical solutions, and advantages of the embodiments of the present disclosure clearer, the technical solutions in the embodiments of the present disclosure will be clearly and completely described below. It is apparent that the described embodiments are part of the embodiments of the present disclosure, not all the embodiments. On the basis of the described embodiments in the present disclosure, all other embodiments obtained by those of ordinary skill in the art without creative work shall fall within the protection scope of the present disclosure.

The present disclosure may be implemented in other specific forms without departing from the essential properties of the present disclosure. It should be understood that any and all implementations of the present disclosure may be combined with technical features in any other implementations or a plurality of other implementations to obtain additional implementations, provided there is no conflict. The present disclosure includes additional implementations obtained in such combinations.

The present disclosure is further described below with reference to embodiments. However, the present disclosure is not limited to the following embodiments. The implementation conditions used in the embodiments may be further adjusted according to different requirements of the specific use, and the unspecified implementation conditions are conventional conditions in the industry. In the specific embodiments of the present disclosure, raw materials used are commercially available. All temperatures are given in degrees Celsius unless otherwise stated. The technical features involved in the various implementations of the present disclosure may be combined with each other as long as the technical features do not constitute a conflict with each other.

The following abbreviated letters represent the following reagents: DCM: Dichloromethane; PE: Petroleum Ether; EA: Ethyl Acetate; THF: Tetrahydrofuran; DMF: N,N-Dimethylformamide; ACN: Acetonitrile; KOH: potassium hydroxide; DCE: 1,2-Dichloroethane; TsOH•H₂O: p-Toluenesulfonic acid; EDCI: 1-Ethyl-3-(3-dimethylaminopropyl)carbodiimide hydrochloride; HOBt: 1-Hydroxybenzotriazole; THPTA: Tris(3-hydroxypropyltriazolylmethyl)amine; VCNa: Sodium ascorbate; TFA: Trifluoroacetic acid; TEAB: Triethylamine ammonium bicarbonate aqueous solution; HBTU: O-benzotriazole tetramethylurea hexafluorophosphate; DIPEA: N,N-Diisopropylethylamine; DMAP: 4-Dimethylaminopyridine; TMSOTf: Trimethylsilyl trifluoromethanesulfonate; Py: Pyridine; Ac₂O: Acetic anhydride; and CPG-NH₂: Amino modified controlled pore glass (from WuXi AppTec).

### Example 1: Synthesis of GalNAc compounds

### 1. Synthesis of YK-GAL-401

The synthesis route was as follows.

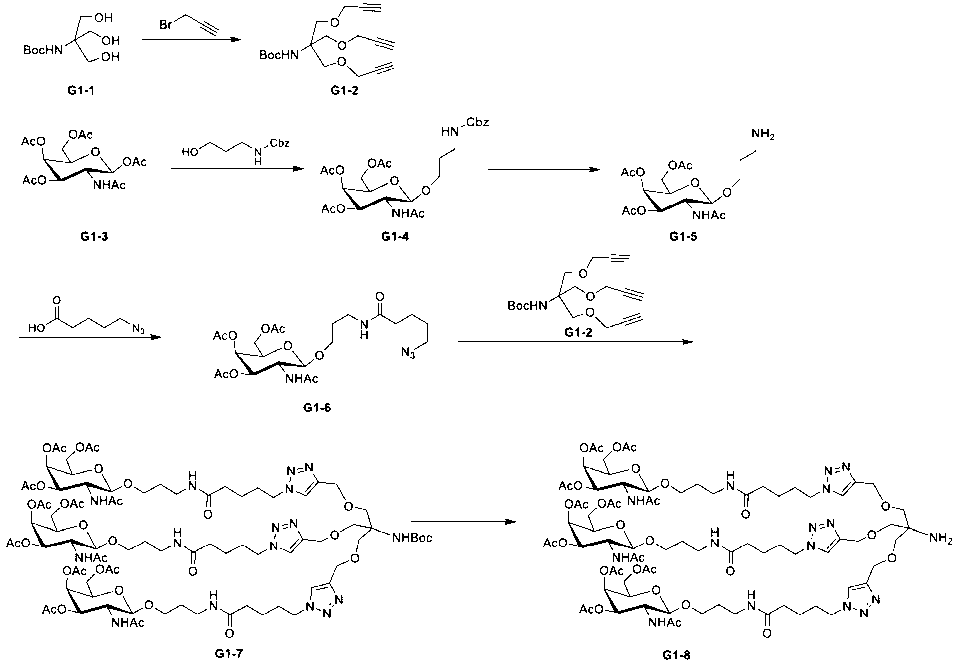

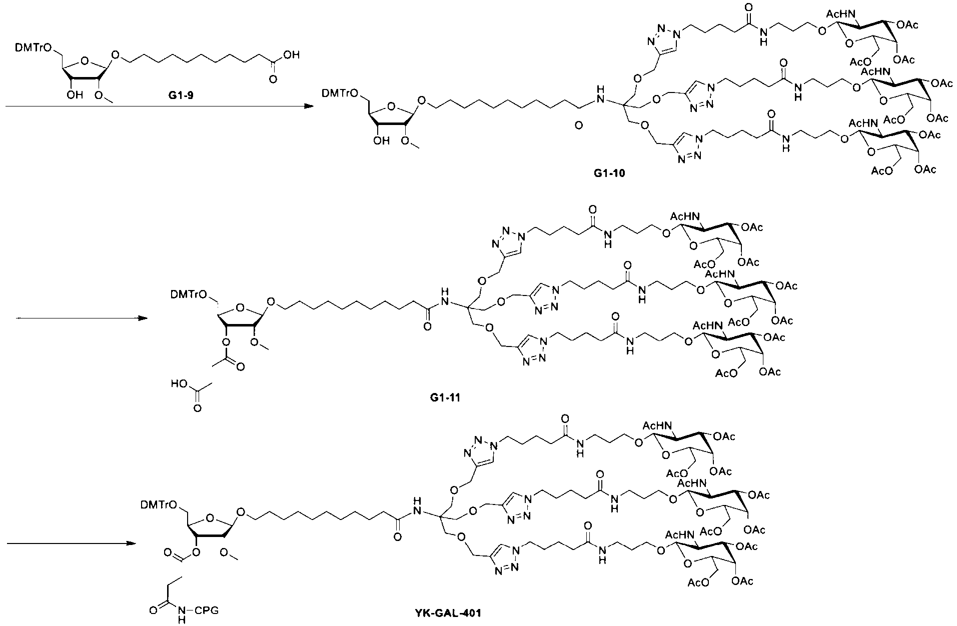

### S1: Synthesis of G1-2

The compound G1-1 (5.0 g, 22.6 mmol, 1.0 eq) was dissolved in anhydrous DMF (60.0 mL), and stirred at 0 °C. Then propargyl bromide (40.3 g, 271 mmol, 29.2 mL, 12.0 eq) was added, and finely-ground KOH (15.2 g, 271 mmol, 12.0 eq) was added to a reaction solution in batches within 15 minutes. The temperature was heated to 35 °C, and stirring was performed for 24 h. Sampling was performed for LC/MS detection, showing the disappearance of the raw material. EA (300 mL) was added to the reaction solution, and washing was performed with water (50 mL × 3). The organic phase was dried with Na₂SO₄, the solvent was removed through vacuum concentration after filtration, and the crude product was purified by a silica gel column (PE/EA) to obtain a yellow oily substance G1-2 (5.30 g, 15.8 mmol, 69.9 % yield). MS (ESI) m/z [M-H]⁻: 334.2; ¹H NMR: δ ppm 4.90 (s, 1 H), 4.14 (d, J=2.4, 6 H), 3.77 (s, 6 H), 2.43-2.41 (m, 3H), 1.41 (s, 9 H).

### S2: Synthesis of G1-4

The compound G1-3 (22.3 g, 57.3 mmol, 1.2 eq) was dissolved in DCE (200 mL), the 3Å molecular sieve (20.0 g) was added, and the temperature was cooled to 0 °C. Then, TMSOTf (21.2 g, 95.6 mmol, 17.3 mL, 2.0 eq) and benzyl n-(3-hydroxypropyl)carbamate (10.0 g, 47.8 mmol, 1.0 eq) were added in sequence at 0 °C. The temperature was heated to 15 °C, and stirring was continuously performed for 10 min. Sampling was performed for LC/MS detection, showing the disappearance of the raw material. The saturated sodium bicarbonate solution (200 mL) was added to the reaction solution, and DCM was used for extraction (200 mL × 3). The organic phase was dried with Na₂SO₄, the solvent was removed through vacuum concentration after filtration, and the crude product was purified by a silica gel column (PE/EA) to obtain a white solid G1-4 (13.4 g, 24.9 mmol, 52.1 % yield). MS (ESI) m/z [M + H]⁺: 539.1; ¹H NMR (400 MHz, CDCl₃) δ ppm 7.38-7.29 (m, 5 H),6.31 (d, J=8.7 Hz, 1 H), 5.33-5.28 (m, 1 H), 5.14-5.02 (m, 3 H), 4.98 (dd, J=11.1, 3.3 Hz, 1 H), 4.32 (d, J=8.5 Hz, 1 H), 4.16-4.09 (m, 3 H),3.98-3.93 (m, 1 H), 3.76 (t, J=6.7 Hz, 1 H), 3.57-3.48 (m, 1 H), 3.39 (td, J=9.9, 3.1 Hz, 1 H), 3.12-3.05 (m, 1 H), 2.13 (s, 3 H), 2.04 (s, 3H), 1.99 (s, 3 H), 1.93 (s, 3 H), 1.83-1.76 (m, 1 H), 1.65-1.57 (m, 1 H).

### S3: Synthesis of G1-5

The compound G1-4 (13.0 g, 24.1 mmol, 1.0 eq) and TsOH•H₂O (4.59 g, 24.1 mmol, 1.0 eq) were added to a THF (130 mL) solution of palladium carbon (6.50 g, 26.4 mmol, 1.09 eq). Under a hydrogen (15 psi) condition, the reaction solution was stirred for 2 h at 25 °C. Sampling was performed for LC/MS detection, showing the disappearance of the raw material. The reaction solution was filtered, and then the solvent was removed through vacuum concentration, so as to obtain a white solid crude product G1-5 (15.0 g). MS (ESI) m/z[M-H]⁻:403.2.

### S4: Synthesis of G1-6

EDCI (7.47 g, 38.9 mmol, 1.05 eq) and HOBt (5.51 g, 40.8 mmol, 1.1 eq) were added to a DCM solution (150 mL) of 5-azido-valerianic acid (5.31 g, 37.1 mmol, 1.0 eq), and a temperature was cooled to 0 °C. Then, DIPEA (9.59 g, 74.2 mmol, 12.9 mL, 2.0 eq) and the compound G1-5 (15.0 g, 37.1 mmol, 1.0 eq) were added to the solution in sequence. The temperature was heated to 20 °C, and stirring was continuously performed for 1 h. Sampling was performed for LC/MS detection, showing the disappearance of the raw material. The reaction solution was diluted with DCM (150 mL), and washing was performed with saturated NaHCO₃ (150 mL × 3). The organic phase was dried with Na₂SO₄, and the solvent was removed through vacuum concentration after filtration, so as to obtain a colorless liquid crude product G1-6 (18.0 g). MS (ESI)m/z[M-H]⁻:528.3; ¹H NMR (400MHz, CDCl₃) δ ppm 6.30 (d, J=8.8Hz, 1H), 6.17 (dd, J=7.1, 4.6Hz, 1H), 5.34 (dd, J=3.4, 1.1Hz, 1H), 5.03 (dd, J=11.2, 3.4Hz, 1H), 4.44 (d, J=8.4Hz, 1H), 4.29-4.08 (m, 3H), 4.03-3.98 (m, 1H), 3.88 (td, J=6.7, 1.2Hz, 1H), 3.71-3.57 (m, 1H), 3.45 (td, J=9.2, 3.5Hz, 1H), 3.31 (t, J=6.7Hz, 2H), 3.14-3.02 (m, 1H), 2.27 (t, J=7.2Hz, 2H), 2.16 (s, 3H), 2.04 (s, 3H), 2.00 (s, 3H),1.96 (s, 3H), 1.84-1.58 (m, 6H).

### S5: Synthesis of G1-7

The G1-2 (400 mg, 1.19 mmol, 1.0 eq) was added to a DMSO solution (50.0 mL) of the G1-6 (2.53 g, 4.77 mmol, 4.0 eq). Then, CuSO₄ (1.71 g, 10.7 mmol, 1.65 mL, 9.0 eq) and THPTA (3.11 g, 7.16 mmol, 6.0 eq) were added to water (15.0 mL), then VC Na (3.54 g, 17.89 mmol, 15.0 eq) was added to prepare into an aqueous solution, the aqueous solution was added to the DMSO solution, and stirring was performed for 2 h at 25 °C. Sampling was performed for LC/MS detection, showing the disappearance of the raw material. The reaction solution was diluted with DCM (100 mL), and washing was performed with water (100 mL × 2). The organic phase was dried with Na₂SO₄, the solvent was removed through vacuum concentration after filtration, and the crude product was purified by a silica gel column (PE/EA, DCM/methanol) to obtain a white solid G1-7 (1.90 g, 987.5 µmol, 82.8% yield). MS (ESI) m/z [M-2H]²⁻: 960.8; ¹H NMR (400MHz, CDCl₃) δ ppm 7.62 (s,3H), 6.99 (d, J=9.0Hz, 3H), 6.73 (t, J=6.2Hz, 3H), 5.32 (d, J=3.3Hz, 3H), 5.28 (s, 3H), 5.05 (dd, J=11.2, 3.4Hz, 3H), 4.55-4.49 (m, 10H), 4.37 (t, J=7.0Hz, 6H), 4.27-4.06 (m, 10H), 4.00-3.95 (m, 3H), 3.92-3.85 (m, 3H), 3.67 (s, 6H), 3.54-3.42 (m, 6H), 3.13-3.08 (m, 3H), 2.32-2.23 (m, 7H), 2.13 (s, 10H), 2.01 (s, 10H), 1.96 (s, 10H), 1.88 (s, 9H), 1.70-1.61 (m, 9H), 1.38 (s, 10H).

### S6: Synthesis of G1-8

TFA (9.21 g, 80.7 mmol, 6.00 mL, 81.7 eq) was added to the DCM solution (18.0 mL) of the G1-7 (1.90 g, 988 µmol, 1.0 eq), and stirring was performed for 0.5 h at 25 °C. Sampling was performed for TLC (DCM/methanol) detection, showing the disappearance of the raw material. The reaction solution was directly subjected to vacuum concentration to remove the solvent, and a crude product and toluene (50.0 mL) were rotatably evaporated for three times, so as to obtain a yellow oily substance G1-8 (1.80 g, 967 µmol, 97.8% yield, 98.0% purity). LC/MS (ESI) m/z [M-2H]²⁻: 910.7.

### S7: Synthesis of G1-10

HBTU (174 mg, 460 µmol, 1.5 eq) and DIPEA (79.4 mg, 614 µmol, 107 µL, 2.0 eq) were added to a DMF solution (3.0 mL) of the G1-9 (the preparation method being referred to CN116854754B) (200 mg, 307 µmol, 1.0 eq). Then, the crude product G1-8 (955 mg, 491 µmol, 1.6 eq, TFA) (DIPEA being used to regulate pH to be appropriate 7) was added to the reaction solution, and stirring was performed for 6 h at 25 °C. Sampling was performed for LC/MS detection, showing the disappearance of the raw material. The reaction solution was directly used for the next step. MS (ESI) m/z [M-2H]²⁻: 1227.0.

### S8: Synthesis of G1-11

The G1-10 (750 mg, 305 µmol, 1.0 eq) was added to DMF (4.0 mL). Then DIPEA (118 mg, 915 µmol, 159 µL, 3.0 eq), succinic anhydride (244 mg, 2.44 mmol, 8.00 eq), and DMAP (37.3 mg, 305 µmol, 1.0 eq) were added, and the reaction solution was stirred for 40 h at 25 °C. Sampling was performed for LC/MS detection, and the main peak was a target product. The reaction solution was filtered, and filtrate was purified with preparative chromatography, so as to obtain a white solid G1-11 (120.0 mg, 46.0 µmol). MS (ESI) m/z [M-2H]²⁻:1276.8; ¹H NMR (400 MHz, CDCl₃) δ 7.55 (s, 2H), 7.43-7.36 (m, 2H), 7.26 (dd, J=9.1, 2.8 Hz, 3H), 7.21-7.17 (m, 6H), 7.11 (dd, J=8.3, 6.2 Hz, 1H), 6.88 (d, J=9.0 Hz, 2H), 6.76-6.70 (m, 3H), 6.68 (t, J=6.0 Hz, 2H), 5.28 (d, J=3.3 Hz, 2H), 5.09 (t, J=5.2 Hz, 1H), 5.00 (dd, J=11.2, 3.4 Hz, 2H), 4.93 (d, J=2.3 Hz, 1H), 4.57-4.41 (m, 7H), 4.32(t, J=6.9 Hz, 4H), 4.24-4.00 (m, 8H), 3.95-3.90 (m, 2H), 3.86-3.82 (m,3H), 3.71-3.61 (m, 10H), 3.48-3.37 (m, 5H), 3.34-3.29 (m, 3H), 3.18-3.02 (m, 5H), 2.73 (q, J=7.3 Hz, 7H), 2.64-2.57 (m, 2H), 2.47 (t, J=6.9 Hz, 2H), 2.32-1.75 (m, 54H), 1.72-1.58 (m, 11H), 1.50-1.41 (m, 4H), 1.19-1.09 (m, 20H).

### S9: Synthesis of YK-GAL-401

The G1-11 (160 mg, 62.5 µmol, 1.0 eq) was added to DMF (10.0 mL). Then DIPEA (64.7 mg, 500 µmol, 87.20 µL, 8.0 eq), HBTU (118 mg, 312 µmol, 5.0 eq), DMAP (37.3 mg, 305 µmol, 1.0 eq), and CPG-NH₂ (1.1 g) were added, and the reaction solution was stirred for 16 h at 40 °C. Sampling was performed for LC/MS detection, showing the disappearance of the raw material. The reaction solution was filtered, and the filter cake was washed sequentially using MeOH (20 mL × 4) and DCM (20 mL × 4), and dried under a nitrogen condition, so as to obtain a light yellow solid, which was directly used for the next step.

The crude product (1.2 g) was dissolved in pyridine (10.0 mL). Then acetic anhydride (2.17 g, 21.3 mmol, 2.0 mL, 45.3 eq) was added, the reaction solution was stirred for 1 h at 40 °C and was then filtered, and the filter cake was washed sequentially using MeOH (20 mL × 4) and DCM (20 mL × 4) and was dried under the nitrogen condition, so as to obtain a light yellow solid YK-GAL-401 (1.0 g, and carrying capacity: 37.1 µmol/g).

A generally method for determining the carrying capacity of a carrier included: the YK-GAL-401 was weighed in a 1.5 mL centrifuge tube, and then 1.0 mL PTSA (CAS: 104-15-4) (0.1 M ACN solution) was added to the centrifuge tube. A test tube was sealed and well mixed. The solution was orange in color, and allowed to stand for 1 hour. 100 µL of the solution was taken and added to a 4.0 mL centrifuge tube. 3.0 mL PTSA (0.1 M ACN solution) was added to the centrifuge tube. The test tube was sealed and well mixed, and the PTSA (0.1 M ACN) was used to fill a reference cuvette. The absorbance of a blank solution at 411 nm was measured. A sample solution was used to fill the cuvette, the absorbance of the sample solution at 411 nm was measured, and a carrying capacity might be obtained through calculation.

### 2. Synthesis of YK-GAL-402

The synthesis route was as follows.

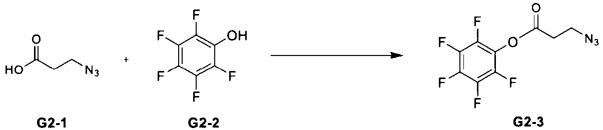

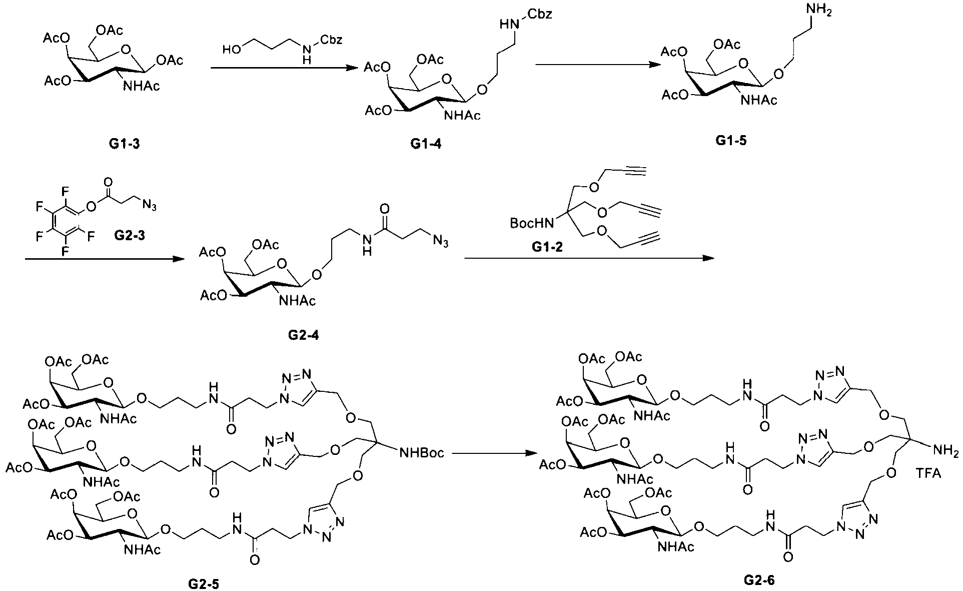

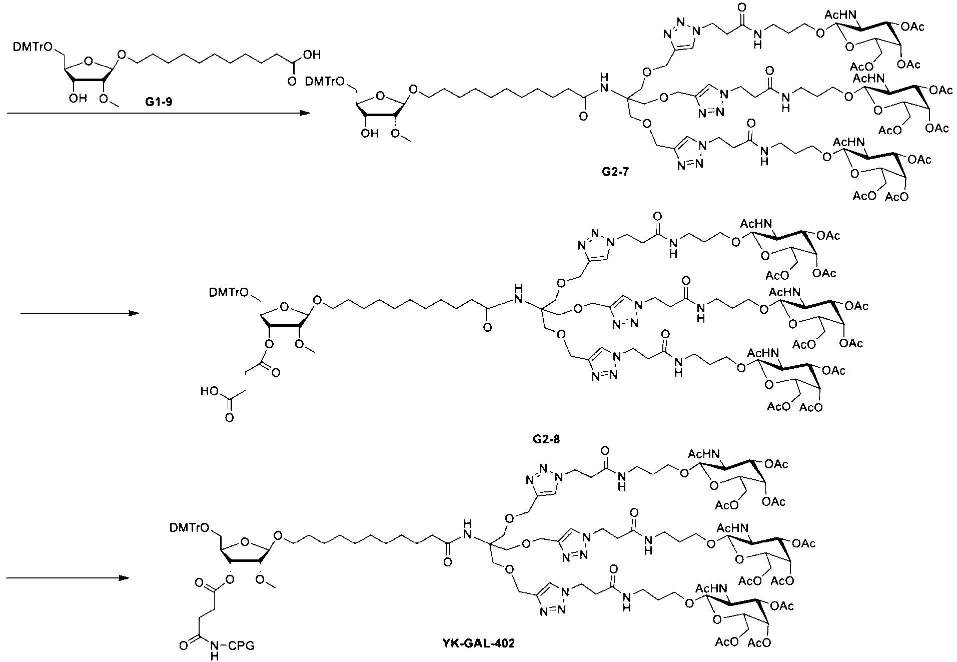

### S1: Synthesis of G2-3

The compounds G2-1 (1.30 g, 11.3 mmol, 1.04 eq) and G2-2 (2.00 g, 10.9 mmol, 1.0 eq) were dissolved in anhydrous EA (20.0 mL), and the mixture was cooled to 0 °C. Then EDCI (2.17 g, 11.3 mmol, 1.04 eq) was added in batches, and stirring was continuously performed for 1 h at 0 °C. The temperature was then slowly heated to 25 °C, and stirring was performed for 4 h. Sampling was performed for LC/MS detection, showing the disappearance of the raw material. The reaction solution was sequentially washed with water (20 mL × 3), with 10% NaHCO₃ aqueous solution (20 mL × 3), with water (20 mL × 2), with 5% KHSO₄ aqueous solution (20 mL × 2), and with water (20 mL × 3). The organic phase was dried with Na₂SO₄, and the solvent was removed through vacuum concentration after filtration, so as to obtain a brown oily substance G2-3 (2.50 g, 8.89 mmol, 81.8 % yield). ¹H NMR (400 MHz, CDCl₃) δ ppm 3.72 (t, J=6.4 Hz, 2 H), 2.94 (t, J=6.4 Hz, 2 H); ¹⁹F NMR (376 MHz, CDCl₃) δ -152.54 - -152.63 (m, 2 F), -157.46 (t, J=21.8Hz, 1 F), -161.98 - -162.13 (m, 2 F).

### S2: Synthesis of G2-4

DIPEA (1.54 g, 11.9 mmol, 2.08 mL, 2.0 eq) was added to a DCM solution (30.0mL) of the compound G1-5 (3.55 g, 5.97 mmol, 1.0 eq) and the compound G2-3 (1.68 g, 5.97 mmol, 1.0 eq). Stirring was performed for 2 h at 25 °C. Sampling was performed for LC/MS detection, showing the disappearance of the raw material. The reaction solution was sequentially washed with NaHCO₃ aqueous solution (30.0 mL × 1), and with water (30 mL × 3). The organic phase was dried with Na₂SO₄, the solvent was removed through vacuum concentration after filtration, and the crude product was purified by preparative chromatography to obtain a white solid G2-4 (710 mg, 1.42 mmol, 23.7 % yield). MS (ESI) m/z [M-H]⁻: 500.2; ¹H NMR (400 MHz, CDCl₃) δ ppm 6.37-6.28 (m, 1 H), 6.14(d, J=8.8 Hz, 1 H), 5.35 (d, J=3.3 Hz, 1 H), 5.02 (dd, J=11.2, 3.4 Hz,1 H), 4.43 (d, J=8.4 Hz, 1 H), 4.30-4.23 (m, 1 H), 4.20-4.10 (m, 2 H),4.06-4.01 (m, 1 H), 3.92-3.84 (m, 1 H), 3.71-3.57 (m, 3 H), 3.46 (td, J=9.1, 3.3 Hz, 1 H), 3.21-3.09 (m, 1 H), 2.62-2.44 (m, 2 H), 2.17 (s, 3H), 2.04 (s, 3 H), 2.01 (s, 3 H), 1.98 (s, 3 H), 1.71-1.66 (m, 2 H).

### S3: Synthesis of G2-5

The G1-2 (110 mg, 328 µmol, 1.0 eq) was added to a DMSO solution (20.0 mL) of the G2-4 (575 mg, 1.15 mmol, 3.5 eq). Then, CuSO₄ (471 mg, 2.95 mmol, 453 µL, 9.0 eq) and THPTA (855 mg, 1.97 mmol, 6.0 eq) were added to water (4.0 mL), then VC Na (974 mg,4.92 mmol, 15.0 eq) was added to prepare into an aqueous solution, the aqueous solution was added to the DMSO solution, and stirring was performed for 2 h at 25 °C. Sampling was performed for LC/MS detection, showing the disappearance of the raw material. The reaction solution was added with water (2.0 mL), extracted with DCM (3 mL × 3), and washed with water (2 mL × 3). The organic phase was dried with Na₂SO₄, the solvent was removed through vacuum concentration after filtration, and the crude product was purified by preparative chromatography to obtain a white solid G2-5 (350 mg, 190 µmol, 58.0% yield). MS (ESI) m/z [M-2H]²⁻: 918.6; ¹H NMR (400 MHz, CDCl₃) δ ppm 7.69 (s, 3 H), 7.09 (t, J=5.7 Hz, 3 H), 6.97 (d, J=9.0 Hz, 3 H), 5.35 (d, J=3.2 Hz, 3 H), 5.08 (dd, J=11.3, 3.4Hz, 3 H), 4.77-4.66 (m, 6 H), 4.52 (s, 6 H), 4.47 (d, J=8.5 Hz, 3 H),4.26-4.11 (m, 9 H), 3.96-3.91 (m, 6 H), 3.60 (s, 6 H), 3.52-3.47 (m, 2H), 3.42-3.36 (m, 3 H), 3.16-3.11 (m, 3 H), 3.04-2.97 (m, 3 H), 2.86-2.80 (m, 3 H), 2.15 (s, 9 H), 2.04 (s, 9 H), 2.00 (s, 9 H), 1.85 (s, 9 H),1.78 (s, 8 H), 1.42 (s, 9 H).

### S4: Synthesis of G2-6

TFA (1.07 g, 9.42 mmol, 700 µL, 49.5 eq) was added to the DCM solution (2.8 mL) of the G2-5 (350 mg, 190 µmol, 1.0 eq), and stirring was performed for 0.5 h at 25 °C. Sampling was performed for LC/MS detection, showing the disappearance of the raw material. The reaction solution was directly subjected to vacuum concentration to remove a solvent, and the crude product and toluene (10.0 mL) were rotatably evaporated for three times, so as to obtain a red oily substance G2-6 (523 mg). MS (ESI) m/z [M-2H]²⁻: 868.6.

### S5: Synthesis of G2-7

HBTU (65.6 mg, 173 µmol, 1.5 eq) and DIPEA (29.8 mg, 230 µmol, 40.2 µL, 2.0 eq) were added to a DMF solution (1.5 mL) of the G1-9 (75.0 mg, 115 µmol, 1.0 eq). Then, the crude product G2-6 (200 mg, 115 µmol, 1.0 eq, TFA) (DIPEA being used to regulate pH to be appropriate 7) was added to the reaction solution, and stirring was performed for 2 h at 25 °C. Sampling was performed for LC/MS detection, showing the disappearance of the raw material. The reaction solution was directly used for a next step. MS (ESI) m/z [M-2H]²⁻: 1184.8.

### S6: Synthesis of G2-8

The G2-7 (273 mg, 115 µmol, 1.0 eq) was added to DMF (3.0 mL). Then DIPEA (44.7 mg, 345 µmol, 60.2 µL, 3.0 eq), succinic anhydride (104 mg, 1.04 mmol, 9.0 eq), and DMAP (56.3 mg, 461 µmol, 4.0 eq) were added, and the reaction solution was stirred for 24 h at 30 °C. Sampling was performed for LC/MS detection, showing the disappearance of the raw material. The reaction solution was filtered, and filtrate was purified with preparative chromatography, so as to obtain a white solid G2-8 (110 mg, 44.5 µmol, 38.6% yield). MS (ESI) m/z [M-2H]²⁻: 1234.9; ¹H NMR (400 MHz, CDCl₃) δ ppm 7.66 (s, 3 H), 7.45 (d, J=7.7 Hz, 2 H), 7.33 (d, J=8.8 Hz, 4 H), 7.28 (s, 1 H), 7.24 (s, 1 H), 7.20-7.13 (m, 4 H), 7.02 (d, J=9.0 Hz, 2 H), 6.81 (d, J=8.7 Hz, 3 H), 5.35 (d, J=3.3 Hz, 3 H), 5.08 (dd, J=11.2, 3.4 Hz,3 H), 4.73-4.69 (m, 5 H), 4.53-4.47 (m, 8 H), 4.26-4.10 (m, 11 H), 3.97-3.89 (m, 7 H), 3.78 (s, 6 H), 3.65 (s, 5 H), 3.56-3.44 (m, 4 H), 3.40-3.37 (m, 5 H), 3.24-3.21 (m, 2 H), 3.16-3.09 (m, 4 H), 3.01-2.94 (m, 4H), 2.82-2.78 (m, 2 H), 2.69-2.64 (m, 2 H), 2.56-2.53 (m, 2 H), 2.15 (s,9 H), 2.04 (s, 9 H), 2.00 (s, 9 H), 1.93-1.76 (m, 32 H), 1.27 (br, 10 H).

### S7: Synthesis of YK-GAL-402

The G2-8 (110 mg, 42.74 µmol, 1.0 eq, TEA) was added to DMF (8.0 mL), then DIPEA (44.2 mg, 342 µmol, 59.5 µL, 8.0 eq), HBTU (81.0 mg, 213 µmol, 5.0 eq), DMAP (5.22 mg, 42.7 µmol, 1.0 eq), and CPG-NH₂ (713 mg, 1.21 mmol, 28.4 eq) were added, and a reaction solution was stirred for 16 h at 40 °C. Sampling was performed for LC/MS detection, showing the disappearance of the raw material. The reaction solution was filtered, and the filter cake was washed sequentially using MeOH (10mL × 4) and DCM (10 mL × 4), and dried under a nitrogen condition, so as to obtain a light yellow solid, which was directly used for the next step.

The crude product was dissolved in a pyridine and acetic anhydride solution (Py/Ac₂O=5/1, 9.0 mL), the reaction solution was stirred for 40 min at 40 °C and was then filtered, and the filter cake was washed sequentially using DCM (5.0 mL × 4) and MeOH (5.0 mL × 4) and was dried under the nitrogen condition, so as to obtain a light yellow solid YK-GAL-402 (726 mg, and carrying capacity: 33.15 µmol/g).The carrying capacity of a carrier was based on the above generally method for determining the carrying capacity of the carrier.

### 3. Synthesis of YK-GAL-403

The synthesis route was as follows.

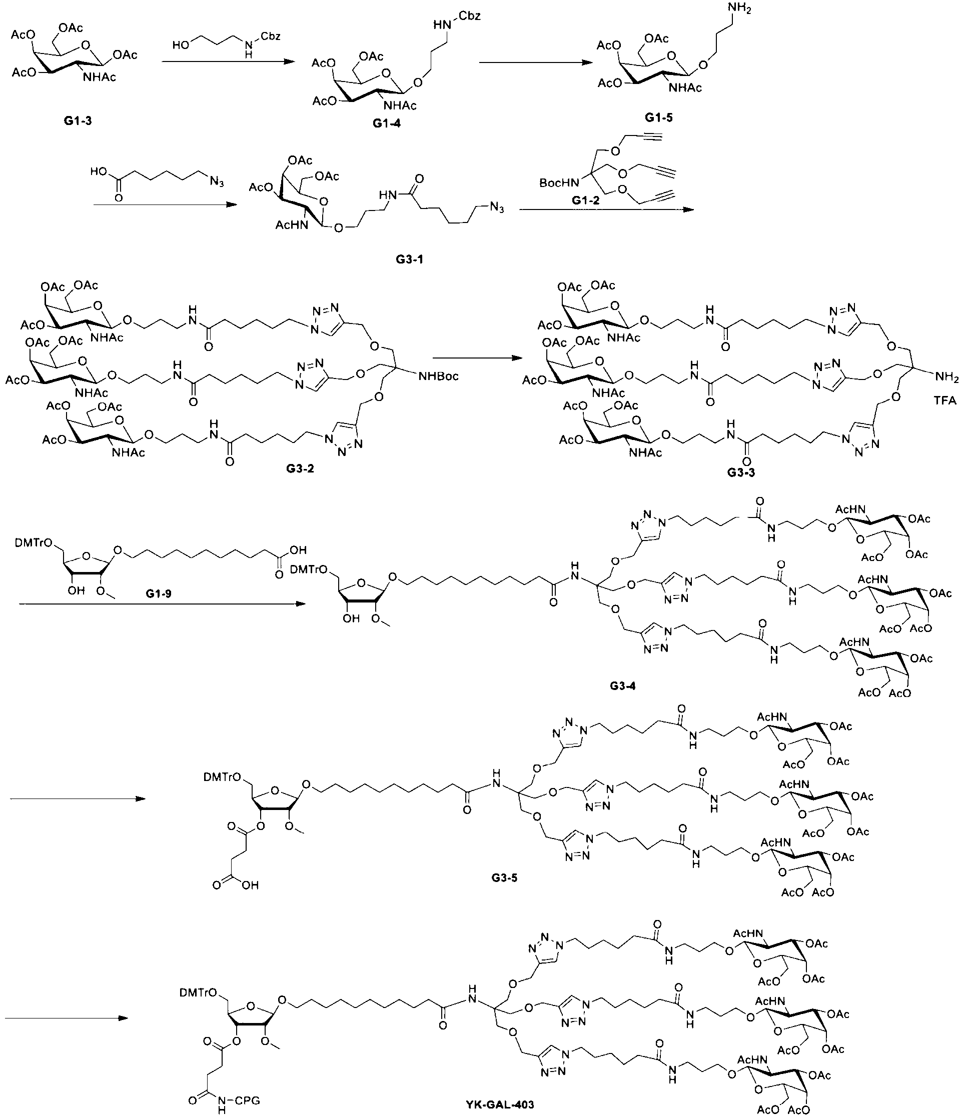

### S1: Synthesis of G3-1

EDCI (1.92 g, 10.0 mmol, 1.05 eq) and HOBt (1.42 g, 10.50 mmol, 1.1 eq) were added to a DCM solution (40.0 mL) of 6-azido-hexanoic acid (1.50 g, 9.54 mmol, 1.0 eq), and the temperature was cooled to 0 °C.

Then, DIPEA (2.47 g, 19.1 mmol, 3.32 mL, 2.0 eq) and the compound G1-5 (3.86 g, 9.54 mmol, 1.0 eq) were added to the solution in sequence. The temperature was heated to 25 °C, and stirring was continuously performed for 2 h. Sampling was performed for LC/MS detection, showing the disappearance of the raw material. The reaction was diluted with DCM (20.0 mL), first washed with saturated NaHCO₃ (40.0 mL), and then washed with water (40.0 mL). The organic phase was dried with Na₂SO₄, and the solvent was removed through vacuum concentration after filtration, so as to obtain a yellow oily substance G3-1 (1.42 g, 2.61 mmol, 32.7 % yield). MS (ESI) m/z [M-H]⁻: 542.2; ¹H NMR (400 MHz, CDCl₃) δ ppm 6.32 (d, J=8.8 Hz, 1 H), 6.09-6.01 (m, 1 H), 5.34 (d, J=2.7 Hz, 1 H), 5.04 (dd, J=11.2, 3.3 Hz, 1 H), 4.45 (d, J=8.4 Hz, 1 H), 4.28-4.09 (m, 3 H), 4.03-3.98 (m, 1 H), 3.88 (td, J=6.7, 1.2 Hz, 1 H),3.71-3.62 (m, 1 H), 3.44 (td, J=9.3, 3.4 Hz, 1 H), 3.28 (t, J=6.8 Hz, 2H), 3.10-3.03 (m, 1 H), 2.24 (t, J=7.5 Hz, 2 H), 2.16 (s, 3 H), 2.04 (s, 3 H), 2.01 (s, 3 H), 1.96 (s, 3 H), 1.76-1.63 (m, 6 H), 1.46-1.38 (m, 2H).

### S2: Synthesis of G3-2

The G1-2 (230 mg, 686 µmol, 1.0 eq) was added to a DMSO solution (50.0 mL) of the G3-1 (1.30 g, 2.40 mmol, 3.5 eq). Then, CuSO₄ (985 mg, 6.17 mmol, 947 µL, 9.0 eq) and THPTA (1.79 g, 4.11 mmol, 6.0 eq) were added to water (6.0 mL), then VC Na (2.04 g,10.3 mmol, 15.0 eq) was added to prepare into an aqueous solution, the aqueous solution was added to the DMSO solution, and stirring was performed for 2 h at 25 °C. Sampling was performed for LC/MS detection, showing the disappearance of the raw material. The reaction solution was diluted with water (30.0 mL), extracted with DCM (30.0 mL × 3), and then washed with water (30.0 mL × 3). The organic phase was dried with Na₂SO₄, the solvent was removed through vacuum concentration after filtration, and the crude product was purified by preparative chromatography to obtain a white solid G3-2 (1.0 g, 508 µmol, 74.2% yield). MS (ESI) m/z [M-2H]²⁻: 981.8; ¹H NMR(400 MHz, CDCl₃) δ ppm 7.60 (s, 3 H), 6.84 (d, J=8.9 Hz, 3 H), 6.62-6.54 (m, 3 H), 5.34 (d, J=3.3 Hz, 3 H), 5.06 (dd, J=11.2, 3.4 Hz, 3 H), 4.57 (s, 6 H), 4.50 (d, J=8.5 Hz, 3 H), 4.36 (t, J=6.9 Hz, 6 H), 4.28-4.07 (m, 10 H), 4.00-3.95 (dt, J=9.9, 5.0 Hz, 3 H), 3.90 (t, J=6.7 Hz, 3 H), 3.69 (s, 6 H), 3.59-3.55 (m, 3 H), 3.51-3.41 (m, 3 H), 3.11-3.06 (m, 3 H), 2.21 (t, J=7.4 Hz, 6 H), 2.15 (s, 9 H), 2.03 (s, 9 H), 1.99 (s, 9 H), 1.93 (s, 9 H), 1.82 (s, 9 H), 1.72-1.61 (m, 9 H), 1.40 (s, 9H), 1.37-1.29 (m, 6 H).

### S3: Synthesis of G3-3

TFA (3.07 g, 26.9 mmol, 2.0 mL, 52.9 eq) was added to the DCM solution (10.0 mL) of the G3-2 (1.00 g, 508 µmol, 1.00 eq), and stirring was performed for 1 h at 25 °C. Sampling was performed for TLC (DCM/methanol) detection, showing the disappearance of the raw material. The reaction solution was directly subjected to vacuum concentration to remove a solvent, and the crude product and toluene (20.0 × 3 mL) were rotatably evaporated for three times, so as to obtain a yellow oily substance G3-3 (950 mg, 458.21 µmol, 90.09% yield, 90% purity). MS (ESI) m/z [M-2H]²⁻: 931.7.

### S4: Synthesis of G3-4

HBTU (174 mg, 460 µmol, 1.5 eq) and DIPEA (79.4 mg, 614 µmol, 107 µL, 2.0 eq) were added to a DMF solution (3.0 mL) of the G1-9 (200 mg, 307 µmol, 1.0 eq). Then, the crude product G3-3 (837 mg, 430 µmol, 1.4 eq, TFA) (DIPEA being used to regulate pH to be appropriate 7) was added to the reaction solution, and stirring was performed for 4 h at 25 °C. Sampling was performed for LC/MS detection, showing the disappearance of the raw material. The reaction solution was directly used for the next step. MS (ESI) m/z [M-2H]²⁻: 1247.8.

### S5: Synthesis of G3-5

The G3-4 (760 mg, 304 µmol, 1.0 eq) was added to DMF (4.0 mL). Then DIPEA (112 mg, 912 µmol, 158 µL, 3.0 eq), succinic anhydride (243 mg, 2.43 mmol, 8.0 eq), and DMAP (37.1 mg, 304 µmol, 1.0 eq) were added, and the reaction solution was stirred for 24 h at 35 °C. Sampling was performed for LC/MS detection, showing the disappearance of the raw material, and the target product was detected. The reaction solution was filtered, and filtrate was purified with preparative chromatography, so as to obtain a white solid G3-5 (400 mg, 142 µmol, 46.7% yield, 96.0% purity, TEA). MS (ESI) m/z [M-2H]²⁻: 1298.2; ¹H NMR (400 MHz, CDCl₃) δ ppm 7.53 (s, 3 H), 7.40-7.36 (m, 2 H), 7.31-7.24(m, 4 H), 7.22-7.07 (m, 6 H), 6.84 (d, J=8.9 Hz, 2 H), 6.77-6.70 (m, 4H), 6.59 (t, J=6.1 Hz, 3 H), 6.08 (s, 1 H), 5.28 (d, J=3.3 Hz, 3 H), 5.09(t, J=5.1 Hz, 1 H), 5.04-4.90 (m, 4 H), 4.50 (s, 6 H), 4.45 (d, J=8.4 Hz, 3 H), 4.30 (t, J=6.9 Hz, 6 H), 4.21-4.02 (m, 10 H), 3.93-3.88 (m, 3H), 3.87-3.81 (m, 4 H), 3.70 (d, J=9.3 Hz, 12 H), 3.50 (d, J=7.8 Hz, 3H), 3.39 (td, J=9.2, 3.7 Hz, 3 H), 3.32 (s, 3 H), 3.16 (dd, J=9.8, 4.3Hz, 2 H), 3.08-2.99 (m, 4 H), 2.76 (q, J=7.3 Hz, 9 H), 2.63-2.57 (m, 2H), 2.47-2.44 (m, 2 H), 2.15 (t, J=7.3 Hz, 6 H), 2.09 (s, 9 H), 1.97 (s,9 H), 1.92 (s, 9 H), 1.87 (s, 9 H), 1.71-1.67 (m, 3 H), 1.66-1.56 (m, 9H), 1.51-1.40 (m, 4 H), 1.29-1.24 (m, 6 H), 1.10 (t, J=7.2 Hz, 11 H).

### S6: Synthesis of YK-GAL-403

The G3-5 (400 mg, 154 µmol, 1.0 eq) was added to DMF (10.0 mL). Then DIPEA (159 mg, 1.23 mmol, 214 µL, 8.0 eq), HBTU (291 mg, 769 µmol, 5.0 eq), DMAP (18.8 mg, 154 µmol, 1.00 eq), and CPG-NH₂ (2.25 g) were added, and a reaction solution was stirred for 16 h at 40 °C. Sampling was performed for LC/MS detection, showing the disappearance of the raw material. The reaction solution was filtered, and the filter cake was washed sequentially using MeOH (20 mL × 4) and DCM (20 mL × 4), and dried under a nitrogen condition, so as to obtain a light yellow solid, which was directly used for a next step.

The crude product (2.4 g) was dissolved in pyridine (15.0 mL). Then acetic anhydride (3.26 g, 31.9 mmol, 3.0 mL, 34.5 eq) was added, the reaction solution was stirred for 1 h at 40 °C and was then filtered, and the filter cake was washed sequentially using MeOH (30 mL × 4) and DCM (30 mL × 4) and was dried under the nitrogen condition, so as to obtain a light yellow solid YK-GAL-403 (2.2 g, and carrying capacity: 32.8 µmol/g).The carrying capacity of a carrier was based on the above generally method for determining the carrying capacity of the carrier.

### 4. Synthesis of YK-GAL-404

The synthesis route was as follows.

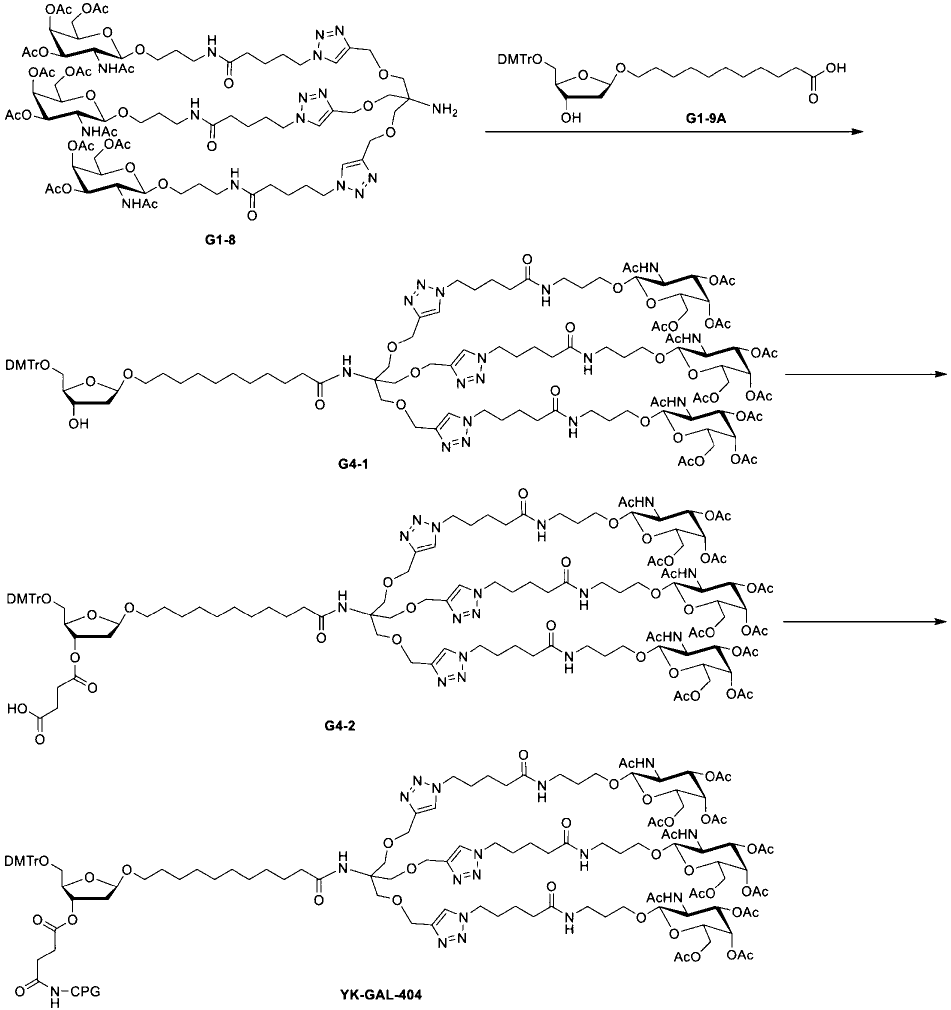

### S1: Synthesis of G4-1

The crude products G1-8 (537 mg, 280 µmol, 1.4 eq, TFA) and G1-9A (the preparation method being referred to CN116854754B) (124 mg, 200 µmol, 1.0 eq) were used as raw materials, a reaction was performed according to the method for synthesizing the G1-10, and sampling was performed for LC/MS detection, showing the disappearance of the raw material. The reaction solution was directly used for a next step. MS (ESI) m/z [M-2 H]²⁻: 1212.0.

### S2: Synthesis of G4-2

The G4-1 (485 mg, 200 µmol, 1.0 eq) was used as a raw material, and a reaction was performed according to the method for synthesizing the G1-11, so as to obtain a white solid G4-2 (187 mg, 74.2 µmol, 37.1%). MS (ESI) m/z [M-2H]²⁻: 1262.1; ¹H NMR(400 MHz, CDCl₃) δ 7.63 (s, 2H), 7.53-7.45 (m, 2H), 7.36-7.33 (m, 3H),7.29-7.11 (m, 7H), 6.92 (d, J=9.1 Hz, 2H), 6.79-6.68 (m, 5H), 5.12-5.05 (m, 3H), 5.06 (d, J=2.3 Hz, 1H), 5.01-4.97 (m, 2H), 4.79-4.61 (m,8H), 4.53 (t, J=6.0 Hz, 4H), 4.34-4.20 (m, 3H), 4.14-4.09 (m, 4H), 3.98-3.86 (m, 5H), 3.80 (s, 6H), 3.77-3.65 (m, 2H), 3.47-3.32 (m, 10H), 3.25-3.12 (m, 3H), 2.85-2.61 (s, 9H), 2.53-2.44 (m, 2H), 2.39-1.89 (m, 52H), 1.82-1.49 (m, 19H), 1.19-1.09 (m, 18H).

### S3: Synthesis of YK-GAL-404

The G4-2 (126 mg, 50.0 µmol, 1.0 eq) was used as a raw material, and a reaction was performed according to the method for synthesizing the YK-GAL-401, so as to obtain a light yellow solid YK-GAL-404 (783 mg, and carrying capacity: 35.9 µmol/g). A carrying capacity of a carrier was based on the above generally method for determining the carrying capacity of the carrier.

### 5. Synthesis of YK-GAL-405

The synthesis route was as follows.

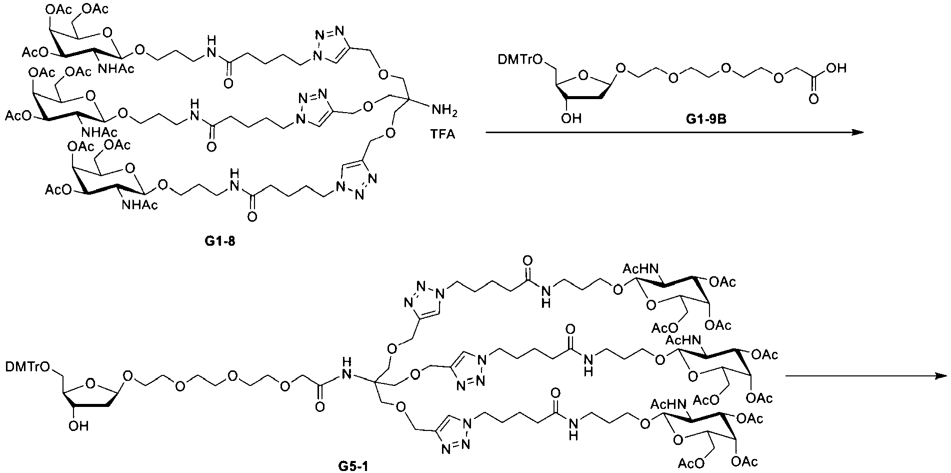

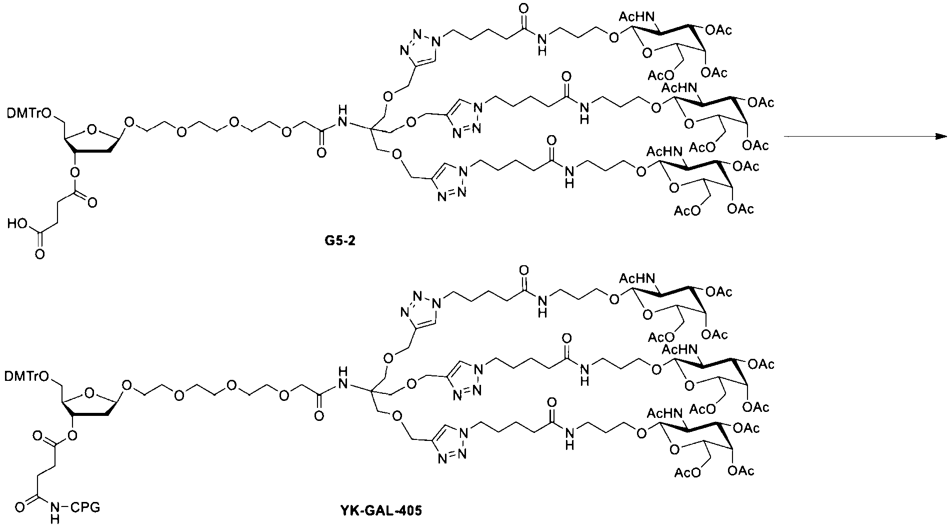

### S1: Synthesis of G5-1

The crude products G1-8 (537 mg, 280 µmol, 1.4 eq, TFA) and G1-9B (the preparation method being referred to CN116854754B) (125 mg, 200 µmol, 1.0 eq) were used as raw materials, a reaction was performed according to the method for synthesizing the G1-10, and sampling was performed for LC/MS detection, showing the disappearance of the raw material. The reaction solution was directly used for the next step. MS (ESI) m/z [M-2 H]²⁻: 1215.2.

### S2: Synthesis of G5-2

The G5-1 (481 mg, 198 µmol, 1.0 eq) was used as a raw material, and a reaction was performed according to the method for synthesizing the G1-11, so as to obtain a white solid G5-2 (173 mg, 68.4 µmol). MS (ESI) m/z [M-2H]²⁻: 1265.2; ¹H NMR (400MHz, CDCl₃) δ ppm 7.53 (s, 1H), 7.50 (s, 1H), 7.44 (d, J=8.0 Hz, 2H), 7.25-7.16 (m, 4H), 7.05-6.91 (m, 4H), 6.85-6.72 (m, 6H), 6.85-6.72 (m,2H), 5.48-5.45 (m, 4H), 5.39 (d, J=4.0 Hz, 2H), 5.12-4.99 (m, 3H), 4.57-4.32 (m, 11H), 4.19-4.06 (m, 6H), 3.96-3.87 (m, 4H), 3.85-3.79 (m, 14H), 3.68 (s, 6H), 3.63-3.56 (m, 12H), 3.37-3.25 (m, 11H), 3.15 (t, J=5.9 Hz, 5H), 3.11-2.89 (m, 4H), 2.48-2.34 (m, 12H), 2.21-1.98 (m, 25H),1.82-1.61 (m, 14H), 1.28-1.08 (m, 13H).

### S3: Synthesis of YK-GAL-405

The G5-2 (171 mg, 67.5 µmol, 1.0 eq) was used as a raw material, and a reaction was performed according to the method for synthesizing the YK-GAL-401, so as to obtain a light yellow solid YK-GAL-405 (1.1 g, and carrying capacity: 35.9 µmol/g). The carrying capacity of a carrier was based on the above generally method for determining the carrying capacity of the carrier.

### 6. Synthesis of YK-GAL-406

The synthesis route was as follows.

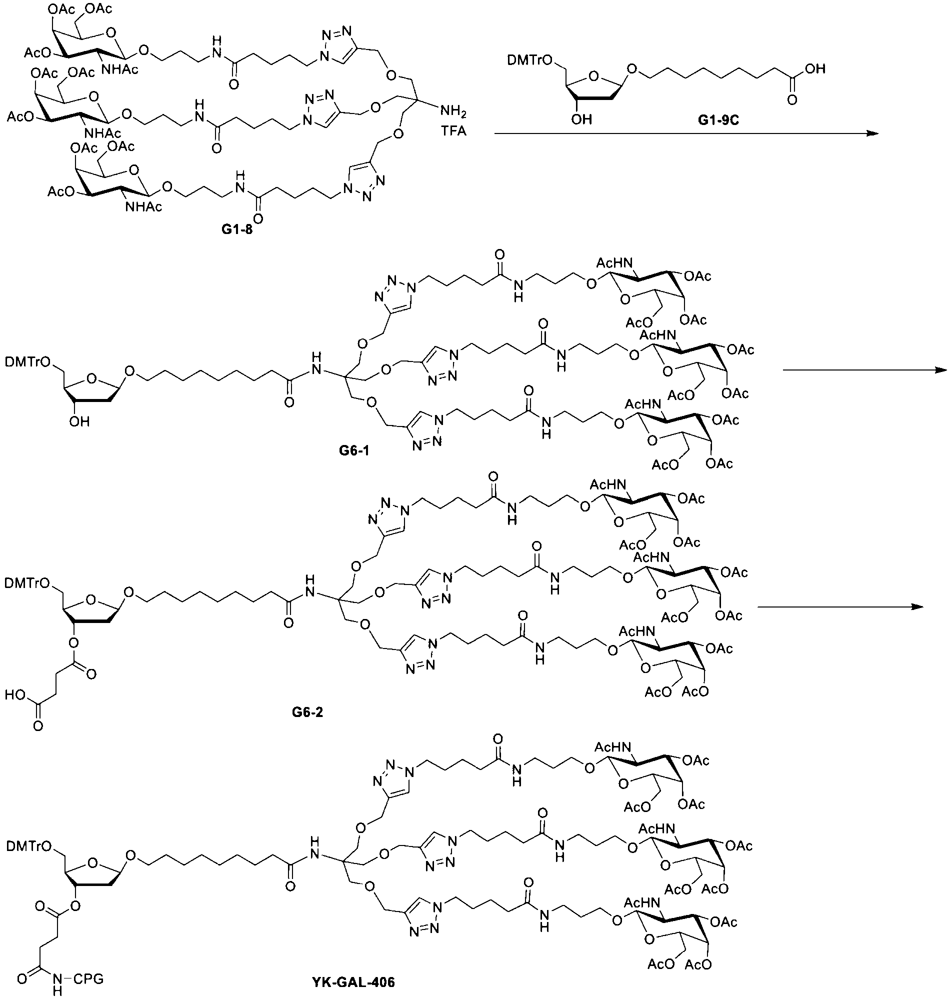

### S1: Synthesis of G6-1

The crude products G1-8 (402 mg, 210 µmol, 1.4 eq, TFA) and G1-9C (the preparation method being referred to CN116854754B) (88 mg, 150 µmol, 1.0 eq) were used as raw materials, a reaction was performed according to the method for synthesizing the Gl-10, and sampling was performed for LC/MS detection, showing the disappearance of the raw material. The reaction solution was directly used for the next step. MS (ESI) m/z [M-2H]²⁻: 1198.0.

### S2: Synthesis of G6-2

The G6-1 (479 mg, 150 µmol, 1.0 eq) was used as a raw material, and a reaction was performed according to the method for synthesizing the G1-11, so as to obtain a white solid G6-2 (146 mg, 58.8 µmol). MS (ESI) m/z [M-2H]²⁻: 1247.8; ¹H NMR (400MHz, CDCl₃) δ ppm 7.47 (d, J=6.4 Hz, 2H), 7.36-7.31 (m, 3H), 7.27-7.23(m, 4H), 7.20 (d, J=6.0 Hz, 1H), 7.15-7.08 (m, 2H), 7.04-6.97 (m, 4H), 6.90-6.83 (m, 3H), 5.54-5.51 (m, 2H), 5.42 (d, J=3.9 Hz, 2H), 5.36 (t, J=4.0 Hz, 2H), 4.89-4.81 (m, 1H), 4.55-4.47 (m, 5H), 4.38-4.24 (m,7H), 4.16-4.04 (m, 4H), 3.80 (s, 6H), 3.74-3.65 (m, 11H), 3.59-3.52 (m, 4H), 3.46-3.28 (m, 15H), 2.83-2.78 (m, 7H), 2.64 (t, J=6.5 Hz, 4H), 2.59-2.50 (m, 4H), 2.47-2.41 (m, 4H), 2.33-1.97 (m, 33H), 1.86-1.62 (m, 20H), 1.37-1.29 (m, 5H), 1.16-1.13 (m, 13H).

### S3: Synthesis of YK-GAL-406

The G6-2 (146 mg, 58.8 µmol, 1.0 eq) was used as a raw material, and a reaction was performed according to the method for synthesizing the YK-GAL-401, so as to obtain a light yellow solid YK-GAL-406 (832 mg, and carrying capacity: 34.1 µmol/g). The carrying capacity of a carrier was based on the above generally method for determining the carrying capacity of the carrier.

### 7. Synthesis of control YK-GAL-301

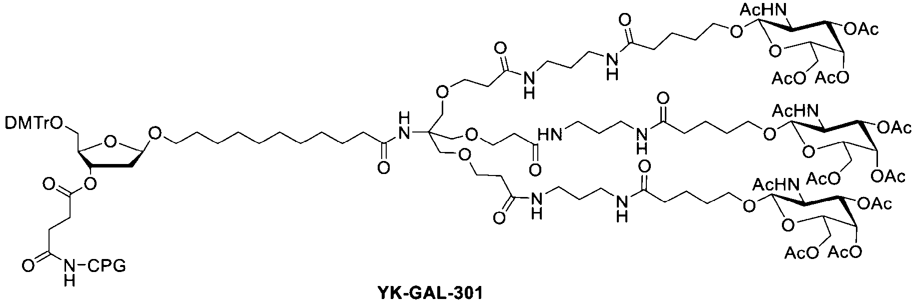

A target product was obtained according to the synthesis method of YK-GAL-301 in Page 35 of the specification of CN116854754B.

### 8. Synthesis of control GalNAc 1b

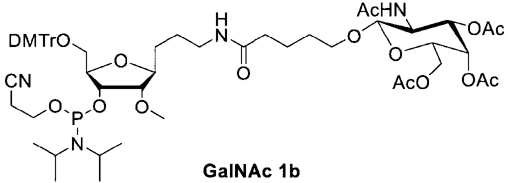

A product (377.2 mg, total yield: 0.58%, MS (ESI) m/z [M+H]⁺=1137.9) was obtained according to the synthesis method of GalNAc 1b in Page 145 of the specification of WO 2023014938A1.

### 9. Synthesis of control NAG0052

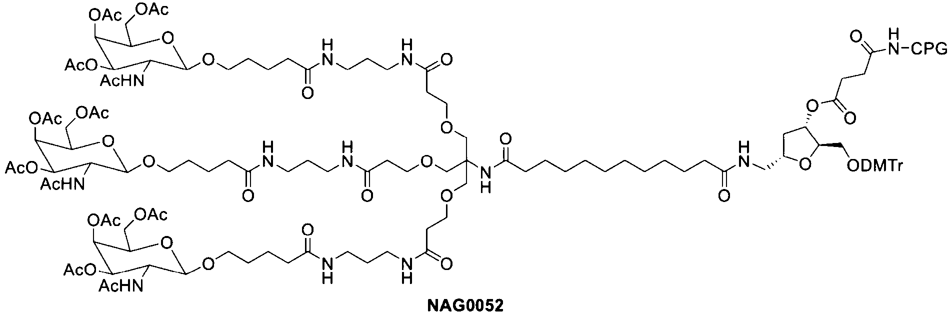

A product (288 mg, total yield: 1.26%, and carrying capacity: 31.5 µmol/g) was obtained according to the synthesis method of NAG0052 in Page 100 of the specification of WO 2023109938A1.

### Example 2: Coupling of GalNAc compound and oligonucleotide

In this example, the same siRNA sequence was used for synthesis, a conjugated siRNA sequence was a sequence numbered inc, and the inc sequence was as follows.
Sense strand (inc-SS):
   5'-Cms-Ums-Am-Gm-Am-Cm-Cf-Um-Gf-Um-dT-Um-Um-Gm-Cm-Um-Um-Um-Um-Gm-Um-3' (SEQ ID NO: 46).
Antisense strand (inc-AS):
   5'-Ams-Cfs-Am-Af-Af-Af-Gm-Cf-Am-Af-Am-Af-Cm-Af-Gm-Gf-Um-Cf-Um-Am-Gms-Ams-Am-3' (SEQ ID NO: 47).

Among them, A, U, C, and G represent base compositions of nucleotides; dT represents deoxythymidine nucleotide; m represents that the nucleotide adjacent to the left side of m was 2'-OMe modification; f represents that the nucleotide adjacent to the left side of f was 2'-F modification; and s represents that two nucleotides on the left and right of s were connected through a phosphorothioate group.

The sequence was from a public sequence, and was an oligonucleotide sequence of a foreign marketed siRNA medicine inclisiran.

The inclisiran sequence was the synthetic, chemically-modified and double-stranded siRNA, which inhibited the generation of a PCSK9 protein by binding to mRNA encoding the PCSK9 protein in a targeting manner through an RNA interference mechanism, thereby regulating and controlling the recycling of an LDL receptor, and enhancing the binding to LDL to achieve the purpose of reducing an LDL level in the blood.

### 1. Preparation of GalNAc-conjugated siRNA sense strand

The oligonucleotide GalNAc conjugates were synthesized on the solid carrier according to the phosphoramidite chemical method. When inc-YK-Gal-401, inc-YK-Gal-402, inc-YK-Gal-403, inc-YK-Gal-404, inc-YK-Gal-405, inc-YK-Gal-406, inc-YK-Gal-301, and inc-NAG0052 conjugates were synthesized, the GalNAc-CPG compound synthesized in Example 1 was used as the solid carrier. When the inc-GalNAc 1b conjugate was synthesized, the control inc-GalNAc 1b monomer was coupled as a first monomer. The GalNAc was conjugated to the 3' end of the siRNA sense strand, and specific structures of the GalNAc compounds used were shown in Table 1.

**Table 1 GalNAc compound conjugated with siRNA sense strand**

| Name | Chemical structure | Source |
|---|---|---|
| YK-GAL-401 | 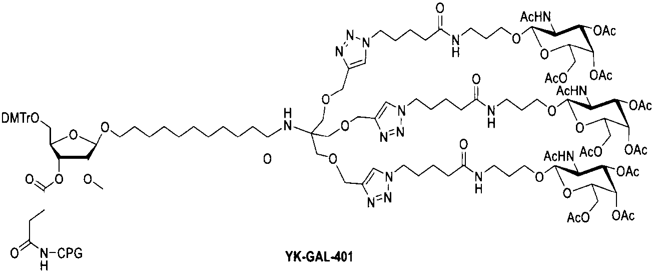 | Designed in the present disclosure, and synthesized in Example 1 |
| YK-GAL-402 | 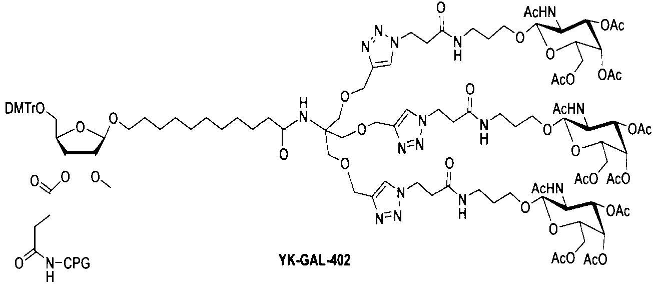 | Designed in the present disclosure, and synthesized in Example 1 |
| YK-GAL-403 | 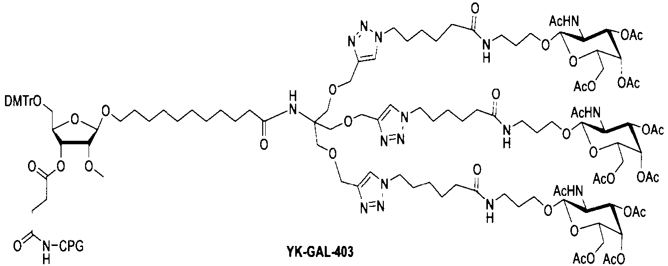 | Designed in the present disclosure, and synthesized in Example 1 |
| YK-GAL-404 | 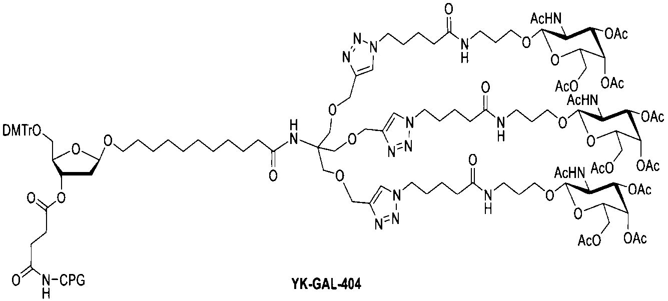 | Designed in the present disclosure, and synthesized in Example 1 |
| YK-GAL-405 | 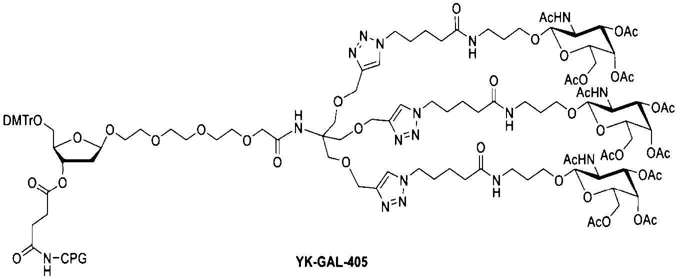 | Designed in the present disclosure, and synthesized in Example 1 |
| YK-GAL-406 | 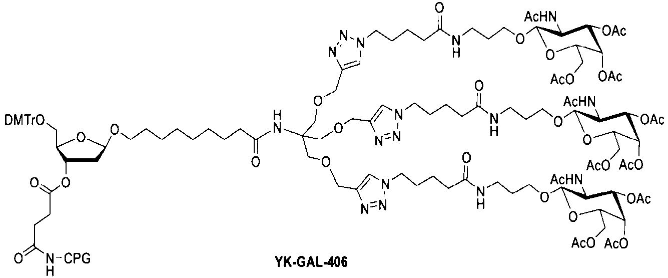 | Designed in the present disclosure, and synthesized in Example 1 |
| Reference Material YK-GAL-301 | 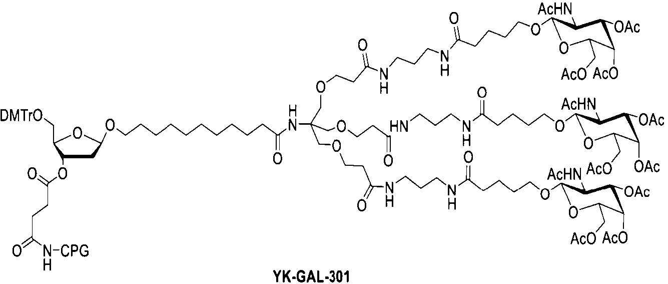 | YK-GAL-301 of CN116854754B, and synthesized in Example 1 |
| Reference Material GalNAc 1b | 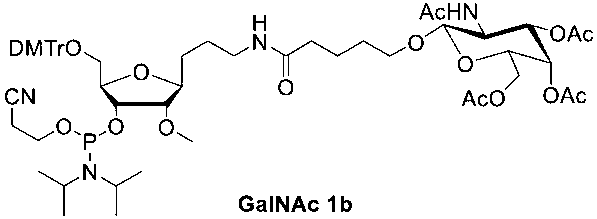 | GalNAclb of WO 2023014938A1, and synthesized in Example 1 |
| Reference Material NAG0052 | 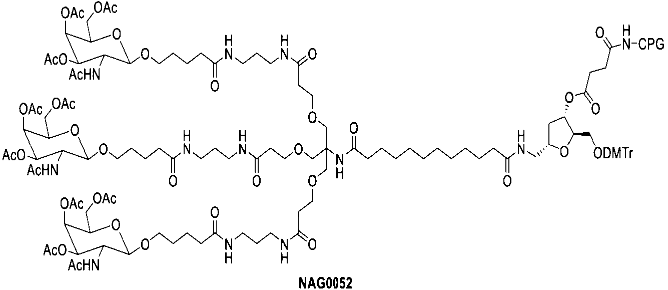 | NAG0052 of WO 2023109938A1, and synthesized in Example 1 |

### Experimental procedure:

### (1) Preparation of reagent and monomer

An acetonitrile solution (1/20, w/v) of the monomer and an acetonitrile solution of 0.25 M 5-benzylthio-1H-tetrazole were used as active agents, an acetonitrile/pyridine (1/4, v/v) solution of 0.2 M xanthane hydride was used as a thio reagent, a water/pyridine (1/9, v/v) solution of 0.05 M iodine was used as an oxidation reagent, 20% acetic anhydride in acetonitrile (v/v) was used as the capping agent A, 20/30/50 (1-methylimidazole/pyridine/acetonitrile, v/v/v) was used as the capping agent B, 20% diethylamine in the acetonitrile (v/v) was used as a decyanation ethyl reagent, 3% dichloroacetic acid in toluene (v/v) was used as a DMT removal reagent, and a corresponding CPG carrier was selected to put in the reagent position specified in the 192 P DNA/RNA automatic synthesizer.

### (2) Crude synthesis

The specified oligonucleotide sequence was inputted, the synthesis process was set, and after no error was checked, the cycle of oligonucleotide synthesis starts. The time for monomer coupling was appropriate 1 minute, an oxygenation time was appropriate 30-45 seconds, and a thio time was appropriate 2 minutes. After the cycle ended, the solid phase synthesis of the oligonucleotide was completed.

### (3) Deprotection

After synthesis was completed, the solid carrier was transferred to a reactor, the cracking of the oligonucleotide was performed for 16-24 hours on the solid phase with concentrated ammonia water (25-28%) at 50-60 °C, the system was cooled to room temperature, then filtration was performed, leaching was performed with a mixed solution of purified water and ethanol, and filtrate was combined and subjected to low-temperature concentration, so as to obtain a crude residue.

### (4) Purification

The crude residue after deprotection was dissolved with the purified water for HPLC purification, the product peak solution was collected, the content was measured with a microplate reader, and the molecular weight was verified through ESI MS.

### 2. Preparation of GalNAc-conjugated siRNA antisense strand

The siRNA antisense strand was synthesized according to the method for synthesizing the siRNA sense strand. A generally CPG solid carrier was used, and a synthesis scale of each antisense strand complementary to the sense strand was 1 µmol.

### 3. Preparation of conjugated double-stranded siRNA

The siRNA sense strand and the complementary antisense strand were mixed according to an ultraviolet absorption content 1:1, heated to 95 °C, and cooled to room temperature after 3 minutes, so as to form double strands. After the product purity of the obtained double-strand solution was characterized to be qualified by HPLC, the content was determined with the microplate reader, and solid powder was obtained through freeze-drying and saved for later use.

The GalNAc compounds in Table 1 were conjugated with the siRNA numbered inc, and the obtained conjugates were respectively named as inc-YK-Gal-401, inc-YK-Gal-402, inc-YK-Gal-403, inc-YK-Gal-404, inc-YK-Gal-405, inc-YK-Gal-406, inc-YK-Gal-301, inc-GalNAc 1b, and inc-NAG0052.

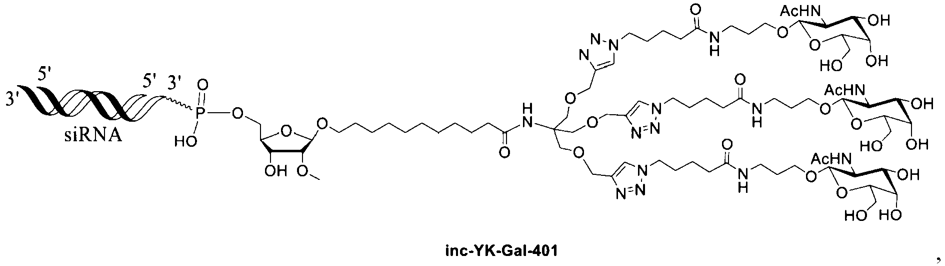

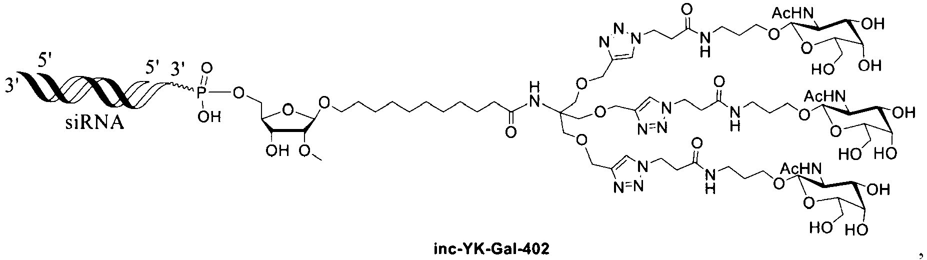

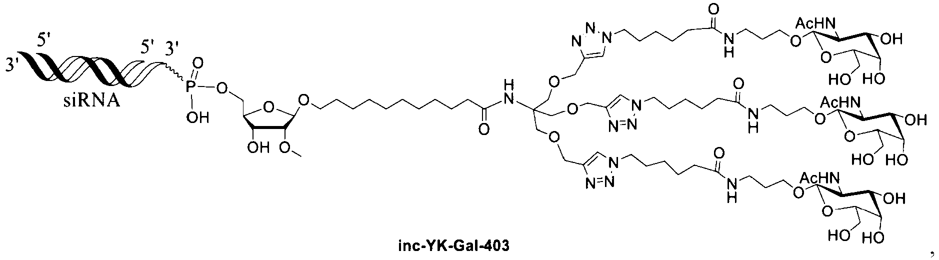

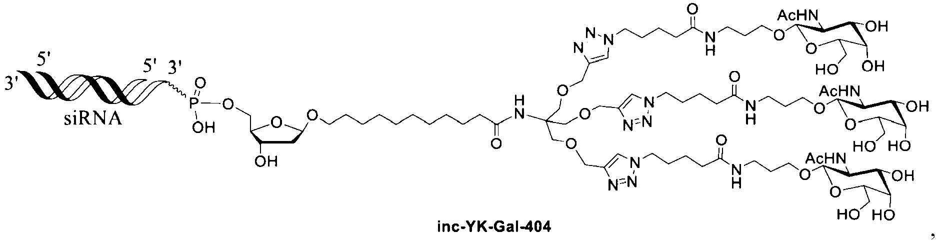

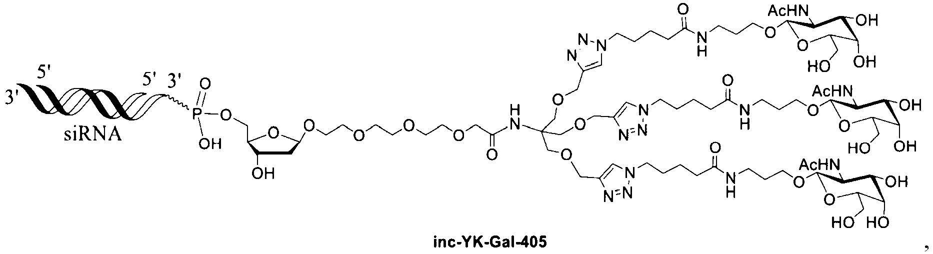

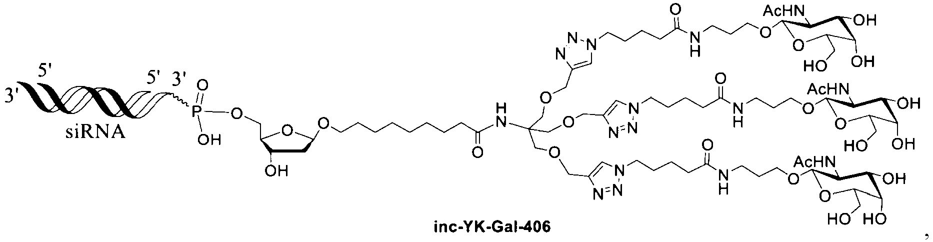

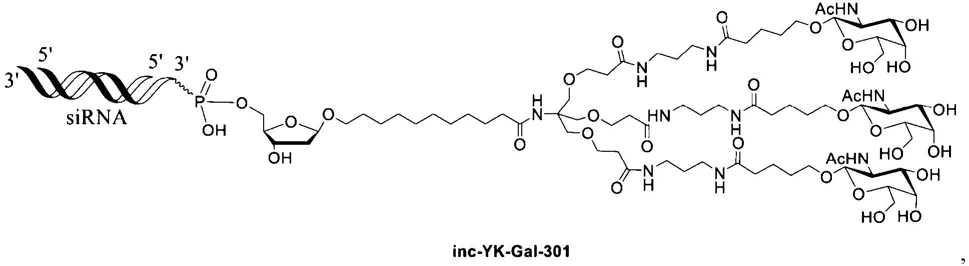

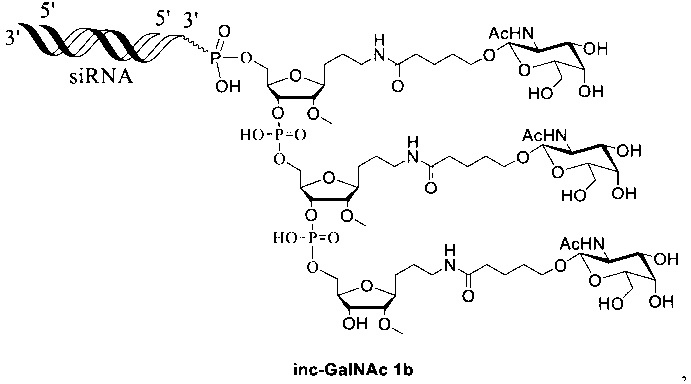

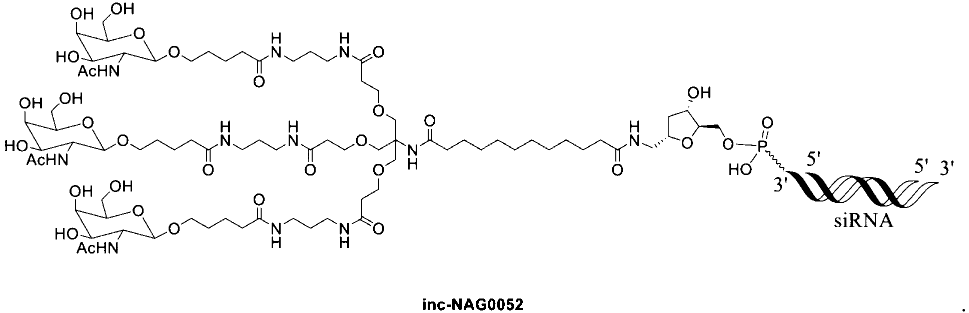

### Example 3: Inhibition of GalNAc-conjugated siRNA on PCSK9 gene in mouse serum and liver and impact on LDL-C level

The GalNAc-conjugated inclisiran siRNA sequence entered the blood, and then bound to an ASGPR on a liver plasma membrane through the GalNAc, so as to enter the liver cells. The inclisiran siRNA sequence entered the liver cells, then bound to an RNA-induced silencing complex (RISC), and bound to the mRNA encoding a PCSK9 protein under mediation of the antisense strand, so as to inhibit generation of the PCSK9 protein. The reducing of the PCSK9 protein in the liver promoted the circulation of LDL-R, increased the number of LDL-R receptors on surfaces of the liver cells, and could further reduce the LDL-C level in plasma. Therefore, the higher the amount of inclisiran siRNA sequence delivered to the liver (i.e. the higher the GalNAc delivery efficiency), the lower the amount of PCSK9 protein in both the liver and serum, and consequently, the lower the LDL-C level in the liver.Results showed that, compared with a GalNAc oligonucleotide conjugate in the prior art, the inhibition rate of PCSK9 gene expression and LDL-C lowering level in the mouse serum and liver in experimental groups inc-YK-Gal-401, inc-YK-Gal-402, and inc-YK-Gal-403 were all significantly improved. For example, compared with inc-YK-Gal-301, inc-GalNAc 1b, and inc-NAG0052, the inhibition rate of inc-YK-Gal-401 to the PCSK9 protein in serum was increased by 21.5%, 14.5%, and 12.7% respectively on Day 7, and increased by 17.3%, 10.4%, and 10.8% respectively on Day 14; the inhibition rate of the PCSK9 gene in mouse liver was increased by 21.1%, 9.8%, and 5.1% respectively on Day 14; and the LDL-C lowering level was increased by 33.4%, 19.1%, and 16.7% respectively on Day 7, and was increased by 36.4%, 20.6%, and 17.8% respectively on Day 14, which was significantly improved.

### Animal preparation:

First, hPCSK9 transgenic mice (SPF grade, purchased from GemPharmatech Co., Ltd) were adaptively fed, and were randomly grouped into a negative control group (without administration) and a siRNA test medicine group according to the amount of the PSCK9 protein in serum, and each group had 5 mice, which were all male.

### Administration route and administration dose:

The mice were administered via a single subcutaneous injection, an administration dose was 6 mg/kg, an administration volume was 1 mL/kg, an administration concentration was 6 mg/mL, and the day of administration was recorded as Day 0.

### 1. Inhibition efficiency of different GalNAc-conjugated siRNAs to PCSK9 protein expression in mouse serum

Compared with the GalNAc in the prior art, the oligonucleotide conjugates prepared by the GalNAc compounds of the present disclosure showed a significantly higher inhibition rate of PCSK9 protein expression in mouse serum. For example, the inhibition rate of inc-YK-Gal-401 on Day 7 was increased by 21.5%, 14.5%, and 12.7% compared with inc-YK-Gal-301, inc-GalNAc 1b, and inc-NAG0052, and the inhibition rate on Day 14 was increased by 17.3%, 10.4%, and 10.8%, respectively.

### Experimental procedure:

Before administration (D0) of experimental animals, 7 days (D7) after administration, and 14 days (D14) after administration, about 200 µL of blood (without anticoagulation) was obtained from the orbital venous plexus. A whole blood sample was temporarily stored in an ice box before centrifugation, centrifugation was performed for 10 min at 4 °C at 4000 r/min to separate the serum, and an ELISA kit (Sino Biological) was used to detect the PCSK9 protein level in the serum.

### Experimental results:

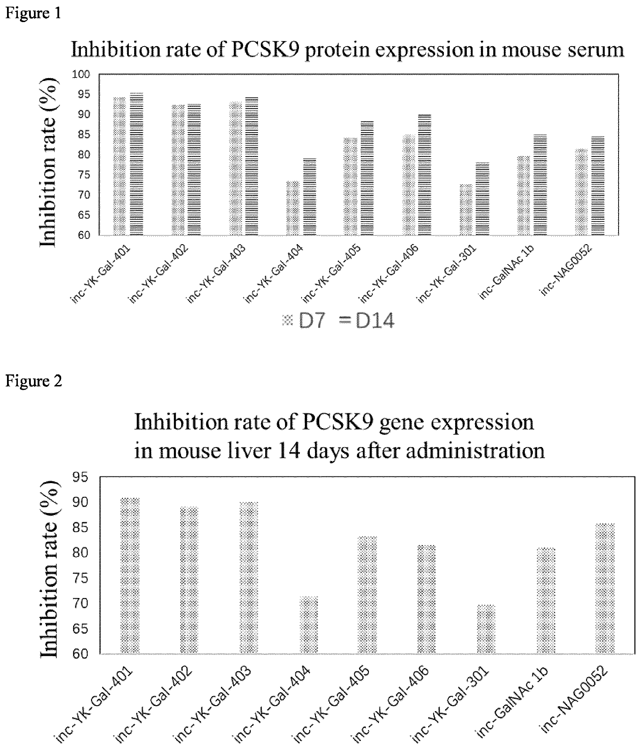

The inhibition rates of the PCSK9 protein in the serum 7 days after administration and 14 days after administration in the negative control group and each test medicine group were shown in Table 3 and Fig. 1. Data statistics and analysis were performed with GraphPad Prism 9 software.

### 1. Difference in GalNAc compound structures

**Table 2 Comparison of GalNAc compound structures**

| Name | Ribose l' position | Ribose 2' substituent | Main chain structure | Number of antennae | Does antenna contain triazole structure? |
|---|---|---|---|---|---|
| YK-GAL-401 | Oxygen | OMe | -(CH₂)_{10C}(O)NH- | 3 | Yes |
| YK-GAL-402 | Oxygen | OMe | -(CH₂)₁₀C(O)NH- | 3 | Yes |
| YK-GAL-403 | Oxygen | OMe | -(CH₂)_{10C}(O)NH- | 3 | Yes |
| YK-GAL-404 | Oxygen | H | -(CH₂)₁₀C(O)NH- | 3 | Yes |
| YK-GAL-405 | Oxygen | H | -(CH₂CH₂O)₃CH₂C(O)NH- | 3 | Yes |
| YK-GAL-406 | Oxygen | H | -(CH₂)₈C(O)NH- | 3 | Yes |
| YK-GAL-301 | Oxygen | H | -(CH₂)₁₀C(O)NH- | 3 | No |
| GalNAc 1b | Carbon | OMe | -(CH₂)₂NHC(O)- | 1 | No |
| NAG0052 | Carbon | H | -NHC(O)(CH₂)₁₀C(O)NH- | 3 | No |

| | | | | | |
|---|---|---|---|---|---|
| 1) Chemical structures of the GalNAc compounds YK-GAL-401toYK-Gal-406 designed in the present disclosure were very close. | | | | | |

From Table 1 and Table 2, it might be seen that, the chemical structures of the GalNAc compounds YK-GAL-401, YK-GAL-402, YK-GAL-403, YK-GAL-404, YK-GAL-405, and YK-GAL-406 designed in the present disclosure were very close, for example, ribose 1' positions were all oxygen, the number of antennae was 3, and the antennae all contained triazole structures.

These compounds differed only slightly in individual groups. For example, ribose 2' substituents of YK-GAL-401, YK-GAL-402, and YK-GAL-403 were methoxy (OMe), YK-GAL-404, YK-GAL-405, and YK-GAL-406 were H; main chain structures of YK-GAL-401-YK-Gal-404 were -(CH₂)₁₀C(O)NH-, YK-GAL-405 was -(CH₂CH₂O)₃CH₂C(O)NH-, and YK-GAL-406 was -(CH₂)₈C(O)NH-. Furthermore, a length of a chain in the antenna was slightly different, and other structures were identical.

2) Compared with the GalNAc compound in the prior art, in the GalNAc compounds YK-GAL-401 to YK-GAL-406 designed in the present disclosure, some chemical structures were very close, and some were very different.

Compared with the GalNAc compounds YK-GAL-301 and NAG0052 in the prior art, the GalNAc compounds YK-GAL-401, YK-GAL-402, YK-GAL-403, YK-GAL-404, YK-GAL-405, and YK-GAL-406 designed in the present disclosure had very close chemical structures, for example, YK-GAL-301 and NAG0052 also had 3 antennae, the main chain structure contained an aliphatic chain and an amide bond, and only the antennae of YK-GAL-301 and NAG0052 did not contain the triazole structure.

The GalNAc compound designed in the present disclosure had a very different chemical structure from GalNAc 1b, for example, the GalNAc 1b only had one antenna, the chain was very short, and the chain did not contain the triazole structure.

### 2. Inhibition rate of different GalNAc-conjugated siRNAs to PCSK9 protein expression in mouse serum

**Table 3 Inhibition rate of PCSK9 protein expression in mouse serum**

| Experimental group | Inhibition rate on Day 7 (%) | Inhibition rate higher than (%) | | | Inhibition rate on Day 14 (%) | Inhibition rate higher than (%) | | |
|---|---|---|---|---|---|---|---|---|
| | | inc-YK-Gal-301 | inc-GalNAc 1b | inc-NAG0052 | | inc-YK-Gal-301 | inc-GalNAc 1b | inc-NAG0052 |
| Negative control | -3.4 | - 76.1 | -83.1 | -84.9 | -5.9 | - 84.0 | -90.9 | -90.5 |
| inc-YK-Gal-401 | 94.2 | 21.5 | 14.5 | 12.7 | 95.4 | 17.3 | 10.4 | 10.8 |
| inc-YK-Gal-402 | 92.4 | 19.7 | 11.9 | 10.9 | 92.8 | 14.7 | 7.8 | 8.2 |
| inc-YK-Gal-403 | 93.1 | 20.4 | 13.4 | 11.6 | 94.3 | 16.2 | 9.3 | 9.7 |
| inc-YK-Gal-404 | 73.5 | 0.8 | -6.2 | -8.0 | 79.4 | 1.3 | -5.6 | -5.2 |
| inc-YK-Gal-405 | 84.3 | 11.6 | 4.6 | 2.8 | 88.7 | 10.6 | 3.7 | 4.1 |
| inc-YK-Gal-406 | 85.0 | 12.3 | 5.3 | 3.5 | 90.2 | 12.1 | 5.2 | 5.6 |
| inc-YK-Gal-301 | 72.7 | 0 | -7.0 | -8.8 | 78.1 | 0 | -6.9 | -6.5 |
| inc-GalNAc 1b | 79.7 | 7.0 | 0 | -1.8 | 85.0 | 6.9 | 0 | 0.4 |
| inc-NAG0052 | 81.5 | 8.8 | 1.8 | 0 | 84.6 | 6.5 | -0.4 | 0 |

1) The siRNA conjugated by the GalNAc compound of the present disclosure had a significant difference in the inhibition rate of PCSK9 protein expression in the mouse serum, and YK-GAL-401 to YK-Gal-403 were significantly higher than YK-GAL-404 to 406. For example, inc-YK-Gal-401 on Day 7 and Day 14 was 20.7% and 16.0% higher than inc-YK-Gal-404.

From Table 3, it can be seen that, the GalNAc compound-conjugated siRNAs of the present disclosure all inhibit PCSK9 protein expression in the mouse serum. Different GalNAc conjugates had a significant difference in the inhibition rate of PCSK9 protein expression in the mouse serum. The inhibition rates of inc-YK-Gal-401, inc-YK-Gal-402, inc-YK-Gal-403, inc-YK-Gal-404, inc-YK-Gal-405, and inc-YK-Gal-406 were respectively 94.2%, 92.4%, 93.1%, 73.5%, 84.3%, and 85.0% on Day 7, and were respectively 95.4%, 92.8%, 94.3%, 79.4%, 88.7%, and 90.2% on Day 14.

The inhibition rates of inc-YK-Gal-401, inc-YK-Gal-402, and inc-YK-Gal-403 all exceeded 90%, and the inhibition rate of inc-YK-Gal-404 was between 70% and 80%. The inc-YK-Gal-401 with the highest inhibition rate was 20.7% and 16.0% higher than the inc-YK-Gal-404 with the lowest inhibition rate on Day 7 and Day 14, respectively, which was significantly improved.

2) Compared with the GalNAc compound in the prior art, the siRNA conjugated by the GalNAc compounds YK-GAL-401 to YK-GAL-403 of the present disclosure showed a significantly higher inhibition rate of PCSK9 protein expression in the mouse serum. For example, inc-YK-Gal-401 on Day 7 and Day 14 was 21.5% and 17.3% higher than inc-YK-Gal-301.

The inhibition rates of the siRNA conjugates inc-YK-Gal-301, inc-GalNAc 1b, and inc-NAG0052 prepared by the GalNAc compound in the prior art were 72.7%, 79.7%, and 81.5% respectively on Day 7, and were 78.1%, 85.0%, and 84.6% respectively on Day 14.

Compared with the GalNAc compound in the prior art, the siRNA conjugated by the GalNAc compounds of the present disclosure showed a significantly higher inhibition rate of PCSK9 protein expression in the mouse serum. For example, the inhibition rates of inc-YK-Gal-401, inc-YK-Gal-402, and inc-YK-Gal-403 on Day 7 were respectively increased by 21.5%, 19.7%, and 20.4% compared with inc-YK-Gal-301, were respectively increased by 14.5%, 11.9%, and 13.4% compared with inc-GalNAc 1b, and were respectively increased by 12.7%, 10.9%, and 11.6% compared with inc-NAG0052, and thus were significantly improved; and the inhibition rates on Day 14 were respectively increased by 17.3%, 14.7%, and 16.2% compared with inc-YK-Gal-301, were respectively increased by 10.4%, 7.8%, and 9.3% compared with inc-GalNAc 1b, and were respectively increased by 10.8%, 8.2%, and 9.7% compared with inc-NAG0052, and thus were significantly improved.

3) The GalNAc compounds having similar structures and siRNA conjugates showed a significant difference in the inhibition rate of PCSK9 protein expression in the mouse serum.

The structures of the GalNAc compounds designed in the present disclosure were very close, but the activity of the siRNA conjugates was significantly different. For example, YK-GAL-401 differed only in a 2' position of a ribose ring compared to YK-GAL-404, YK-GAL-401 was methoxy, YK-GAL-404 was oxygen, and other structures were identical. However, the inhibition rate of inc-YK-Gal-401 to PCSK9 protein expression in the mouse serum was increased by more than 20% compared with ine-YK-Gal-404, and thus was significantly improved.

YK-GAL-406 was only different from YK-GAL-301 in terms of the main chain and the antennary chain. The main chain of YK-GAL-406 was 2 methylene groups less than that of YK-GAL-301. The antennary chain of YK-GAL-406 had the triazole structure, and YK-GAL-301 did not. However, the inhibition rate of inc-YK-Gal-406 on Day 7 was increased by 12.3% compared with inc-YK-Gal-301, and thus was significantly improved.

YK-GAL-401 was only slightly different from inc-NAG0052 in terms of individual groups. However, the inhibition rate of inc-YK-Gal-401 on Day 7 was increased by 12.7% compared with inc-NAG0052, and thus was significantly improved.

Therefore, not all the GalNAc compounds having similar chemical structures have the close efficiency of delivering the oligonucleotide, the inhibition rate of PCSK9 protein expression in the mouse serum by the GalNAc oligonucleotide conjugates prepared by the compounds was not consistent and might be significantly variable.

### 3. Inhibition efficiency of different GalNAc-conjugated siRNAs to PCSK9 in mouse liver

Compared with the GalNAc in the prior art, the oligonucleotide conjugates prepared by the GalNAc compounds of the present disclosure showed a significantly higher inhibition rate of PCSK9 gene expression in mouse liver. For example, the inhibition rate of inc-YK-Gal-401 was increased by 21.1% and 9.8% respectively compared with inc-YK-Gal-301 and inc-GalNAc 1b, and thus was significantly increased.

### Experimental procedure:

On Day 14 (D14) after administration of the experimental animals, the experimental animals were anesthetized, executed, and then perfused and the livers were collected.

The livers were rapidly put in a prepared 1.5 mL RNA enzyme-free EP tube held with 1 mL RNA lysis solution (trizol, Ambion) according to tissue weight:RNA lysis solution =100 mg:1 mL, three 3-mm steel balls (RNA-free treated) were added to the tube, and then put in a tissue homogenizer, the operation was performed for 30 seconds at 50 Hz, stopped for 10 seconds, and performed for 3 times, and tissue homogenate might be prepared.

Centrifugation was performed for 3 minutes at 4 °C with 12,000 × g, 400 µL of homogenate supernatant was transferred to the 1.5 mL RNA enzyme-free EP tube, and then put on ice. 80 µL of chloroform was added to each tube, violent oscillation was performed for 15 seconds, and standing was performed for 5 minutes at room temperature. Centrifugation was performed for 15 minutes at 4 °C with 12,000 × g, and 150 µL of supernatant was transferred to a new EP tube.

Equal volume isopropanol was added, liquid in the tube was placed upside down and gently mixed, standing was performed for 10 minutes at -20 °C, centrifugation was performed for 15 minutes at 4 °C with 12,000 × g, and supernatant was discarded.

1 mL 75% ethanol was added, an RNA precipitate was gently washed, centrifugation was performed for 5 minutes at 4 °C with 7,500 × g, and supernatant was sucked. Rinsing was repeatedly performed once for 5 minutes at 4 °C with 7,500 × g, and residual ethanol was clearly removed with a micro pipette tip. The residual ethanol was air-dried for 10 minutes at room temperature, and 150 µL RNase-free ddH₂O was added and dissolved. An RNA concentration was detected with a micro ultraviolet spectrophotometer. PCSK9 gene expression situations were detected through qPCR.

### Experimental results:

Through statistics of qPCR results (Mean±SD), drawing and data analysis were performed with GraphPad Prism 9 software, and specific results were shown in Table 4 and Fig. 2.

**Table 4 Inhibition rate of PCSK9 gene expression in mouse liver**

| Experimental group | Inhibition rate (%) | Inhibition rate higher than (%) | | |
|---|---|---|---|---|
| | | inc-YK-Gal-301 | inc-GalNAc 1b | inc-NAG0052 |
| Negative control | 0 | -69.8 | -81.1 | -85.8 |
| inc-YK-Gal-401 | 90.9 | 21.1 | 9.8 | 5.1 |
| inc-YK-Gal-402 | 89.3 | 19.5 | 8.2 | 3.5 |
| inc-YK-Gal-403 | 90.1 | 20.3 | 9.0 | 4.3 |
| inc-YK-Gal-404 | 71.5 | 1.7 | -9.6 | -14.3 |
| inc-YK-Gal-405 | 83.2 | 13.4 | 2.1 | -2.6 |
| inc-YK-Gal-406 | 81.6 | 11.8 | 0.5 | -4.2 |
| inc-YK-Gal-301 | 69.8 | 0 | -11.3 | -16.0 |
| inc-GalNAc 1b | 81.1 | 11.3 | 0 | -4.7 |
| inc-NAG0052 | 85.8 | 16.0 | 4.7 | 0 |

1) The siRNA conjugated by the GalNAc compound of the present disclosure had a significant difference in the inhibition rate of PCSK9 gene expression in the mouse liver, and YK-GAL-401 to YK-GAL-403 were significantly higher than YK-GAL-404 to YK-GAL-406. For example, inc-YK-Gal-401 was 19.4% higher than inc-YK-Gal-404.

From Table 4, it can be seen that, the GalNAc compound-conjugated siRNAs of the present disclosure all inhibit PCSK9 gene expression in the mouse liver. Different GalNAc conjugates had a significant difference in the inhibition rate of PCSK9 gene expression in the mouse liver. The inhibition rates of inc-YK-Gal-401, inc-YK-Gal-402, inc-YK-Gal-403, inc-YK-Gal-404, inc-YK-Gal-405, and inc-YK-Gal-406 were respectively 90.9%, 89.3%, 90.1%, 71.5%, 83.2%, and 81.6%.

The inhibition rates of inc-YK-Gal-401 and inc-YK-Gal-403 all exceeded 90%, inc-YK-Gal-402 also reached 89.3%, and the inhibition rate of inc-YK-Gal-404 was only 71.5%. The inc-YK-Gal-401 with the highest inhibition rate was 19.4% higher than the inc-YK-Gal-404 with the lowest inhibition rate, which was significantly improved.

2) Compared with the GalNAc compound in the prior art, the siRNA conjugated by the GalNAc compounds YK-GAL-401 to YK-GAL-403 of the present disclosure showed a significantly higher inhibition rate of PCSK9 gene expression in the mouse liver. For example, inc-YK-Gal-401 was 21.1% higher than inc-YK-Gal-301.

The inhibition rates of the siRNA conjugates inc-YK-Gal-301, inc-GalNAc 1b, and inc-NAG0052 prepared by the GalNAc compound in the prior art were 69.8%, 81.1%, and 85.8% respectively.

Compared with the GalNAc compound in the prior art, the siRNA conjugated by the GalNAc compounds of the present disclosure showed a significantly higher inhibition rate of PCSK9 protein expression in the mouse serum. For example, the inhibition rates of inc-YK-Gal-401, inc-YK-Gal-402, and inc-YK-Gal-403 were respectively increased by 21.1%, 19.5%, and 20.3% compared with inc-YK-Gal-301, and increased by 9.8%, 8.2%, and 9.0% compared with inc-GalNAc 1b, and thus was significantly improved.

3) The GalNAc compounds having similar structures and siRNA conjugates showed a significant difference in the inhibition rate of PCSK9 gene expression in the mouse liver.

The structures of the GalNAc compounds designed in the present disclosure were very close, but the activity of the siRNA conjugates was significantly different. For example, YK-GAL-401 differed only in a 2' position of a ribose ring compared to YK-GAL-404, YK-GAL-401 was methoxy, YK-GAL-404 was oxygen, and other structures were identical. However, the inhibition rate of inc-YK-Gal-401 to PCSK9 gene expression in the mouse liver might be increased by 19.4% compared with inc-YK-Gal-404, and thus was significantly improved.

YK-GAL-406 was only different from YK-GAL-301 in terms of the main chain and the antennary chain. The main chain of YK-GAL-406 was 2 methylene groups less than that of YK-GAL-301. The antennary chain of YK-GAL-406 had the triazole structure, and YK-GAL-301 did not. However, the inhibition rate of inc-YK-Gal-406 was increased by 11.8% compared with inc-YK-Gal-301, and thus was significantly improved.

Therefore, not all the GalNAc compounds having similar chemical structures had the close efficiency of delivering the oligonucleotide, the inhibition rate of PCSK9 gene expression in the mouse liver by the GalNAc oligonucleotide conjugates prepared by the compounds was not consistent and might be significantly variable.

### 4. Impact of different GalNAc-conjugated siRNAs to LDL-C level in mouse serum

Compared with the GalNAc in the prior art, the oligonucleotide conjugates prepared by the GalNAc compounds of the present disclosure significantly elevated an LDL-C lowering level in the mouse serum. For example, the LDL-C lowering levels in the mice in the inc-YK-Gal-401 group on Day 14 were increased by 36.4%, 20.6%, and 17.8% respectively compared with those in the inc-YK-Gal-301, inc-GalNAc 1b, and NAG0052 groups, and thus were significantly increased.

### Experimental procedure:

Before administration (D0) of experimental animals, Day 7 (D7) after administration, and Day 14 (D14) after administration, about 200 µL of blood (without anticoagulation) was obtained from the orbital venous plexus. A whole blood sample was temporarily stored in an ice box before centrifugation, centrifugation was performed for 10 min at 4 °C at 4000 r/min to separate the serum, and an LDL-C level in serum was detected.

### Experimental results:

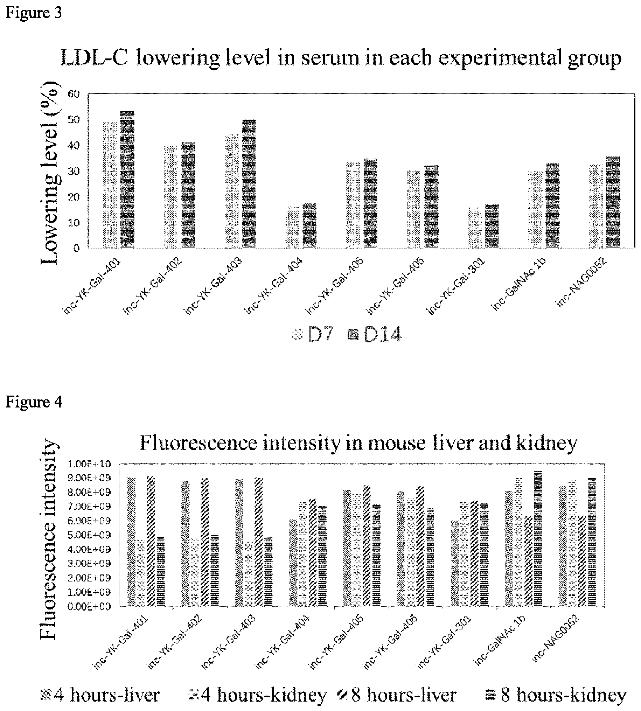

Through statistics of LDL-C in serum (Mean±SD), drawing and data analysis were performed with GraphPad Prism 9 software, and specific results were shown in Table 5 and Fig. 3.

**Table 5 LDL-C lowering level in mouse serum**

| Experimental group | Lowering level on Day 7 (%) | Lowering level higher than (%) | | | Lowering level on Day 14 (%) | Lowering level higher than (%) | | |
|---|---|---|---|---|---|---|---|---|
| | | inc-YK-Gal-301 | inc-GalNAc 1b | inc-NAG0052 | | inc-YK-Gal-301 | inc-GalNAc 1b | inc-NAG0052 |
| Negative control | -1.9 | -17.7 | -32.0 | -34.4 | -2.3 | -19.4 | -35.2 | -38.0 |
| inc-YK-Gal-401 | 49.2 | 33.4 | 19.1 | 16.7 | 53.5 | 36.4 | 20.6 | 17.8 |
| inc-YK-Gal-402 | 39.6 | 23.8 | 9.5 | 7.1 | 41.3 | 24.2 | 8.4 | 5.6 |
| inc-YK-Gal-403 | 44.5 | 28.7 | 14.4 | 12.0 | 50.4 | 33.3 | 17.5 | 14.7 |
| inc-YK-Gal-404 | 16.2 | 0.4 | -13.9 | -16.3 | 17.5 | 0.4 | -15.4 | -18.2 |
| inc-YK-Gal-405 | 33.7 | 17.9 | 3.6 | 1.2 | 35.0 | 17.9 | 2.1 | -0.7 |
| inc-YK-Gal-406 | 30.3 | 14.5 | 0.2 | -2.2 | 32.2 | 15.1 | -0.7 | -3.5 |
| inc-YK-Gal-301 | 15.8 | 0 | -14.3 | -16.7 | 17.1 | 0 | -15.8 | -18.6 |
| inc-GalNAc 1b | 30.1 | 14.3 | 0 | -2.4 | 32.9 | 15.8 | 0 | -2.8 |
| inc-NAG0052 | 32.5 | 16.7 | 2.4 | 0 | 35.7 | 18.6 | 2.8 | 0 |

1) The siRNA conjugated by the GalNAc compound of the present disclosure had a significant difference in the reducing of the LDL-C level in the mouse serum, and the reduced level in YK-GAL-401 to YK-GAL-403 was significantly higher than that in YK-GAL-404 to 406. For example, inc-YK-Gal-401 on Day 7 and Day 14 was 33.0% and 36.0% higher than inc-YK-Gal-404, which was significantly increased. From Table 5, it can be seen that, the GalNAc compound-conjugated siRNAs of the present disclosure all reduce the LDL-C level in the mouse serum. Different GalNAc conjugates showed a significant difference in the LDL-C lowering level in the mouse serum. The lowering level of inc-YK-Gal-401, inc-YK-Gal-402, inc-YK-Gal-403, ine-YK-Gal-404, inc-YK-Gal-405, and inc-YK-Gal-406 were respectively 49.2%, 39.6%, 44.5%, 16.2%, 33.7%, and 30.3% on Day 7, and were respectively 53.5%, 41.3%, 50.4%, 17.5%, 35.0%, and 32.2% on Day 14.

The lowering level of inc-YK-Gal-401 and inc-YK-Gal-403 on Day 7 both exceeded 40%, and exceeded 50% on Day 14. The lowering level of inc-YK-Gal-402 on Day 7 also reached 39.6%, and reached 41.3% on Day 14. The lowering level of ine-YK-Gal-404 on Day 7 was only 16.2%, and was only 17.5% on Day 14. The lowering levels of inc-YK-Gal-401, inc-YK-Gal-402, and inc-YK-Gal-403 on Day 7 were respectively increased by 33.0%, 23.4%, and 28.3% compared with inc-YK-Gal-404, and were respectively increased by 36.0%, 23.8%, and 32.9% on Day 14, and thus were significantly improved.

2) Compared with the GalNAc compound in the prior art, the siRNA conjugated by the GalNAc compounds YK-GAL-401 to YK-GAL-403 of the present disclosure significantly elevated the LDL-C lowering level in the mouse serum. For example, inc-YK-Gal-401 on Day 7 and Day 14 was 33.4% and 36.4% higher than inc-YK-Gal-301.

The LDL-C lowering levels of the siRNA conjugates inc-YK-Gal-301, inc-GalNAc 1b, and inc-NAG0052 prepared by the GalNAc compound in the prior art were 15.8%, 30.1%, and 32.5% respectively on Day 7, and were 17.1%, 32.9%, and 35.7% respectively on Day 14.

Compared with the GalNAc compound in the prior art, the siRNA conjugated by the GalNAc compounds of the present disclosure significantly elevated the LDL-C lowering level in the mouse serum. For example, the lowering levels of inc-YK-Gal-401, inc-YK-Gal-402, and inc-YK-Gal-403 on Day 7 were respectively increased by 33.4%, 23.8%, and 28.7% compared with inc-YK-Gal-301, were respectively increased by 19.1%, 9.5%, and 14.4% compared with inc-GalNAc 1b, and were respectively increased by 16.7%, 7.1%, and 12.0% compared with inc-NAG0052, and thus were significantly improved. The lowering levels on Day 14 were respectively increased by 36.4%, 24.2%, and 33.3% compared with inc-YK-Gal-301, were respectively increased by 20.6%, 8.4%, and 17.5% compared with inc-GalNAc 1b, and were respectively increased by 17.8%, 5.6%, and 14.7% compared with inc-NAG0052, and thus were significantly improved.

3) The GalNAc compounds having similar structures and siRNA conjugates showed a significant difference in the LDL-C lowering level in the mouse serum.

The structures of the GalNAc compounds designed in the present disclosure were very close, but the activity of the siRNA conjugates was significantly different. For example, YK-GAL-401 differed only in a 2' position of a ribose ring compared to YK-GAL-404, YK-GAL-401 was methoxy, YK-GAL-404 was oxygen, and other structures were identical. However, the LDL-C level in the mouse serum reduced by inc-YK-Gal-401 might be increased by more than 30% compared with inc-YK-Gal-404, and thus was significantly improved.

YK-GAL-406 was only different from YK-GAL-301 in terms of the main chain and the antennary chain. The main chain of YK-GAL-406 was 2 methylene groups less than that of YK-GAL-301. The antennary chain of YK-GAL-406 had the triazole structure, and YK-GAL-301 did not. However, the LDL-C level in the mouse serum reduced by inc-YK-Gal-406 was increased by 15.1% compared with inc-YK-Gal-301, and thus was significantly improved.

YK-GAL-401 was only slightly different from inc-NAG0052 in terms of individual groups. However, the LDL-C level in the mouse serum reduced by inc-YK-Gal-401 was increased by 17.8% compared with inc-NAG0052, and thus was significantly improved.

Therefore, not all the GalNAc compounds having similar chemical structures have the close efficiency of delivering the oligonucleotide, the inhibition rate of PCSK9 protein expression in the mouse serum by the GalNAc oligonucleotide conjugates prepared by the compounds was not consistent and might be significantly variable.

Example 4: Distribution of different GalNAc-conjugated siRNAs in different tissue organs in mice Results showed that, the GalNAc compound designed in the present disclosure might efficiently deliver nucleic acids to the animal livers.

### Experimental procedure:

6-8-week wild-type C57BL/6 mice were used, and each group had 6 mice. Each group of mice were administrated with siRNA conjugates inc-YK-GAL-401, inc-YK-GAL-402, inc-YK-GAL-403, inc-YK-GAL-404, inc-YK-GAL-405, inc-YK-GAL-406, inc-YK-GAL-301, inc-GalNAc 1b, and inc-NAG0052 with a Cy5 fluorescent group. The negative control group was not administered. The mice were weighed and then administered according to a dose of 6 mg/kg through subcutaneous injection, and determined based on the administration volume, and an injection amount did not exceed 0.1-0.2 mL.

After administration, abdomen furs of the mice were shaved, the mice were narcotized with isoflurane and then placed in a small animal in vivo imaging system in a lying manner, and mouse imaging situations 4 hours and 8 hours after administration were observed under a Cy5 channel. The luminous intensities of the mice were counted with a living imaging software Living Image, and differences between subject groups were compared.

### Experimental results:

Fluorescence intensity (which might represent relative content of oligonucleotide) detection results in mouse liver and kidney were shown in Table 6 and Fig. 4. Results showed that, the GalNAc compound designed in the present disclosure might efficiently deliver nucleic acids to the animal livers.

**Table 6 Fluorescence intensity in mouse liver and kidney**

| Experimental group | 4 hours-liver | 4 hours-kidney | 8 hours-liver | 8 hours-kidney |
|---|---|---|---|---|
| Negative control group | 6.22E+07 | 5.47E+07 | 6.38E+07 | 5.79E+07 |
| inc-YK-Gal-401 | 9.03E+09 | 4.62E+09 | 9.11 E+09 | 4.91E+09 |
| inc-YK-Gal-402 | 8.79E+09 | 4.76E+09 | 8.95E+09 | 5.02E+09 |
| inc-YK-Gal-403 | 8.92E+09 | 4.52E+09 | 9.01E+09 | 4.83E+09 |
| inc-YK-Gal-404 | 6.09E+09 | 7.30E+09 | 7.53E+09 | 7.03E+09 |
| inc-YK-Gal-405 | 8.15E+09 | 7.91E+09 | 8.51E+09 | 7.11E+09 |
| inc-YK-Gal-406 | 8.10E+09 | 7.58E+09 | 8.41E+09 | 6.90E+09 |
| inc-YK-Gal-301 | 6.02E+09 | 7.36E+09 | 7.40E+09 | 7.21E+09 |
| inc-NAG0052 | 8.41E+09 | 8.92E+09 | 6.36E+09 | 9.03E+09 |
| inc-GalNAc 1b | 8.09E+09 | 9.06E+09 | 6.35E+09 | 9.48E+09 |

From Table 6, it might be seen that, the oligonucleotide marked with Cy5 fluorescence was distributed in the mouse liver and kidney. The fluorescence intensity of inc-YK-Gal-401 in the mouse liver was the highest (i.e., the relative content of oligonucleotide was the highest), and the fluorescence intensity in the liver for 4 hours and 8 hours might respectively reach 9.03E+09 and 9.11E+09. The fluorescence intensities of inc-YK-Gal-402 and inc-YK-Gal-403 in the liver were also very high, and were respectively 8.79E+09 and 8.92E+09 for 4 hours, and were respectively 8.95E+09 and 9.01E+09 for 8 hours. It indicated that the GalNAc compound designed in the present disclosure might efficiently deliver the oligonucleotide to the liver.

To sum up, the present disclosure designed a series of new GalNAc compounds, which had a completely different chemical structure from a GalNAc compound in the prior art. For the GalNAc compound designed in the present disclosure, the triazole structure was introduced in a linker arm. By means of the advantage of click chemistry, a series of new GalNAc compounds containing triazole structures might be constructed rapidly. The GalNAc oligonucleotide conjugate prepared by the GalNAc compound of the present disclosure might realize efficient liver targeting delivery, and activity was significantly improved compared with that of the representative GalNAc compounds in the prior art.
1. Compared with the GalNAc in the prior art, the oligonucleotide conjugates prepared by the GalNAc compounds YK-GAL-401 to YK-GAL-403 of the present disclosure showed a significantly higher inhibition rate of PCSK9 protein expression in mouse serum. For example, the inhibition rate of inc-YK-Gal-401 on Day 7 was increased by 21.5%, 14.5%, and 12.7% compared with inc-YK-Gal-301, inc-GalNAc 1b, and inc-NAG0052, and the inhibition rate on Day 14 was increased by 17.3%, 10.4%, and 10.8%, respectively.
   1) The siRNA conjugated by the GalNAc compound of the present disclosure had a significant difference in the inhibition rate of PCSK9 protein expression in the mouse serum, and YK-Gal-401 to YK-Gal-403 were significantly higher than YK-Gal-404 to 406. For example, inc-YK-Gal-401 on Day 7 and Day 14 was 20.7% and 16.0% higher than inc-YK-Gal-404.
   2) Compared with the GalNAc compound in the prior art, the siRNA conjugated by the GalNAc compounds YK-Gal-401 to YK-Gal-403 of the present disclosure showed a significantly higher inhibition rate of PCSK9 protein expression in the mouse serum. For example, inc-YK-Gal-401 on Day 7 and Day 14 was 21.5% and 17.3% higher than inc-YK-Gal-301.
   3) The GalNAc compounds having similar structures and siRNA conjugates showed a significant difference in the inhibition rate of PCSK9 protein expression in the mouse serum. Therefore, not all the GalNAc compounds having similar chemical structures had the close efficiency of delivering the oligonucleotide, the inhibition rate of PCSK9 protein expression in the mouse serum by the GalNAc oligonucleotide conjugates prepared by the compounds was not consistent and might be significantly variable.
2. Compared with the GalNAc in the prior art, the oligonucleotide conjugates prepared by the GalNAc compounds YK-GAL-401 to YK-GAL-403 of the present disclosure showed a significantly higher inhibition rate of PCSK9 gene expression in mouse liver. For example, the inhibition rate of inc-YK-Gal-401 was increased by 21.1% and 9.8% respectively compared with inc-YK-Gal-301 and inc-GalNAc 1b, and thus was significantly increased.
   1) The siRNA conjugated by the GalNAc compound of the present disclosure had a significant difference in the inhibition rate of PCSK9 gene expression in the mouse liver, and YK-Gal-401 to YK-Gal-403 were significantly higher than YK-Gal-404 to 406. For example, inc-YK-Gal-401 was 19.4% higher than inc-YK-Gal-404.
   2) Compared with the GalNAc compound in the prior art, the siRNA conjugated by the GalNAc compounds YK-Gal-401 to YK-Gal-403 of the present disclosure showed a significantly higher inhibition rate of PCSK9 gene expression in the mouse liver. For example, inc-YK-Gal-401 was 21.1% higher than inc-YK-Gal-301.
   3) The GalNAc compounds having similar structures and siRNA conjugates showed a significant difference in the inhibition rate of PCSK9 gene expression in the mouse liver. Therefore, not all the GalNAc compounds having similar chemical structures had the close efficiency of delivering the oligonucleotide, the inhibition rate of PCSK9 gene expression in the mouse liver by the GalNAc oligonucleotide conjugates prepared by the compounds was not consistent and might be significantly variable.
3. Compared with the GalNAc in the prior art, the oligonucleotide conjugates prepared by the GalNAc compounds YK-GAL-401 to YK-GAL-403 of the present disclosure significantly elevated a LDL-C lowering level in the mouse serum. For example, the LDL-C lowering levels in the mice in the inc-YK-Gal-401 group on Day 14 were increased by 36.4%, 20.6%, and 17.8% respectively compared with those in the inc-YK-Gal-301, inc-GalNAc 1b, and NAG0052 groups, and thus were significantly increased.
   1) The siRNA conjugated by the GalNAc compound of the present disclosure had a significant difference in the reducing of the LDL-C level in the mouse serum, and the reduced level in YK-Gal-401 toYK-Gal-403 was significantly higher than that in YK-Gal-404 to YK-Gal-406. For example, inc-YK-Gal-401 on Day 7 and Day 14 was 33.0% and 36.0% higher than inc-YK-Gal-404, which was significantly increased.
   2) Compared with the GalNAc compound in the prior art, the siRNA conjugated by the GalNAc compounds YK-Gal-401 to YK-Gal-403 of the present disclosure significantly elevated the LDL-C lowering level in the mouse serum. For example, inc-YK-Gal-401 on Day 7 and Day 14 was 33.4% and 36.4% higher than inc-YK-Gal-301.
   3) The GalNAc compounds having similar structures and siRNA conjugates showed a significant difference in the LDL-C lowering level in the mouse serum. Therefore, not all the GalNAc compounds having similar chemical structures had the close efficiency of delivering the oligonucleotide, the inhibition rate of PCSK9 protein expression in the mouse serum by the GalNAc oligonucleotide conjugates prepared by the compounds was not consistent and might be significantly variable.
4. The compound designed in the present disclosure might efficiently deliver the oligonucleotide to the liver.

For example, the liver fluorescence intensity at 4 hours in the inc-YK-Gal-401, inc-YK-Gal-402, and inc-YK-Gal-403 groups might respectively reach 9.03E+09, 8.79E+09, and 8.92E+09, and respectively reached 9.11E+09, 8.95E+09, and 9.01E+09 at 8 hours, indicating that the GalNAc compound designed in the present disclosure might efficiently deliver the oligonucleotide to animal liver.

## Claims

1. A GalNAc compound shown in formula (I) or a pharmaceutically acceptable salt thereof, 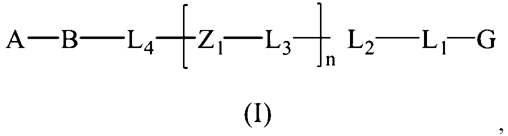 wherein,
L₁ is -C(O)NH-*, and the end * is connected to G;
L₂ is -(CH₂)n₁- or -(CH₂CH₂O)n₂CH₂-, wherein n₁ and n₂ are integers from 1 to 7;
Z₁ is -(CH₂)n₃-, wherein n₃ is an integer from 1 to 7;
L₃ is -HNC(O)- or -C(O)NH-;
n is 0;
L₄ is -(CH₂)n₄-, wherein n₄ is an integer from 0 to 7;
A is 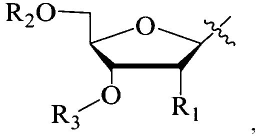 R₁ is H or C₁₋₆ alkoxy; R₂ is 4,4'-dimethoxytrityl or monomethoxytrityl; R₃ is -CO(CH₂)₂CONH-E, and E is Controlled Pore Glass (CPG); B is O or S;
G is 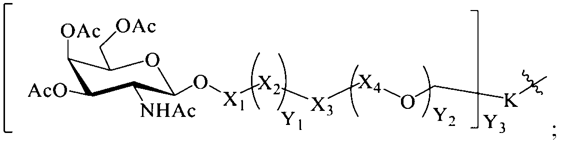
wherein,
X₁ is -(CH₂)₃-;
X₂ is -HNC(O)-**, and the end ** is connected to X₃;
X₃ is -(CH₂)_{b}-, b is an integer from 1 to 6;
X₄ is 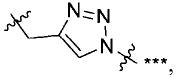
and the end *** is connected to X₃;
Y₁ is 1;
Y₂ is 1;
Y₃ is 3;
K is a carbon atom.

2. The GalNAc compound shown in formula (I) or the pharmaceutically acceptable salt thereof according to claim 1, wherein the GalNAc compound shown in formula (I) meets one or more of the following conditions:
(1) L₂ is -(CH₂)₃-, -(CH₂)₄-, -(CH₂)₅-, -(CH₂)₆-, or -(CH₂)₇-;
(2) L₄ is not present, or is -(CH₂)₃-, -(CH₂)₄-, -(CH₂)₅-, -(CH₂)₆-, or -(CH₂)₇-;
(3) R₁ is H or methoxy;
(4) R₂ is 4,4'-dimethoxytrityl;
(5) B is O.

3. The GalNAc compound shown in formula (I) or the pharmaceutically acceptable salt thereof according to claim 1, wherein L₂ is -(CH₂CH₂O)₃CH₂-.

4. The GalNAc compound shown in formula (I) or the pharmaceutically acceptable salt thereof according to claim 1, wherein G is: 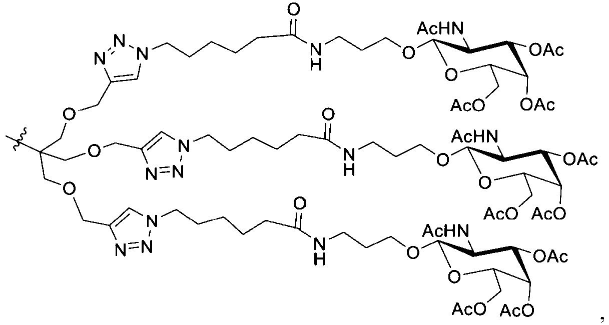 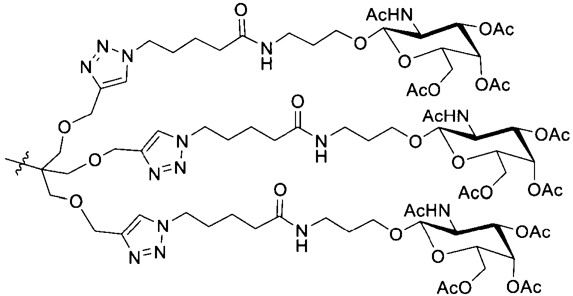 or

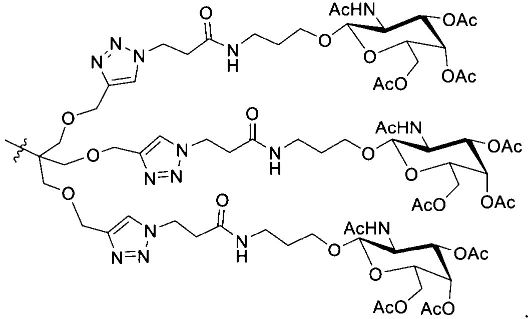

5. The GalNAc compound shown in formula (I) or the pharmaceutically acceptable salt thereof according to claim 1, wherein the compound is compound YK-GAL-401, YK-GAL-402, YK-GAL-403, YK-GAL-404, YK-GAL-405, or YK-GAL-406 having the following structure, wherein E is the CPG; 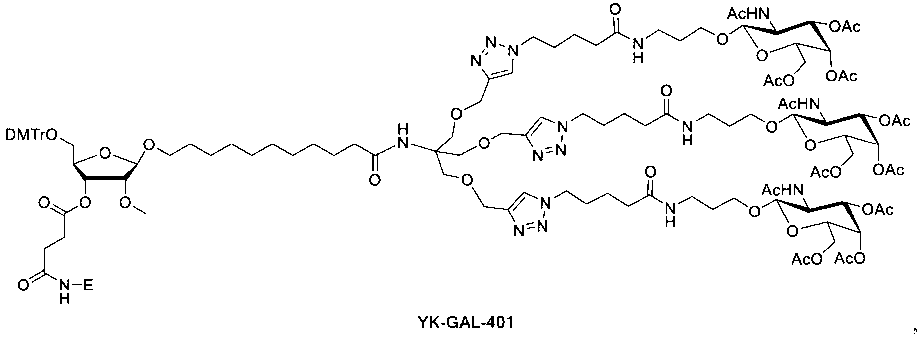 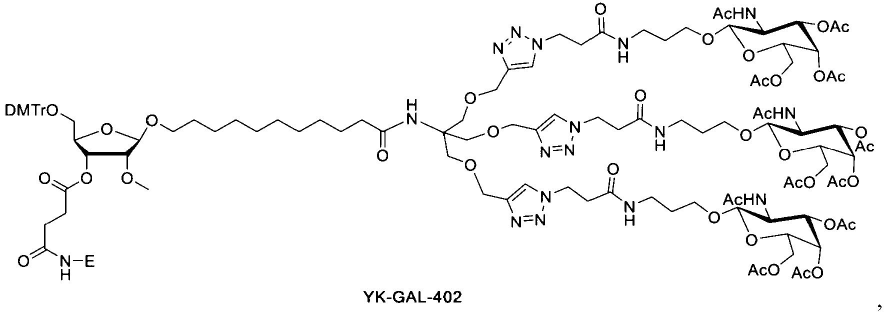 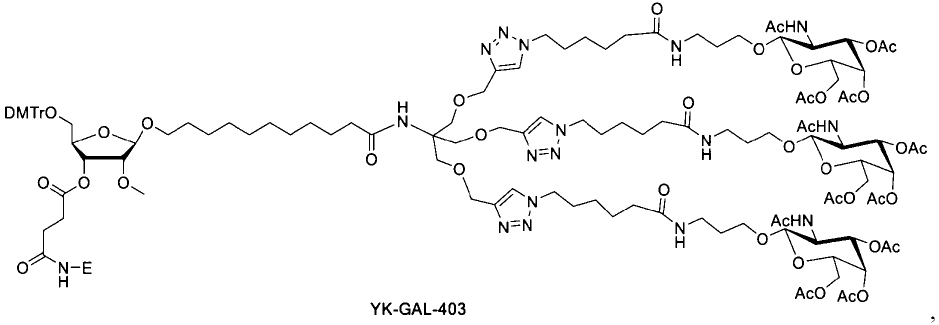 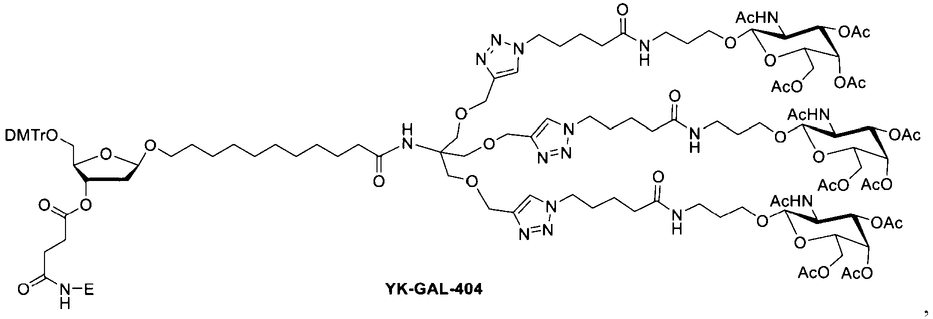 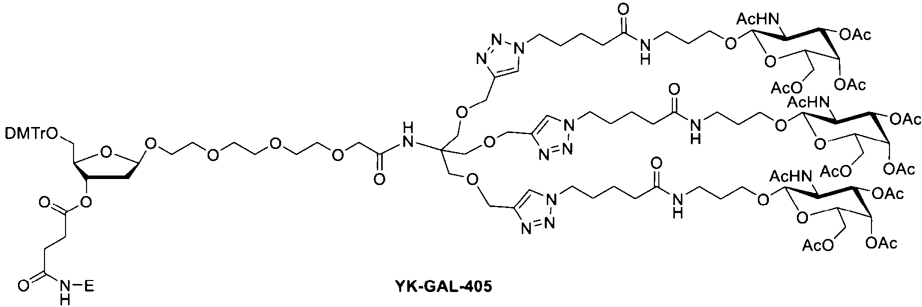 or

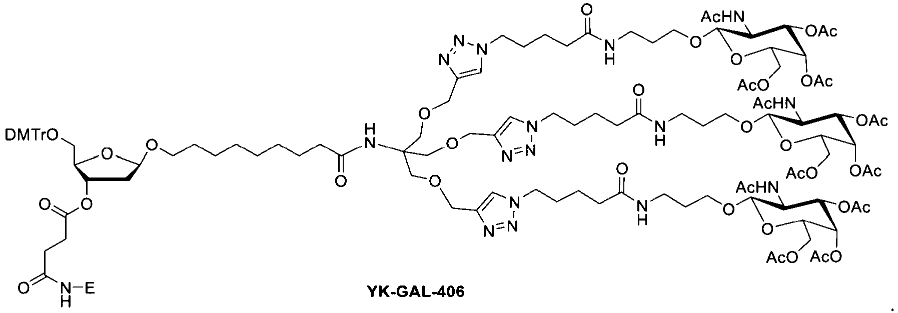

6. A conjugate shown in formula (IIA) or (IIB) or a pharmaceutically acceptable salt thereof, 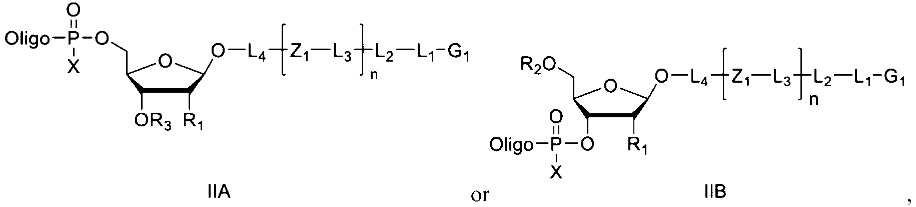
wherein Oligo represents oligonucleotide, X represents hydroxyl or sulfhydryl, and G₁ is 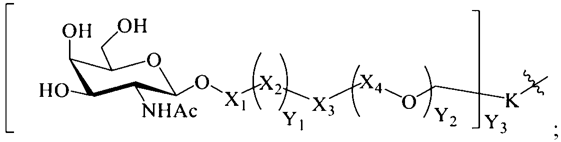
wherein R₂ is H, R₃ is H; L₁, L₂, Z₁, L₃, L₄, n, R₁, X₁, X₂, X₃, X₄, Y₁, Y₂, Y₃, and K are defined as described in claim 1;
a sense strand of the oligonucleotide has a sequence shown in SEQ ID NO: 46, an antisense strand of the oligonucleotide has a sequence shown in SEQ ID NO: 47.

7. The conjugate or a pharmaceutically acceptable salt thereof according to claim 6, wherein the conjugate is conjugated by the oligonucleotide and YK-GAL-401, YK-GAL-402, YK-GAL-403, YK-GAL-404, YK-GAL-405, or YK-GAL-406 according to claim 5, and the sense strand of the oligonucleotide has a sequence shown in SEQ ID NO: 46, the antisense strand of the oligonucleotide has a sequence shown in SEQ ID NO: 47.

8. The conjugate or the pharmaceutically acceptable salt thereof according to claim 6, wherein the conjugate is any one of the following conjugates: 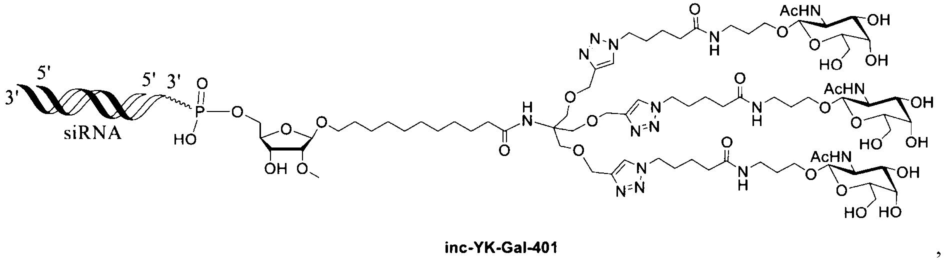 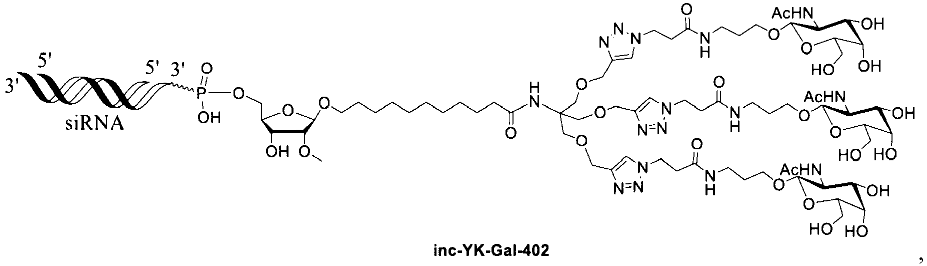 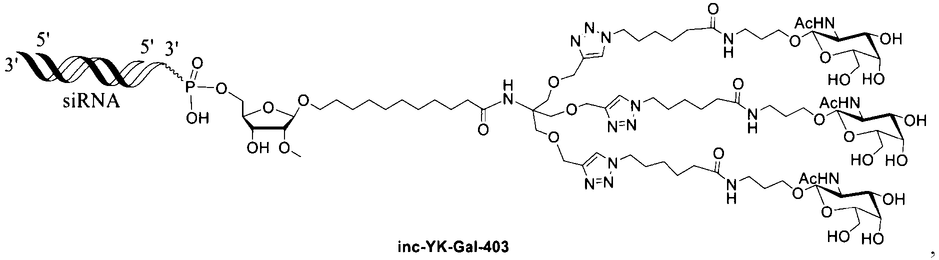 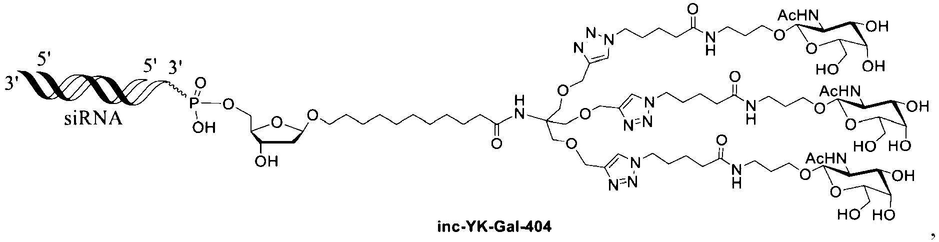 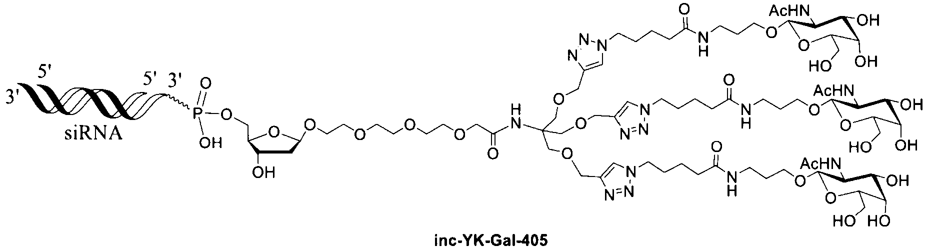 or 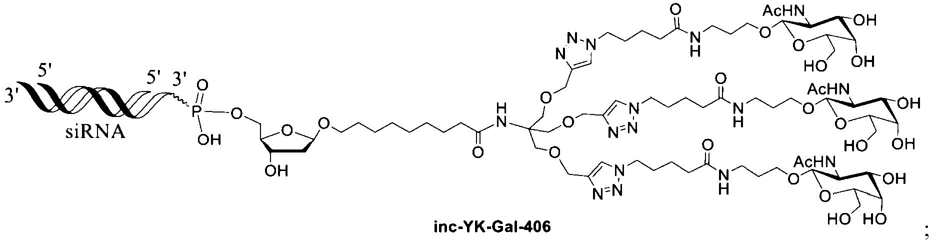 wherein the sense strand of siRNA has a sequence shown in SEQ ID NO: 46, and the antisense strand of the siRNA has a sequence shown in SEQ ID NO: 47.

9. A pharmaceutical composition, comprising the conjugate or a pharmaceutically acceptable salt thereof according to any one of claims 6 to 8, and at least one pharmaceutically acceptable excipient.

10. A kit, comprising the conjugate or a pharmaceutically acceptable salt thereof according to any one of claims 6 to 8.
